# EUROPEAN PATENT APPLICATION

(11) **EP 1 454 987 A1**
(43) Date of publication of application: **08.09.2004**
(21) Application number: 03004826.8
(22) Date of filing: 05.03.2003
(51) Int. Cl.: C12N 15/31, C12N 15/62, C07K 14/195, A61K 39/02, G01N 33/50

(54) **Polypeptides translocated into cells by the VirB/VirD4 type IV secretion system and uses thereof**

(71) Applicant: Universität Basel, 4003 Basel (CH)
(72) Inventor: Dehio, Christoph, 4125 Riehen (CH); Schmid, Michael, 4056 Basel (CH); Schülein, Ralf, 4056 Basel (CH); Rhomberg, Thomas, 4056 Basel (CH); Guye, Patrick, 4056 Basel (CH); Dehio, Michaela, 4125 Riehen (CH)

(57) **Abstract**

The invention includes polypeptides of *Bartonella*, which are translocated by the VirB/VirD4 type IV secretion system into cells, preferably mammalian cells, as well as the nucleic acids encoding these polypeptides. The invention includes the use of the C-terminal translocation signal of these polypeptides to direct the translocation of an N-terminally fused peptide sequence into mammalian cells. This method also allows screening for compounds that block or inhibit the type IV secretion process of Gram-negative bacteria into mammalian cells. Additionally, the invention includes the manipulation of mammalian cells by bioactive peptide sequences represented in these translocated polypeptides or encoded by the corresponding nucleic acids. This method includes introduction of these bioactive sequences as parts of polypeptides or as recombinant DNA. Finally the invention includes methods for using these sequences for the production of diagnostics, vaccines or therapeutics.

## Description

### Technical Field

The present invention relates to polypeptides which are translocated into cells by the VirB/VirD4 type IV secretion system as well as to the nucleic acids encoding said polypeptides. Furthermore, the present invention is directed to the use of said polypeptides and nucleic acids for diagnosis, therapy and in screening methods.

### Background Art

The transfer of macromolecules into the cytosol of cells is e.g. important for studying protein function and for therapeutical purposes. Delivery of proteins to the cytosol is crucial for the induction of cell-mediated immunity and is a significant challenge facing the rational design of vaccines to intracellular pathogens.

There exist already several methods for the introduction of macromolecules into cells such as electroporation, microinjection, fusion methodologies such as fusion with vesicles and liposomes, chemical treatments employing the use of ATP or EDTA, or the external additions of molecules mixed with pore-forming toxins such as alpha-toxin of Staphylococcus aureus. Although these methods are well established in the field, many of these methods have disadvantages such as the molecule to be delivered may require laborious purification or the delivery method is limited to in vitro use. In order to overcome these obstacles, investigators have sought to use biological vectors that can enter tissues, cells and specific cellular compartments for the delivery of macromolecules. These vectors are often derived from bacteria that have evolved to invade and replicate in specific hosts, organs or cell types. Bacterial vectors have been exploited primarily based on the type III secretion machinery present in numerous gram-negative bacteria. However, these bacterial delivery systems have obstacles in that many proteins are not translocated if already folded in the bacterial cytoplasm or require specific chaperones to remain in a partially unfolded state.

There is therefore a need for an improved and more efficient and widely applicable method for delivering proteins produced by bacteria to the cytosol of eukaryotic cells.

### Disclosure of the Invention

Hence it is a first object of the present invention to provide a polypeptide comprising at least one VTP (VirB/VirD4-translocated protein) domain or a functional fragment thereof wherein said polypeptide is translocated into a cell by the VirB/VirD4 translocation system.

In a preferred embodiment said VirB/VirD4 translocation system is from a Gram-negative bacteria, preferably from a *Bartonella species,* preferably from *Bartonella henselae.*

In a further preferred embodiment the proteins of the present invention do not comprise a sequence as set forth in Seq. Id. No. 29 (fic-1).

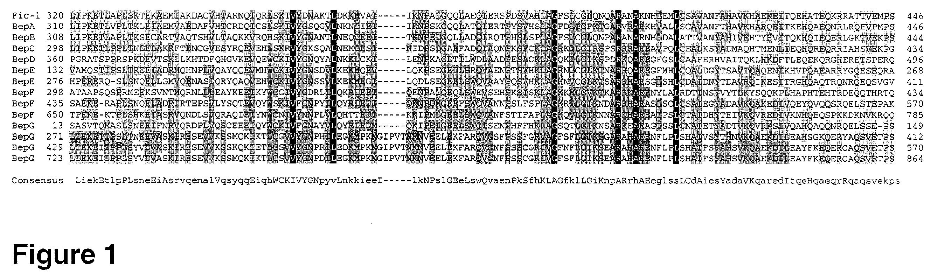

In a further preferred embodiment said polypeptide comprises a VTP domain which is at least 15% identical to the VTP domain consensus of Figure 1 (Seq. Id. No. 30) if fully aligned, preferably 30%, more preferably 40%, even more preferably 50% and most preferably 60%.

In a further preferred embodiment said polypeptide is selected from the group consisting of Seq.Id. No. 3, 7, 9, 11, 13, 15, 17 and 18.

In a second aspect the present invention relates to a nucleotide sequence encoding a polypeptide of the present invention.

In a preferred embodiment said nucleotide sequence is selected from the group consisting of the coding sequences (CDS) of Seq. Id. No. 2, 6, 8, 10, 12, 14 and 16.

In a further aspect, the present invention is directed to a fusion polypeptide comprising a VTP domain or a functional fragment thereof and a polypeptide to be translocated into a cell by the VirB/VirD4 translocation system.

Said fusion polypeptide preferably further comprises C-terminal to the VTP domain or a functional fragment thereof a positively charged amino acid sequence motif comprising at least three positively charged amino acids, preferably said motif comprises the amino acid sequence HRHHRRHH (Seq. Id. No. 30).

In another preferred embodiment said bacteria encoding a functional type IV secretion apparatus are Gram-negative bacteria, preferably said bacteria belong to the genus Bartonella, most preferably Bartonella *henselae.*

In a further preferred embodiment said VTP domain has a sequence which is at least 15% identical to the consensus of the VTP domain set forth in Seq. Id. No. 30 when aligned over the full length consensus sequence, more preferably 30%, even more preferably 40%, even more preferably 50% and most preferably 60% and/or stems from a polypeptide of the present invention.

In yet another preferrred embodiment said fusion polypeptide comprises a fic-domain which shows at least 22% identity, preferably 25% identity over at least 100 aligned amino acids or at least 40% identity over 40 aligned amino acids to the consensus fic sequence set forth in Seq. Id. No. 31 and with a central conserved motif HPFXXGNG (Seq. Id. No. 36) in which at least 4 amino acids are identical.

A further preferred embodiment relates to a fusion polypeptide which further comprises a repeated tyrosine-containing domain having a conserved tyrosine residue and which shows at least 25%, preferably 40%, even more preferably 70% and most preferably 80% sequence identity over at least 12 aligned aminoacids to one of the motifs set forth in Seq. Id. Nos. 32 to 34.

In a preferred embodiment said VTP domain stems from a polypeptide of the present invention.

In another preferred embodiment said polypeptide to be translocated into a cell is fused N-terminally to said VTP domain or its functional fragment.

In a further aspect, the present invention provides a method for introducing polypeptides into eukaryotic cells. Said method comprises the following steps:
contacting eukaryotic cells with bacteria encoding a functional type IV secretion apparatus, said bacteria expressing a fusion protein to be introduced into said eukaryotic cells, wherein said fusion protein comprises (i) a VTP domain or a functional fragment thereof and (ii) a polypeptide to be translocated into said cells.

In yet another preferred embodiment said protein to be translocated is selected from the group consisting of an antibody, an antigen, an immun modulatory protein, an enzyme, a receptor protein, a signalling molecule, a structural protein, a fluorescent protein, a hormone and a toxin or portions thereof.

In a further preferred embodiment said eukaryotic cells are of human or animal origin, preferably mammalian cells, more preferably a cell type selected from the group consisting of endothelial cells, fibroblasts, epithelial cells, leukocytes and erythrocytes, most preferably endothelial cells.

In a preferred embodiment said fusion polypeptid to be introduced into the cytosol of cells is a fusion polypeptide of the present invention.

The method of the present invention for the introduction of polypeptides into eukaryotic cells does not depend on the specific on the specific structural features of the protein to be translocated and could be used with virtually any fusion protein and with a broad range of eukaryotic cells. Such a method is also suitable for a broad range of applications of the translocated proteins, such as e.g. screening.

In a further aspect the present invention provides a method for the identification of compounds which modulate the type IV protein secretion process into eukaryotic cells. Said method comprises the following steps:
contacting eukaryotic cells comprising a reporter system for the detection of protein translocation into the cytosol of said eukaryotic cells with
   (i) bacteria encoding a functional type IV secretion apparatus, said bacteria expressing a fusion protein to be introduced into said eukaryotic cells, wherein said fusion protein comprises (i) a VTP domain or a functional fragment thereof and (ii) a polypeptide showing a biological activity and
   (ii) a candidate compound and
   (iii) detecting compounds having a modulatory activity on the protein translocation process by comparing the read out of the reporter system contacted with the candidate compound to a control sample.

In a preferred embodiment said reporter system comprises a nucleic acid encoding a detectable protein wherein said detectable protein is only expressed when said fusion protein comprising said polypeptide with a biological activity is translocated into said eukaryotic cells.

In a further preferred embodiment said fusion protein further comprises C-terminal to the VTP domain or functional fragment thereof an amino acid motif which comprises at least three positively charged amino acids, preferably said motif comprises the amino acid sequence HRHHRRHH (Seq. Id. No. 35).

In yet another preferred embodiment said detectable protein is a fluorescent protein, preferably a GFP.

In a further preferred embodiment said nucleic acid sequence encoding said detectable protein is flanked by target sites for a recombinase and said polypeptide to be translocated has a recombinase activity recognizing said target sites.

In a further aspect the present invention is directed to the use of the polypeptides of the present invention or the nucleic acids of the present invention for the manipulation of cells, preferably mammalian cells, more preferably endothelial cells.

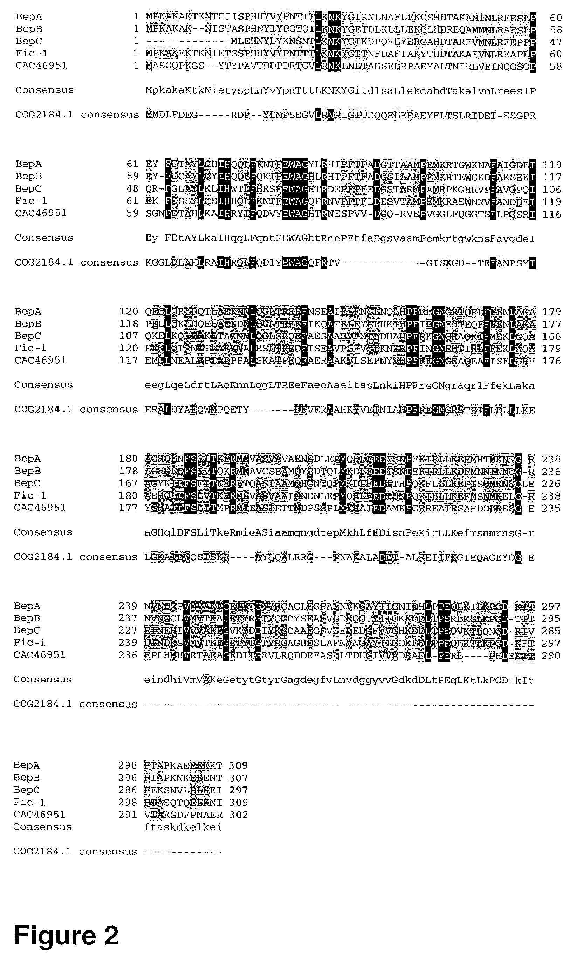

In a preferred embodiment said polypeptides for delivery into mammalian cells comprise a fic-domain (consensus defined in Fig. 2, Seq. Id. No. 31), with at least 22% identity over at least 100 aligned amino acids, preferably 25% over a least 100 aligned amino acids, or at least 40% identiy over at least 40 aligned amino acids. More preferably 35% identity fully (137 aa) aligned, even more preferably 50%. Moreover, a central conserved motif HPFXXGNG (Seq.Id. No. 36) motif has to be present, in which at least 4 positions have to be identical.

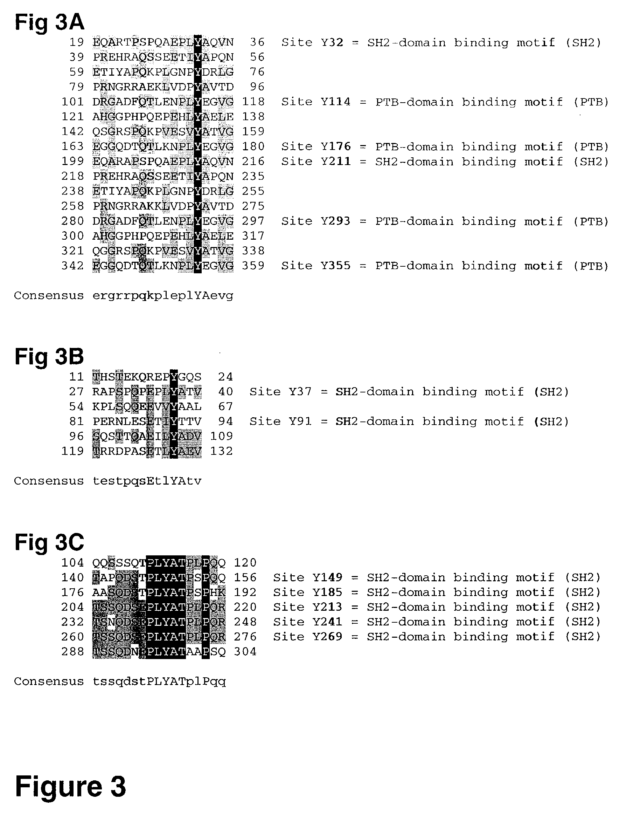

In a preferred embodiment said polypeptides for delivery into mammalian cells comprise a repeated tyrosine-containing-domain as present in BepD, BepE or BepF (consensus sequences defined in Fig. 3, Seq. Id. No. 32 to 34), with two or more repeats, which have at least the conserved tyrosine residue conserved and at least 25%, preferably 40% even more preferably 70%, most preferably 80% sequence identity to one of the motifs over a least 12 aligned amino acids.

In a further aspect the present invention is directed to the use of a polypeptide comprising a fic-1 domain and/or a repeated tyrosine-containing-domain or their homologoues as defined in the above paragraph as medicaments.

In a further aspect the present invention relates to the use of a polypeptide of the present invention and/or a nucleic acid of the present invention as a medicament.

In a further aspect the present invention provides a method for the generation of attenuated bacteria comprising the following steps:
introducing at least one mutation in at least one nucleic acid encoding a protein of the present invention and
selecting attenuated cells.
In a preferred embodiments said bacteria are selected from the genus *Bartonella*.

Said attenuated cells can be used as protein delivery device in ex vivo biotechnological applications e.g. for tissue engineering.

In yet another aspect the present invention provides an ex vivo method for the diagnosis of a bacterial infection comprising the following steps:
detecting in a body fluid and/or tissue sample of an individual a protein of the present invention or a nucleic acid of the present invention.

### Brief Description of the Drawings

The invention will be better understood and objects other than those set forth above will become apparent when consideration is given to the following detailed description thereof. Such description makes reference to the annexed drawings, wherein:
Figure 1 shows an alignment of VTP domains of BepA to BepG (Seq. Id. No. 3, 7, 9, 11, 13, 15, 17 and 18),
Figure 2 shows an alignment of the sequences locating N-terminal to the VTP domain of BepA, BepB, BepC (Seq. Id. No. 3, 7 and 9) and B. tribocorum Fic-1 (Seq. Id. No. 18) with the full-length protein sequence of a homologue of Sinorhizobium melioloti (Accession no. CAC46951), and the consensus sequence of the Fic (filamentation induced by c-AMP) domain deposited in the Conserved Domain Database (accession no. COG2184.1),
Figure 3 summarises the sequence and location of repeated tyrosine-containing sequence motifs in BepD (A), BepE (B) and BepF (C),
Figure 4 summarises the location of all identified domains and sequence motifs in the polypeptides sequences of B. tribocorum Fic-1 and BepA to BepG,
Figure 5 shows typrosine phosphorylation of BepD following translocation into Eahy926 cells by immunoprecipitation and Western blot analysis,
Figure 6 shows the results of a flow cytometer analysis of transfected Eahy926 tester cell line infected with B. henselae cells harboring expression plasmids encoding Cre-fusion proteins as well as quantification of GFP-positive cells,
Figure 7 summarises the percentages of apoptotic endothelial cells in dependency on the infecting B. henselae strain expressing BepA polypeptide,
Figure 8A shows modulation of the inflammatory reaction of endothelial cells as a result of VirB/VirD4-medidated translocation of the protein encoded by BepA,
Figure 8B shows activation of the transcription factor NF-κb by a VirB/VirD4-mediated process,
Figure 9 shows elevation of CAMP levels in endothelial cells as a result of VirB/VirD4-mediated translocation of the protein BepA,
Figure 10 shows changes in the induction in nuclear gene expression by a VirB/VirD4-dependent process of protein translocation,
Figure 11 shows cytoskeletal changes by the polypeptide BepF obtained by transfection of an eukaryotic transfection vector,
Figure 12 demonstrates antigenicity of proteins identified in the scope of the present invention by expression in B. henselae and immunoblotting with serum from a patient reconvalescent from B. henselae infection and

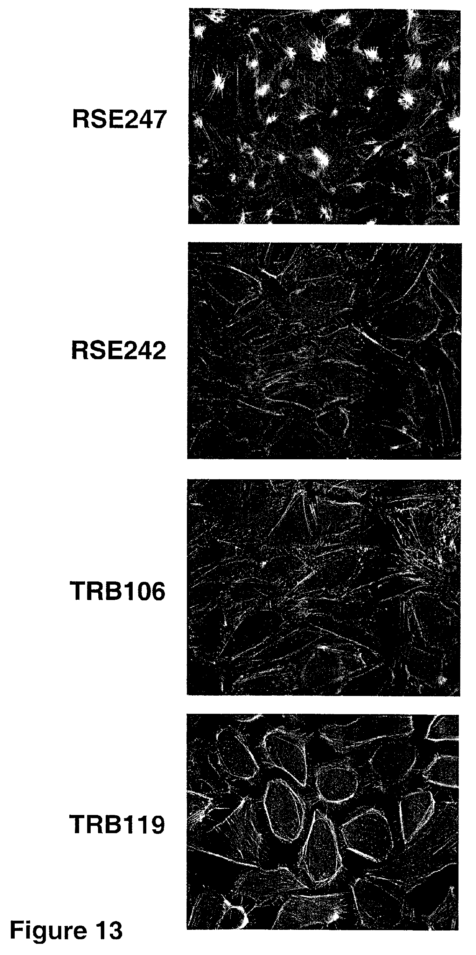

Figure 13 shows the results of a method to produce B. henselae strains with reduced virulence in vitro by deletion of the ORFs identified in the present invention.

### Detailed description of the invention

### Definitions:

As used herein, the terms defined below have the following meanings unless otherwise indicated: "Nucleic Acid Sequence": the term "nucleic acid sequence" includes both DNA and RNA unless otherwise specified, and, unless otherwise specified, includes both doublestranded and single-stranded nucleic acids. Also included are hybrids such as DNA RNA hybrids. In particular, a reference to DNA includes RNA that has either the equivalent base sequence except for the substitution of uracil and RNA for tymine in DNA, or has a complementary base sequence except for the substitution of uracil for tymine, complementarity being determined according to the Watson-Crick base pairing rules.

Reference to nucleic acids sequences can also include modified bases as long as the modifications do not significantly interfere weather with binding of a ligand such as a protein by the nuclei acid or with Watson-Crick base pairing.

In polypeptides, a corresponding amino acid is an amino acid according to the following table:
A, G;
S, T;
D, E;
N, Q;
H, R, K;
I, L, M, V; and
F, Y, W.

Fragments, variants or derivatives of the proteins of the present invention are also included in the invention. A fragment is a polypeptide having an amino acid sequence that entirely is the same as part, but not all, of the amino acid sequence of the aforementioned polypeptides. Fragments may be comprised within a larger polypeptide of which they form a part or region, preferably as a single continuous region.

The term VTP domain relates to a peptide domain having the following consensus sequence: "Lie-kEtlpPLsneEiAsrvqenalVqsyqqEiqhWCKIVYGNpyvLnkkieeIlkNPsl-GEeLswQvaenPkSfhKLAGfklLGiKnpARrhAEeglssLCdAiesYadaVkqa-redItqeHqaeqrRqaqsvekps" (Seq. Id. No. 30). Said consensus sequence was calculated from this alignment by the HMMER package [3]. The term VTP domain encompasses peptide domains which are at least 15% identical to the consensus of the VTP domain set forth in Seq. Id. No. 30 when aligned over the full length consensus sequence, more preferably 30%, even more preferably 40%, even more preferably 50% and most preferably 60%.

Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research 12(1): 387 (1984)), BLASTP, BLASTN, and FASTA (Atschul, S. F. et al., J. Molec. Biol. 215:403-410 (1990). The BLAST X program is publicly available from NCBI and other sources (BLAST Manual, Altschul, S., et. al., NCBI NLM NIH Bethesda, Md. 20894; Altschul, S., et al., J. Mol. Biol. 215: 403-410 (1990). The well known Smith Waterman algorithm and the ClustalX program may also be used to determine identity.

"Antibody": a used herein the term "antibody" includes both intact antibody molecule of the appropriate specificity, and antibody fragments (including Fab, F (ab'), Fv, and F (ab')2), as well as chemically modified intact antibody molecules and antibody fragments, including hybrid antibodies assembled by in vitro reassociation of subunits. Also include are single-chain antibody molecules generally denoted b the term sFv and humanised antibodies in which some or all of the originally non-human constant regions are replaced with constant regions originally derived from human antibody sequences. Both polyclonal and monoclonal antibodies are included unless otherwise specified. Additionally included are modified antibodies or antibodies conjugated to labels or other molecules that do not block or alter the binding capacity of the antibody.

The present invention is based on the discovery of genes, which encode polypeptides synthesised in a Gram-negative bacterium and being translocated via a pilus and pore complex comprising a type IV secretion apparatus into the cytoplasm of mammalian cells, where they affect changes in cell physiology. Inherent to these polypeptide sequences is the signal for protein translocation via the type IV secretion apparatus as well as bioactive peptide sequences affecting defined changes in cell physiology.

WO019535 describes an 8987 bp consecutive sequence of the B. henselae virB operon, which encodes 10 proteins (VirB2-11) forming a putative type IV secretion apparatus. We have recently reported the sequence and functional analysis of the homologous locus in the related species *B. tribocorum* including two additional downstream genes, which encode the proteins Fic-1 (Seq. Id. No. 18) and VirD4 [1]. By the generation of mutations in virB4 and virD4 we have demonstrated that the VirB/VirD4 type IV secretion apparatus of *B. tribocorum* is strictly required for infection in an animal model of bartonellosis [1].

Using PCR-primers derived from the B. tribocorum fic-1 gene a cosmid library of chromosomal DNA of B. henselae ATCC49882^{T} cloned into the vector pLAFR3 [2] has been screened and clones containing the B. henselae homologue of fic-1 (corresponding to the bepA gene of the invention) have been isolated.

DNA sequence analysis of the cosmid inserts revealed a consecutive sequence of 18895 bp (Seq. Id. No. 1) locating in the B. henselae chromosome immediately downstream of the nucleic acid sequence described in WO019535. This novel sequence encodes for eight proteins, designated BepA (Seq. Id. No. 3) [CDS 172-1806], VirD4 (Seq. Id. No. 5) [CDS 1969-3888], BepB (Seq. Id. No. 7) [CDS 4430-6058], BepC (Seq. Id. No. 9) [CDS 7128-8726], BepD (Seq. Id. No. 11) [CDS 9200-10804], BepE (Seq. Id. No. 13) [CDS 11130-12524], BepF (Seq. Id. No. 15) [CDS 12860-15364] and BepG (Seq. Id. No. 17) [CDS 15651-18680].

The corresponding nucleic acid sequences encoding the proteins of the present invention have the following designations:
DNA encoding protein BepA: Seq. Id. No. 2
DNA encoding protein BepB: Seq. Id. No. 6
DNA encoding protein BepC: Seq. Id. No. 8
DNA encoding protein BepD: Seq. Id. No. 10
DNA encoding protein BepE: Seq. Id. No. 12
DNA encoding protein BepF: Seq. Id. No. 14
DNA encoding protein BepG: Seq. Id. No. 16 The above mentioned DNA sequences do not only contain the coding sequences (CDS) for the proteins of the present invention but as well non-coding sequences i.e. protein BepA is encoded by the CDS of Seq. Id. No. 2.

VirD4 is a protein of 638 amino acids displaying 84% identity with the *B. tribocorum* VirD4 homologue [1] over 638 aligned amino acids. BepB (Seq. Id. No. 7) further displays high similarity (E-value 8e-95) to VirD4 proteins (spanning amino acids 46-637, CD-Length = 596 residues, 95.3% aligned) within the Conserved Domain Database (accession COG3505.1).

The 568 amino acid Fic-1 protein annotated in accession no. AJ315957 for the B. tribocorum fic-1 gene [1] appears to be a homologue of BepA (Seq. Id. No. 3), BepB (Seq. Id. No. 6) and BepC (Seq. Id. No. 9). Alignment of these four protein sequences, however, indicate that the identity of Fic-1 was incorrectly predicted as the methionine at amino acid position 25 appears to represent the correct N-terminal methionine. This notion is supported by the fact that a well conserved Shine-Dalgarno sequence is situated in front of this ATG (AGGA-GATGACAATG), while in the original incorrect annotation no conserved Shine-Dalgarno sequence is present (TTTA-GAAAAATATG). We have thus included the sequence of the presumably correct prediction of the Fic-1 protein in Seq. Id. No. 18. The Fic-1 protein is closely related to BepA (Seq. Id. No. 3) (544 amino, 63% identity over 544 aligned amino acids), BepB (Seq. Id. No. 7) (542, 55% identity over 544 aligned amino acids) and BepC (Seq. Id. No. 9) (532 amino acids, 41% identity over 529 aligned amino acids). Moreover, BepA, BepB and BepC display similar identities when compared pairwise.

All four proteins can be aligned with BepD (Seq. Id. No. 11) (534 amino acids), BepE (Seq. Id. No. 13) (464 amino acids), BepF (Seq. Id. No. 15) (834 amino acids) and BepG (Seq. Id. No. 17) (1009 amino acids) along a stretch of about 111 amino acids.

This conserved sequence designated VTP (for "VirB/VirD4-translocated protein")-domain is present in all proteins in the proximity of the C-terminus, while BepE contains one additional, BepF two additional and BepG three additional VTP domains. An alignment of all VTP domains is given in Fig. 1. The consensus sequence "LiekEtlpPLsneEiAsrvqenalVqsyqqEiqhWCKIVYGNpyvLnkkieeIl-kNPslGEeLswQvaenPkSfhKLAGfkILGiKnpARrhAEeglssLCdAiesYada-VkqaredItqeHqaeqrRqaqsvekps" was calculated from this alignment by the HMMER package [3] (Seq. Id. No. 30).

The proteins BepA, BepB, BepC and Fic-1 can further be fully aligned N-terminal to the VTP domain (Fig. 2). All four proteins contain a putative conserved domain termed Fic (accession COG2184.1, CD-Length = 201 residues) as defined in the Conserved Domain Database. In BepA the Fic domain spans amino acids 17-198 (76.6% aligned, E-value 4e-23), in BepB amino acids 15-199 with 81.1% aligned (E-value 4e-17) and in BepC amino acids 3-188 with 81.6% aligned (E-value 4e-21). Fig. 2 denotes also the consensus of the Fic-domain as retrieved from the COG2184.1 entry of the Fic-domain in the Conserved Domain Database. Moreover, a fic homologue in Sinorhizobium meliloti was found to fully align with the Fic-1 and BepA, BepB and BepC sequences aligned in Fig. 2, and a consensus sequence calculated by the HMMER package [3] (MpkakaKtkNietysphnYvYpnTttLKNKYGitdlsaLlekcahdTakalvnL-reeslPEyFDtAYLkaIHqqLFqntFEWAGhtRnePFtfaDgsvaamPemkrtgw-knsFavgdeIeegLqeLdrtLAeKnnLqgLTREeFaeeAaelfssLnkiHPFreGN-graqrlFfekLakaaGHqlDFSLiTkeRmieASiaamqngdtepMkhLfEDisnPe-KirLLKefmsnmrnsGreindhiVmVAKeGetytGtyrGagdegfvLnvdggyvvG-dkdDLtPEqLKtLkPGD-kItftaskdkelkei) (Seq. Id. No. 31) is provided.

Analysis of the peptide sequences encoded by BepD to BepF revealed numerous repeated peptide motifs which contain a conserved tyrosine residue. Fig. 3 shows an alignment of these repeat units as well as a consensus sequence calculated from this alignment by the HMMER package [3]. Scanning the peptide sequences of BepA-G with the software Scansite [4] identified different binding motifs for eukaryotic signalling domains primarily within these repeated peptide sequences, such as SH2-domain binding motifs (designated SH2), PTB (phosphoty-prosine-binding)-domain binding motifs (designated PTB) (Fig. 3) (Seq. Id. No. 32). Moreover, SH3-domain binding motifs (designated SH3), and PDZ-domain binding motifs (designated PDZ) have been identified as well (Seq. Id. No. 33 and 34).

Figure 3 summarises the location of all identified domains and sequence motifs in the polypeptide sequences of B. tribocorum Fic-1 and BepA to BepG.

In a further aspect the present invention relats to polypeptide which comprise a sequence which is at least 75% identical, more preferably at least 80% identical, even more preferably at least 90% identical and most preferably 100% identical to a sequence as set forth in Seq. Id. No. 3, 7 or 9.

Furthermore, the present invention relates to polypeptide comprising a sequence which is at least 30% identical, more preferably 50% identical, even more preferably 75% identical and most preferably 100% identical to an amino acid sequence selected from the group consisting of Seq. Id. No. 11 (BepD), 13 (BepE), 15 (BepF) and 17 (BepG) and to a polypeptide having the sequence set forth in Seq. Id. No. 18 (Fic-1).

In a further aspect, the invention provides a method for targeting proteins produced by Bartonella into the cytosol of mammalian cells comprises:
providing a nucleic acid segment encoding a fusion protein, the fusion protein including: (a) a composite translocation signal encoded by a nucleotide sequence identified herein or a homologue thereof and (b) a polypeptide to be translocated;
expressing the nucleic acid segment in Bartonella cells;
contacting the Bartonella cells expressing the nucleic acid segment with mammalian cells so that the fusion protein is translocated into the cytosol of the eukaryotic cell.

The polypeptide fused to the targeting signal can be selected from the group consisting of an antibody, an antigen, an immunomodulatory protein, an enzyme, a receptor protein, a signalling protein, a structural protein, a fluorescent protein, a hormone, and a toxin or portions thereof in order to provoke changes of the physiology of the target cell.

Generally, the bacterium used for the described method is Gram-negative and encodes a functional type IV secretion apparatus. Typically the bacterium used is pathogenic to mammalian hosts, including humans and animals. Preferably the bacterium used is a species of the genus Bartonella, including B. alsatica, B. bacilliformis, B. bovis, B. clarridgeiae, B. doshiae, B. elizabethae, B. grahamii, B. henselae, B. koelerae, B. quintana, B. schoenbuchensis, B. taylorii, B. tribocorum, B. vinsonni. Most preferably, the Bartonella species employed is B. henselae.

The mammalian cell contacted by this bacterium can be of human or animal origin, and can be in the context of the intact organism or of an isolated cell type in culture. Generally the mammalian cell can be any body cell, but preferably of a cell type selected from the group consisting of endothelial cells, fibroblasts, epithelial cells, leukocytes and erythrocytes. Most preferably, the targeted cell type is an endothelial cell, to which Bartonella species display a specific host tropism in vitro and in vivo.

Using an appropriate reporter system for protein translocation, the method can also be used to screen for compounds that block or inhibit the type IV secretion process of Gram-negative bacteria into mammalian cells. Generally this reporter system is based on a bioassay designed in a way that a positive readout of the biological activity of the translocatable reporter protein requires the intracellular localisation within mammalian cells. Typically, the bioactivity of the reporter system is encoded by the nucleotide sequences of the present invention, calmodulin dependent adenylate cyclase or recombinases. Preferably, the bioactivity of the translocated protein allows the recombination of a nucleic acid substrate previously introduced into a tester mammalian cell line. Generally, the readout of this bioassay is quantifiable. Typically this readout depends on light emission by luciferase or fluorescent proteins. Generally, the mammalian reporter cell can work in the context of a transgenic animal or as an isolated cell line in culture. Typically, the reporter cell is cultured. Preferably, the reporter cell is an endothelial cells. Most preferably, the reporter cell is an endothelial cell transfected with a recombinase substrate, which upon introduction of an exogenous recombinase recombines thereby resulting in the expression of a fluorescent protein, which can be quantified by appropriate techniques.

In a further aspect the invention provides a method for manipulating cells by bioactive sequences encoded by the nucleotide sequences identified herein or a homologue thereof comprises:
providing a nucleic acid segment encoding a bioactive peptide sequence
introducing the bioactive sequence into mammalian cells by (a) generating a fusion protein that can be translocated intracellularly, (b) any other method of intracellular delivery of peptide sequences or (c) introducing an expression vector that mediates the expression of the nucleic acid segment.

Typically, the bioactive peptides are selected to modulate cell proliferation, apoptosis, intracellular signalling processes, cytoskeletal rearrangements, nuclear transcriptional responses, inflammatory responses or antigen presentation by the mammalian target cell or to mediate cytotoxicity to those. Preferably, the bioactive peptides are chosen to modulate the physiology of endothelial cells, which are a target cell type for the peptides encoded by the nucleotide sequences identified herein.

According to another aspect of the invention, the nucleotide sequences identified herein or homologues thereof, as well as the thereby encoded polypeptides can be for therapeutic or diagnostic use, including use in vaccines for prophylactic application.

According to a further aspect of the invention, the genes encoding the nucleotide sequences identified herein or homologues thereof in related bacteria may be mutated to generate microorganism that lack virulence. Such attenuated bacteria can be used as life vaccine carriers or as intracellular delivery vehicles according to the second aspect of the invention.

The generation of molecular biology constructs used in the present invention and the generation of attenuated cells can be done using standard molecular biology techniques as described e.g. in Sambrook J. and Russell D.W., Molecular Cloning: A Laboratory Manual, New York: Cold Spring Harbor Laboratory Press, 2001).

The invention is now further illustrated by means of examples.

### Example 1: Translocation of epitope-tagged BepD (Seq. Id. No. 11) into mammalian cells by the VirB/VirD4 type IV secretion apparatus of B. henselae evidenced by means of demonstrating tyrosine phosphorylation

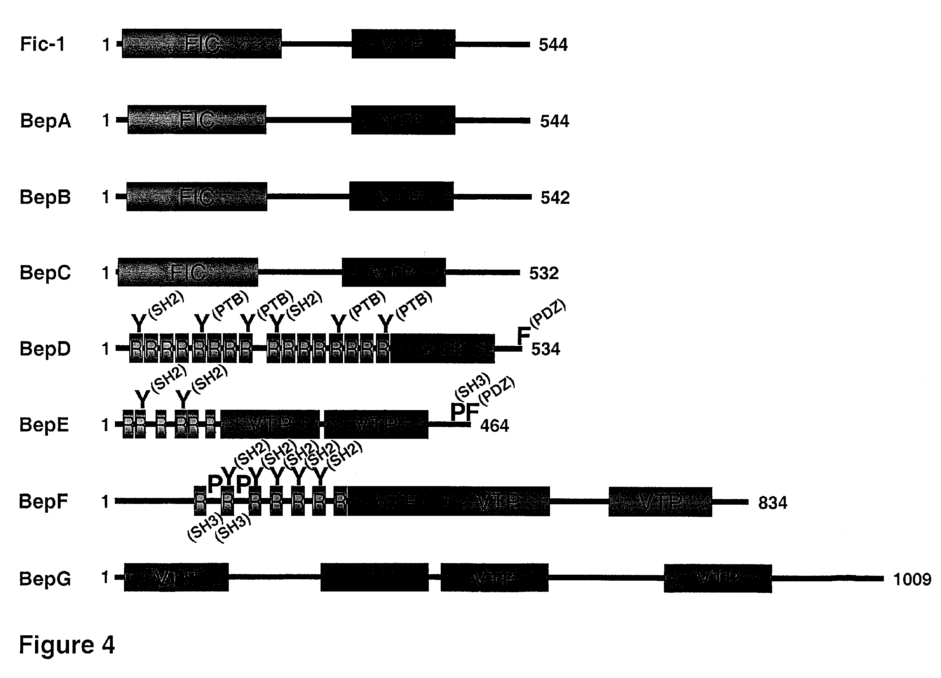

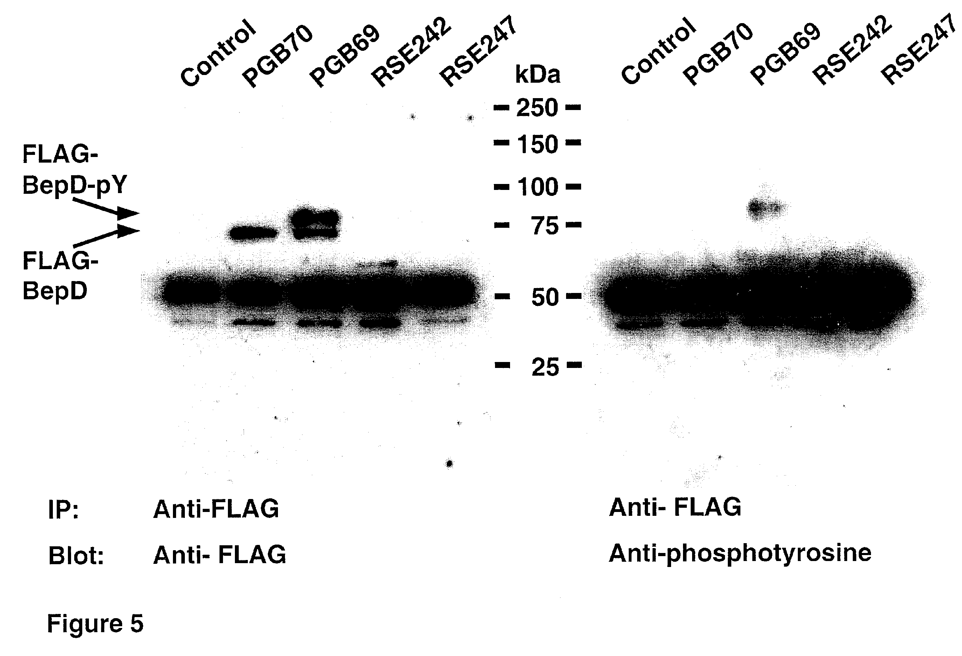

The peptide sequence of BepD (Seq. Id. No. 11) contains repeated tyrosine-containing peptide motifs (Fig. 3 and 4), which can be specifically phosphorylated by protein tyrosine kinases present in mammalian cells, while generally tyrosine phosphorylation does not occur in bacteria. A monoclonal antibody specific for phosphotyrosine was used to demonstrate tyrosine phosphorylation by immuno blot analysis [5]. In order to allow immunoprecipitation of the BepD polypeptide a FLAG-epitope (MDYKDDDDKH) was added to its N-terminus. Plasmid pPG104, a gentamicin-resistant derivative of pCHF03 [6], directs IPTG-inducible expression of FLAG-BepD in B. henselae. The endothelial cells line Eahy926 [7] was left uninfected (control) or infected with a multiplicity of infection of 200 bacteria per cell (MOI=200) of either B. henselae strain PGB70 (virB4 deletion mutant of 49882-SmR with plasmid pPG104), PGB69 (49882-SmR wild-type with plasmid pPG104), RSE242 (virB4 deletion mutant of 49882-SmR) or RSE247 (49882-SmR wild-type) in medium 199/10% fetal calf serum and 500 µM IPTG for 72 h at 35°C with 5% CO₂. Cell were detached with a cell scraper, harvested by centrifugation and lysed with RIPA-buffer in the presence of 1 mM orthovandate and protease inhibitors essentially as described [5]. Following centrifugation the cleared supernatants were incubated with anti-FLAG agarose (Anti-FLAG M2 Affinity Gel Freezer-Safe, Sigma) as described by the manufacturer. Following SDS-polyacrylamid gel electrophoresis and blotting to nitrocellulose membrane, immunoblotting was performed with either anti-Flag (Mab M2, Sigma) or antiphosphotyrosine (Mab G410, UBI) and secondary horseraddish-peroxidase conjugated goat-anti mouse antibody. Blots were developed by chemiluminescence according to standard procedures. As illustrated in Figure 5, this method revealed that similar amounts of FLAG-BepD are formed by strains PGB70 and PGB69 (left panel). In pPGB70, which is deleted for virB4 and therefore defective for the assembly of the VirB/VirD4 type IV secretion apparatus, only one band corresponding to FLAG-BepD is detectable, while PGB69 reveals a second band of increased molecular weight which corresponds to the translocated protein modified by tyrosine phosphorylation. Only this protein species of increased molecular weight is reactive with the anti-phosphotyrosine antibody as demonstrated in the right panel of Figure 5.

In conclusion, by means of mammalian cell-specific phosphorylation on tyrosine residues this method provides compelling evidence for translocation of BepD into the cytosol of mammalian cells in dependency of an intact VirB/VirD4 type IV secretion apparatus.

### Example 2: A method for translocation of a fusion protein composed of a protein to be translocated and a composite translocation signal encoded by nucleic acid sequences identified herein from the bacterium into mammalian cells using the VirB/VirD4 transfer system.

The method used is related to the invention in WO0189283 and a related publication [8] in that a specific recombination assay for translocation of a fusion protein composed of the recombinase Cre and the translocation domain in dependency of the VirB/VirD4 secretion apparatus of Agrobacterium tumefaciens is used. This invention, however, relates to plant cells as A. tumefaciens is a plant pathogen. Moreover, the polypeptides translocated by the A. tumefaciens VirB/VirD4 system, i.e. VirF, VirD2, VirE3 or MobA as well as there respective presumable translocation domains do not display obvious similarities with the BepA to BepG identified herein.

To test whether Cre-fusion proteins produced in B. henselae may translocate into mammalian cells we generated a mammalian recombination reporter cell line. To this end the mammalian transfection vector pRS56 was constructed. pRS56 is a derivative of the vector Cre stoplight [9], in which the DsRed gene was replaced by a neomycin phosphotransferase gene. pRS56 carries loxH and loxP sites, which upon Cre-mediated recombiation result in the expression of eGFP, which can be measured by flow cytometry or fluorescence microscopy. The endothelial cells line Eahy926 [7] was transfected with pRS56 using Effectene transfection reagent (Qiagen) as described by the manufacturer. By aid of the neomycin phosphotransferase gene encoded by pRS56 stable transfectants were isolated. Transfection with plasmid pBS185, a mammalian expression vector directing expression of Cre recombinase under control of the CMV promoter [9], demonstrated that eGFP expression results from Cre-mediated recombination.

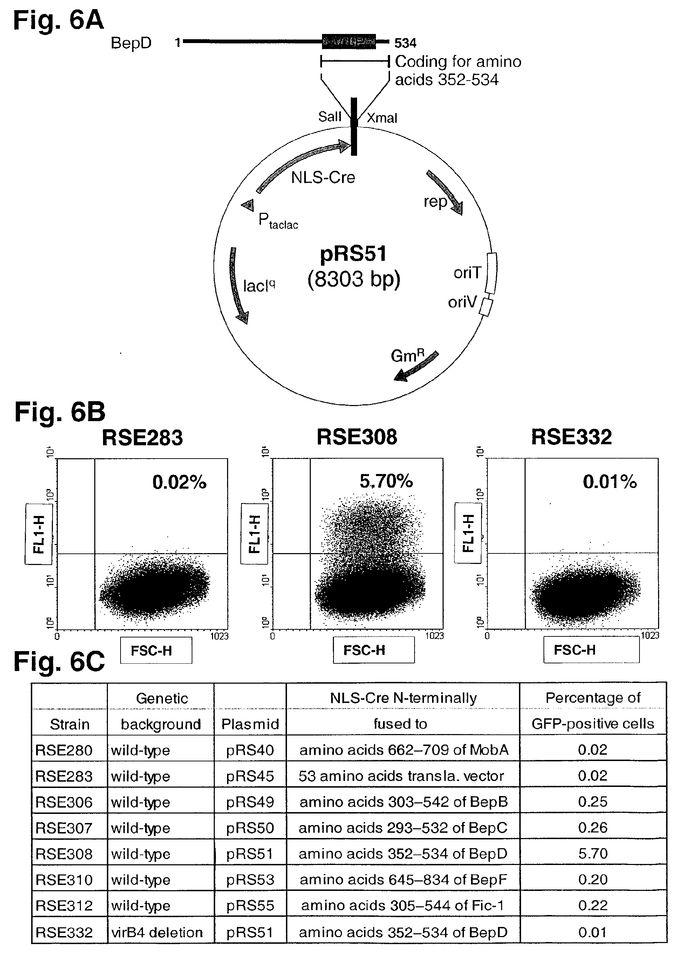

To mediate the production of Cre-fusion proteins in B. henselae the expression plasmid pRS40 was constructed. The backbone of this plasmid is based on the Bartonella-E. coli shuttle vector pCHF3 [6], which was modified by replacing the KmR gene by a GmR gene. Moreover, an expression cassette composed of the IPTG-inducible tac-lac promoter, and a fusion protein composed of an N-terminal nuclear localisation signal (NLS) from SV40, fused in-frame to the Cre-recombinase, fused in-frame to amino acids 662-709 (corresponding to the 48 C-terminal amino acids) of the mobilisation protein MobA from plasmid RSF1010 (accession no. NP_044304). Using SalI and XmaI sites all MobA sequences could be replaced by any sequence to be tested as C-terminal fusion to Cre, i.e. sequences identified herein encoding amino acids 303-542 of BepB (corresponding to the 240 C-terminal amino acids of Seq. ID. No. 7) in plasmid pRS49, amino acids 293-532 of BepC (corresponding to the 240 C-terminal amino acids of Seq. ID. No. 9) in plasmid pRS50, amino acids 352-534 of BepD (corresponding to the 183 C-terminal amino acids of Seq. ID. No. 11) in plasmid pRS51 (Fig. 6.A), amino acids 645-834 of BepF (corresponding to the 190 C-terminal amino acids of Seq. ID. No. 15) in plasmid pRS53 and amino acids 305-544 of B. tribocorum Fic-1 (corresponding to the 240 C-terminal amino acids of Seq. ID. No. 18) in plasmid pRS55. As an additional control to pRS40, plasmid pRS45 was constructed expression NLS-Cre with 53 C-terminal fused amino acids (SRPGCLAVYGG RPARHPPGRCFATFKSAPGGFVLLRRAFTDKQQIKRKAQSFD) (Seq. Id. No. 37) resulting from translation of respective vector sequences. This fused peptide sequence did not show any detectable similarity to protein sequences deposited in public data bases based on BlastP search. All plasmids were introduced in strains RSE247 (B. henselae 49882-SmR wild-type), and to test for VirB/VirD4-dependency pRS51 as well into strain RSE242 (virB4 deletion mutant of 49882-SmR). Eahy926 lines stably transfected with pRS46 were seeded in medium M199/10% fetal calf serum in 24 well plates and infected (MOI=200) with these strains for 72 h in the presence of 500 µM IPTG to induce expression of the Cre-fusion proteins. Then cells were detached by trypsin digestion and analysed for expression of GFP in a flow cytometer according to standard procedures.

Figure 6.B illustrates the results of the flow cytometric analysis as dot blots, and the table in Figure 6.C as quantification of GFP-positive cells. From the neglectable fraction of GFP-positive cells resulting from infection with strains RSE283 and RSE280 we conclude that fusions to the NLS-Cre C-terminus of 53 irrelevant amino acids or the 48 C-terminal amino acids of MobA, respectively, are not recognised by the VirB/VirD4 system of B. henselae as translocation signals. From the high proportion of GFP-positive cells (5.8%) of strain RSE308, however, we conclude that the C-terminal 183 amino acids (352-534) of BepD (Seq. Id. No. 11) fused C-terminally to NLS-Cre are sufficient to mediate efficient transfer of the fusion protein into the endothelial tester cell line. Dependency of this translocation process from the VirB/VirD4 secretion apparatus is illustrated by the negative result for GFP-positive cells (0.01%) in strain RSE322.

Fusions of the C-terminal end of BepB (Seq. Id. No. 7), BepC (Seq. Id. No. 9), BepF (Seq. Id. No. 15) and Fic-1 (Seq. Id. No. 18) were translocated into the tester endothelial cell line as well, although with a much lower frequency (see Figure 6.C). We note that all fusion constructs were of similar size and harboured a complete VTP-domain as defined in Figure 1. The increased translocation of the fusion of BepD sequences (pRS51) in comparison to BepB, BepC, BepF and Fic-1 may relate to a highly positively charged amino acid motif exclusively found in BepD (HRHHRRHH, amino acids 498-505), locating C-terminal to the VTP-domain (amino acids 360-496). The fact that the VirB/VirD4 translocation signal of B. tribocorum Fic-1 is active in B. henselae demonstrates interchangability of this signal across different species and is indicative for functional conservation of the VirB/VirD translocation system across species. It is thus likely that homologues of the BepA to BepG proteins identified herein and even so less related polypeptides translocated by a type IV secretion apparatus may be translocated by the B. henselae VirB/VirD4 system.

### Example 3: A method to demonstrate an anti-apoptotic activity by VirB/VirD4-mediated translocation of the protein encoded by BepA (Seq. Id. No. 3) in endothelial cells

A method is described which demonstrates that VirB/VirD4-dependent protein translocation by B. henselae can rescue endothelial cells from programmed cell death (apoptosis).

B. henselae has previously been demonstrated to rescue human umbilical vein endothelial cells (HUVEC) from apoptosis triggered by Actinomycin D [10]. In order to test if this anti-apoptotic activity is dependent on the VirB/VirD4 apparatus wild-type bacteria (strain RSE247) were compared with a virB4 deletion mutant (strain RSE242), as well as the virB4 deletion strain complemented by a plasmid expressing virB4 in trans (strain RSE364). To specifically test for the role of the BepA to BepG identified herein further deletion strains were included. Strain TR106 carries a deletion of bepB to bepG, thus only BepA remains among the VirB/VirD4-translocated proteins identified herein and can be translocated by the VirB/VirD4 apparatus. Strain TR119 carries a deletion of bepA to bepG, including virD4 locating between bepA and bepB. This strain is thus devoid of any of the sequences identified herein. In the method used HUVEC seeded in M199/10% fetal calf serum in 24-well dishes were infected with different bacterial strains (MOI=200) for 24h or left uninfected (control). Apoptosis was then induced by adding Actinomycin D to a final concentration of 100 nM. After 12 h cells were harvested with trypsin, and washed in cold PBS. The proportion of apoptotic cells was quantified by staining with Annexin V conjugated to Alexa Fluor 488 (Molecular Probes), followed by flow cytometric analysis using standard procedures and the specifications provided by the manufacturer. Necrotic cells were excluded from the analysis by counterstaining with propidium iodite.

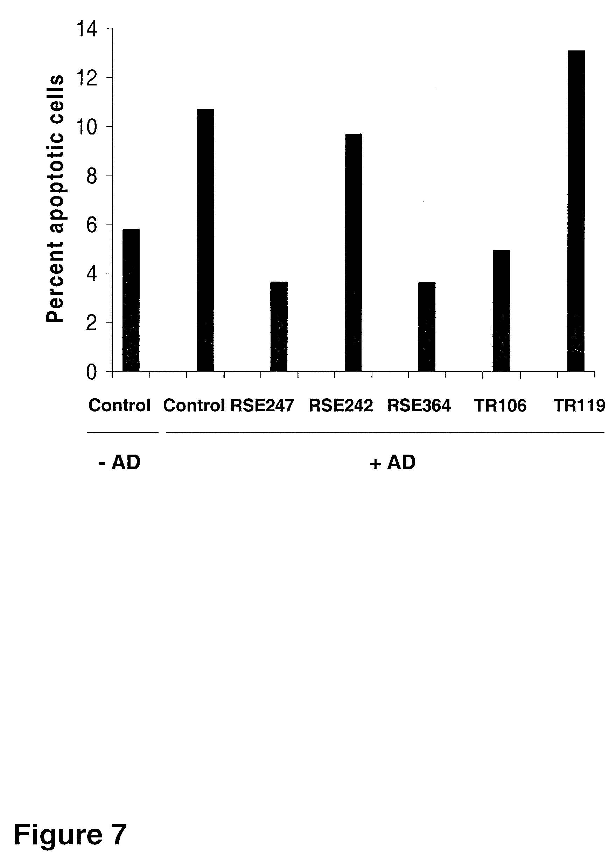

Figure 7 summarises the percentages of apoptotic cells in dependency on the infecting B. henselae strain as obtained by this method. Actinomycin D treatment (+AD) relative to no treatment (-AD) resulted in uninfected cells (control) in an approximately two-fold increase of the apoptotic cell population. Strain RSE247 could fully rescue Actinomycin D-triggered apoptosis and even resulted in partial suppression of apoptosis relative to untreated uninfected cells (control, -AD). In contrast, strain RSE242 did not interfere with Actinomycin D-triggered apoptosis, while RSE364 rescued as effectively as RSE247. From these data we conclude that the anti-apoptotic activity of B. henselae is mediated by a VirB/VirD4-dependent process.

Strain TR106 rescued from Acinomycin D-triggered apoptosis as effective as RSE247, while TR119 was ineffective as RSE242. From these data we conclude that among the sequences identified herein at least BepA mediates anti-apoptosis upon translocation by a VirB/VirD4-dependent process. Given the high degree of conservation with BepB, BepC as well as Fic-1 of B. tribocorum, it is likely that the proteins encoded may exert a similar modulatory effect on apoptotic processes in mammalian cells. Moreover, the Fic-domain conserved in these proteins as well as many other proteins (accession COG2184.1 in the Conserved Domain Database), and/or the VTP-domain identified for BepA to BepG herein (see Figure 1) may be related to this anti-apoptotic activitiy.

### Example 4: A method to demonstrate modulation of the inflammatory reaction of endothelial cells as a result of VirB/VirD4-mediated translocation of the protein encoded by BepA

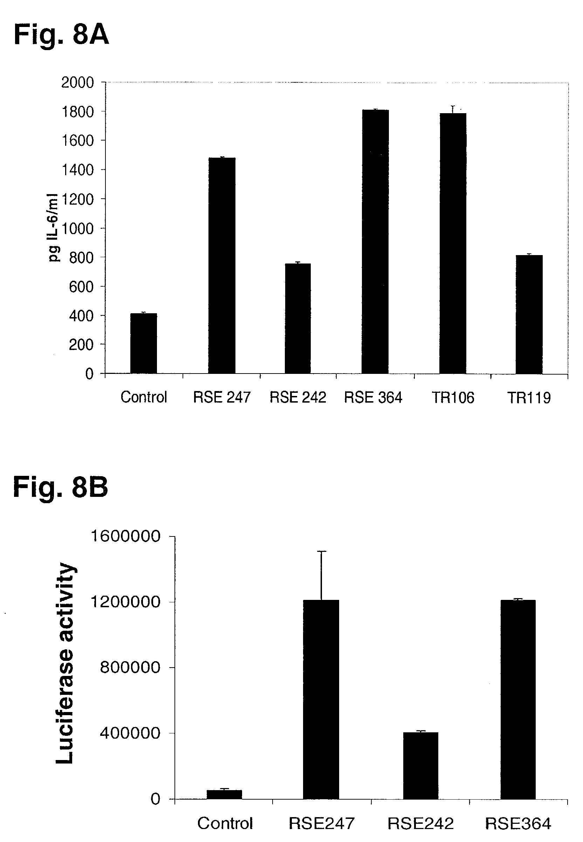

The pro-inflammatory activation of endothelial cells by nuclear translocation of the transcription factor NF-KB resulting in the production of the pro-inflammatory cytokines IL-6, IL-8 and interferon gamma, as well as the surface expression of the adhesion molecules ICAM-1 and E-selectin, is crucial for the induction of an inflammatory response. A method is described to demonstrate that the pro-inflammatory cytokine IL-6 is produced in response to VirB/VirD-4 dependent protein translocation by B. henselae. HUVEC seeded in M199/10% fetal calf serum in 6-well plates were infected with different bacterial strains (see Example 3, MOI=600) for 54 h or left uninfected (control). Using standard procedures, the culture supernatant was then harvested and IL-6 was quantified by a sandwich ELISA (Duo Set ELISA development kit DY206 from R&D Systems) according to the specifications of the manufacturer. The IL-6 amounts measured by this method in dependency of the infecting B. henselae strain are summarised in Figure 8.A. Infection with strain RSE247 results in an approximately 3-fold increase in IL-6 amounts compared to uninfected cells (control), while infection with RSE242 does cause only a moderate increase relative to uninfected control. RSE364 behaves like RSE242. From these data we conclude that in B. henselae a VirB/VirD-dependent process mediates a specific increase of IL-6 production. Furthermore, in contrast to infection with TRB106, infection with TRB119 did not result in an increase of IL-6 levels over the level observed for RSE242. We thus conclude that among the sequences identified in this invention at least BepA can mediate increased production of IL-6 levels in endothelial cells upon VirB/VirD4-dependent translocation. Given the high degree of conservation with BepB, BepC and Fic-1 of B. tribocorum, it is likely that the protein encoded may exert a similar modulatory effect on CAMP-levels and the respective signalling pathways affected by these changes in mammalian cells. Moreover, the Fic-domain conserved in these proteins as well as many other proteins (accession COG2184.1 in the Conserved Domain Database), and/or the VTP-domain identified for BepA to BepG herein (see Figure 1) may be related to this interference with inflammatory signalling.

In a related method activation of the transcription factor NF-KB by a VirB/VirD4-mediated process was demonstrated. To this end the human embryonic kidney cell line HEK293T was transfected by the calcium phosphate co-precipitation method with the reporter plasmid pNFconluc, bearing a consensus promotor region from NF-KB fused to the luciferase reporter gene, and pEF61acZ, a plasmid bearing a lacZ gene under control of the human elongation factor 1 α (hEF-1α) subunit promoter. 24 h later the cells were infected with different bacterial strains (MOI= 200) or left uninfected (control). 24 h later cell lysates were prepared and NF-kB dependent luciferase gene expression was measured and normalized for beta-galactosidase activity as described [11]. The results obtained for NF-KB activation by a VirB/VirD4-dependent process depicted in Figure 8.B are consistent with the data obtained for activation of IL-6 production (Figure 8.A) as discussed above.

Taken together, the methods employed in example 4 demonstrate a specific activation of the nuclear transcription factor NF-KB and the resulting production of IL-6 as a consequence of a VirB/VirD4-mediated process, particularly by the translocation of the protein encoded by BepA or related polypeptides. Moreover, these results demonstrate that both primary human endothelial cells (HUVEC) as well as the human embryonic kidney cell line HEK293T can be used as mammalian target cell for VirB/VirD4-mediated translocation of protein sequences identified herein.

### Example 5: A method to demonstrate elevated cAMP levels in endothelial as a result of VirB/VirD4-mediated translocation of the protein encoded by BepA

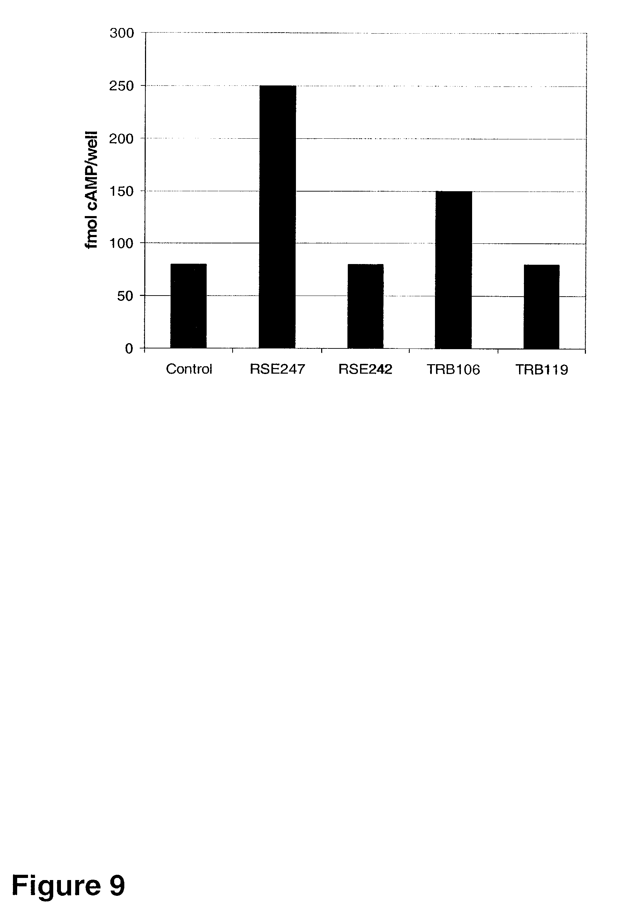

Cyclic AMP (CAMP) is a central signalling molecule in various signal transduction processes in mammalian cells. A method is described to demonstrate that the intracellular CAMP level in mammalian cells is changed in response to VirB/VirD-4 dependent protein translocation by B. henselae. HUVEC seeded in M199/10% fetal calf serum in 48-well plates were infected with different bacterial strains (see Example 4, MOI=600) for 30 h or left uninfected (control). Then cells were lysed and the intracellular CAMP level was determined by the enzyme immunoassay (EIA) CAMP Biotrack (RPN225, Amersham) essentially as described [12] using standard procedures and the specific information provided by the manufacturer. The CAMP amounts measured by this method in dependency of the infecting B. henselae strain are summarised in Figure 9. Infection with strain RSE247 results in an approximately 3-fold increase in the CAMP amount compared to uninfected cells (control), while infection with RSE242 does not cause any increase in CAMP. From these data we conclude that B. henselae mediates an increase in the CAMP level in infected endothelial cells by a VirB/VirD-dependent process. Furthermore, in contrast to infection with TRB106, infection with TRB119 did not result in an increase of CAMP levels. We thus conclude that among the sequences identified herein at least BepA can mediate increased CAMP levels in endothelial cells upon VirB/VirD4-dependent translocation. Given the high degree of conservation with BepB, BepC and Fic-1 of B. tribocorum, it is likely that the protein encoded may exert a similar modulatory effect on CAMP-levels and the respective signalling pathways affected by these changes in mammalian cells. Moreover, the Fic-domain conserved in these proteins as well as many other proteins (accession COG2184.1 in the Conserved Domain Database), and/or the VTP-domain identified for BepA to BepG herein (see Figure 1) may be related to this interference with cellular signalling.

### Example 6: A method to induce changes in nuclear gene expression by a VirB/VirD4 -dependent process of protein translocation

A method is described to monitor changes in nuclear gene expression as a result of VirB/VirD4-dependent protein translocation. HUVEC seeded in M199/10% fetal calf serum in 75 cm² cell culture flasks were infected (MOI=600) with wild-type B. henselae (strain RSE247) or the virB4 deletion mutant (RSE242), or left uninfected (control) for 30 h. Employing standard procedures the cell were then harvested, total RNA was isolated, reverse transcribed by oligo-dT primers and subjected to quantitative PCR using an ABI Prism 7000 Sequence Detection System. The following genes and respective PCR-primers were chosen: (a) cyclic AMP-responsive element modulator (designated CREM), accession no. NM_001881/ D14826/ U44836, ATCGCCCGGAAGTTTGC (forward primer) (Seq. Id. No. 19) , CAGCTCTCGTTTGCGTGTTG (reverse primer) (Seq. Id. No. 20), (b) chemokine (C-X-C motif) receptor 4 (designated CXCR4), accession no. AJ224869/AF348491/L01639, TATGCTTTCCTTGGAGCCAAA (forward primer) (Seq. Id. No. 21), GCTGGACCCTCTGCTCACA (reverse primer) (Seq. Id.No. 22), (c) angiopoietin 2 (designated ANGPT2), accession no. NM_001147/ AF187858, CGCTCGAATACGATGACTCG (forward primer) (Seq. Id. No. 23), CCACTGAGTGTTGTTTTCCA-TGAT (reverse primer) (Seq. Id. No. 24), (d) glyceraldehyde-3-phosphate dehydrogenase (designated GAPDH), accession no. NM_002046, GAAGGTGAAGGTCGGAGTC (forward primer) (Seq. Id. No. 25) , GAAGATGGTGATGGGATTTC (reverse primer) (Seq. Id. No. 26). In the 2(-Delta Delta C(T)) Method the Delta C(T) values are determined by subtracting the average GAPDH C(T) value from the average (test gene) C(T) value as previously described [13].

The data obtained by this method are summarised in Figure 8. Compared to uninfected cells (control), cells infected with RSE247 displayed a clear induction of steady-state transcript levels for CREM, CXCR4 and ANGPT2. In contrast, cells infected with RSE242 display either comparable steady-state transcript levels to uninfected control cells (i.e. CREM), or only moderately increased levels compared to RSE247 (i.e. CXCR4 and ANGPT2). We conclude that B. henselae modulates the expression of discrete genes in a VirB/VirD4-dependent manner. Together with the assumption that these processes are generally triggered by the translocated proteins described in this invention, we conclude those sequences described herein have the capacity to modulated nuclear gene expression. The increased cAMP-levels resulting from the translocation of BepA as described in example 5 are consistent with increased expression of the cAMP-responsive gene CREM (cyclic AMP-responsive element modulator) as demonstrated in this example expression as an VirB/VirD4-dependent process. The nuclear transcription factor NF-KB-dependent induction of IL-6 production as a consequence of BepA translocation has already been described as another example of a specific modulation of nuclear gene expression (see example 4) by sequences described herein.

### Example 7: A method to provoke cytoskeletal changes by BepF obtained by transfection with an eukaryotic transfection vector

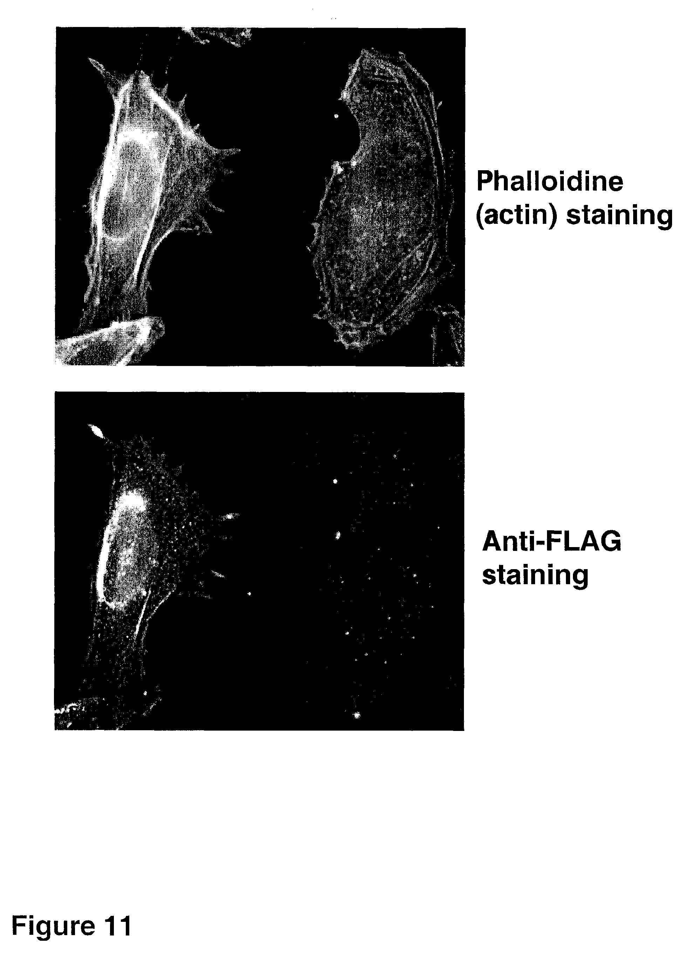

A method is described to provoke cytoskeletal changes by expression of BepF following transfection of a mammalian expression vector. In order to allow specific immunocytochemial detection of transfected cells a FLAG-epitope (MDYKDDDDKH) was introduced at the N-terminus of BepF. The transfection vector pRT1175 is a derivate of the mammalian expression vector pFLAG-CMV2 (Sigma) allowing expression of FLAG-BepF in mammalian cells by the CMV-promoter. Following transfection of the line Eahy926 [7] by Effectene (Quiagen) according to the specification of the manufaturer, cells were incubated for 24 h before they were fixed with 3.7% paraformaldehyde and stained for the FLAG-epitope (Mab M2, Sigma) and cellular actin (TRITC-conjugated phalloidine, Sigma) by standard procedures essentially as described [14]. Figure 11 illustrates an example of a transfected cell (left) next to an non-transfected cell (right). Discrete changes in the actin cytoskeleton are observed in dependency to the expression of FLAG-BepF. We conclude form these data that BepF provokes discrete changes in the architecture of the cellular actin cytoskeleton.

### Example 8: Demonstration of antigenicity of proteins encoded by sequences identified herein by expression in B. henselae and immunoblotting with serum from a patient reconvalescent from B. henselae infection

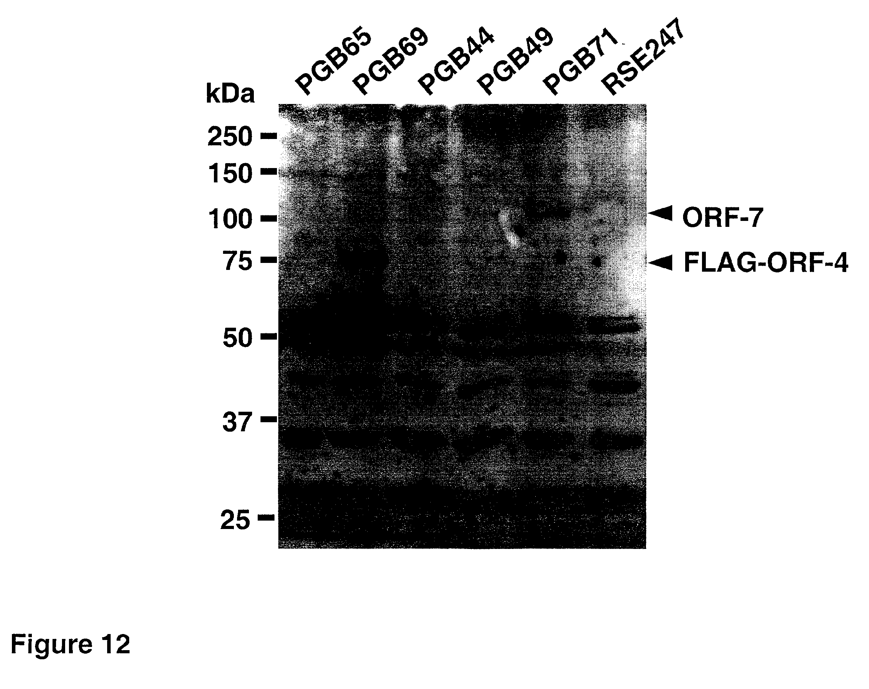

A method is described which illustrates that the serum of patient reconvalescent from B. henselae infection contains antibodies directed against proteins encoded by the sequences identified herein. The method is also useful to express proteins encoded by these sequences in either E. coli or B. henselae. Expression plasmids directing IPTG-inducible expression of FLAG-epitope tagged BepA, BepE and BepF were generated similarly as described for FLAG-BepD in example 1. To this end the BepD sequences contained in pPG104 were exchanged as an NdeI-fragment against sequences encoding either BepA (pPG101), BepE (pPG105) or BepF (pPG106). BepG was cloned into pPG107 in a way that protein translations begins at the native ATG start codon, resulting in the native protein without N-terminal FLAG. All plasmids were introduced into B. henselae wild-type 49882-SmR by standard methods, resulting in strain PGB65 (containing PGB101), PGB69 (containing PGB104), PGB44 (containing PGB105), PGB49 (containing PGB106) and PGB71 (containing PGB107). All strains were cultured on Columbia blood agar containing 5% sheep blood and 500 µM IPTG (the latter to mediate expression of recombinant FLAG-BepA, FLAG-BepD, FLAG-BepE, FLAG-BepF and native BepG) for 3 days before harvesting in phosphate buffered saline. Using standard procedures, total cell lysates were prepared and separated by SDS-polyacrylamid gel electrophoresis, followed by transfer onto nitrocellulose membrane. The blot was incubated with a 1:100 dilution of serum from a patient reconvalescent for B. henselae infection (antibody titer to B. henselae of 1:1024 as measured by standard immunofluorescence tests). Horseraddish-peroxidase conjugated goat anti-human IgG antibodies and chemoluminescence detection according to standard procedures was used to detect B. henselae antigens reactive with the patient serum. Figure 12 demonstrates the presence of specific antibodies reactive with FLAG-BepD and BepG. This method demonstrates that among the sequences identified in this invention at least the BepD and BepG proteins are expressed during human infection with B. henselae and represent antigens triggering the production of specific IgG antibodies. The sequences identified herein are thus relevant for diagnostic use, and may be useful as well for therapeutic use, including representing targets for vaccine development.

### Example 9: A method to produce B. henselae strains with reduced virulence by the chromosomal deletion of DNA sequences identified herein

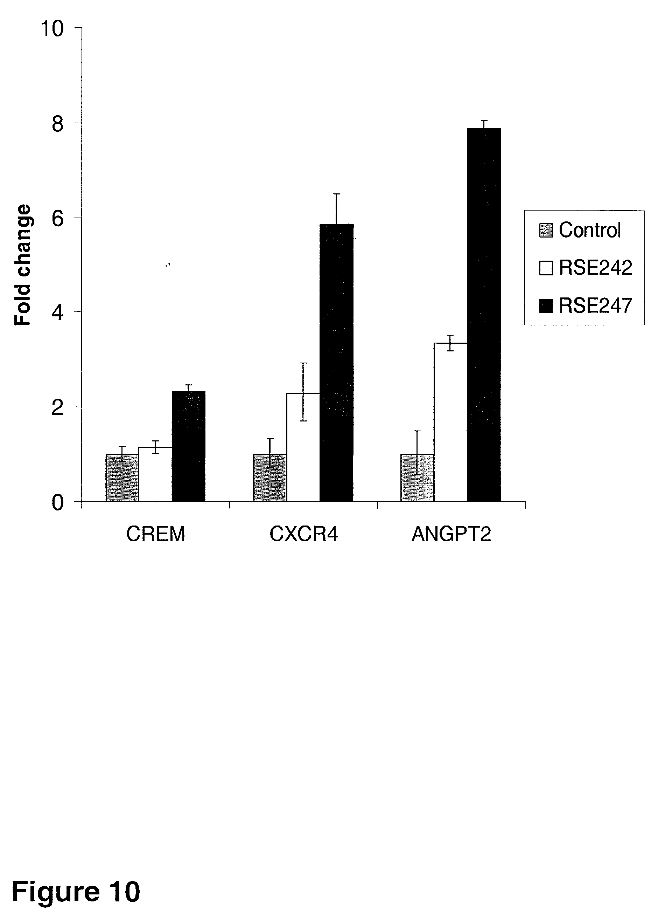

For application of B. henselae as a protein delivery device in human therapeutic infection using a method a described in example 2, it may be desirable to have attenuated strains of B. henselae which are less virulent or avirulent. For application of B. henselae as a protein delivery device in biotechnological applications (ex vivo, i.e. for tissue engineering) it may as well be useful to prevent undesirable effects mediated by the translocation of endogenous substrates of the VirB/VirD4 apparatus. To this end a method is described allowing the generation of deletion mutants of the sequences as described herein. The method is based on applying the genetic system originally described for B. tribocorum [1] for generating mutations in B. henselae. The method was used to generate deletions of the region spanning bepA, bepB, bepC, bepD, bepE, bepF and bepG resulting in strain TRB106, as well as the region spanning bepA, virD4, bepB, bepC, bepD, bepE, bepF and bepG resulting in strain TRB119. By the same method the virB4 gene in the adjacent virB-operon was deleted in-frame (without generating polar effects on the expression of downstream genes in the virB operon) resulting in strain RSE242. In order to test for the effect of these deletion strains infections of endothelial cells have been performed, which are partially included in previous examples of this invention and illustrated in Figs. 6 to 10. Infection of Eahy926 cells [7] with these deletions strains as well as wild-type (RSE247) as described before (see Example 3) for 72 h was followed by fixation in 3.7% paraformaldehyde and staining for cellular actin with TRITC-conjugated phalloidine (Sigma) by standard procedures essentially as described [14]. Figure 11 illustrates that the major cytosekeletal rearrangements (i.e. the invasome structure described by Dehio et al. [Dehio, 1997 #95), which is mediated by the VirB/VirD4 apparatus in wild-type, is not resulting from infection by any of the mutant bacteria. RSE242 as well as TRB119 are defective in components of the VirB/VirD4 system, along with a deletion of all substrates identified herein in TRB119. In contrast, TRB106 expresses a functional VirB/VirD4 apparatus as well as thereby translocates the protein encoded BepA. The reduced virulence of this strain in regard of major cytoskeletal rearrangements as illustrated in this example demonstrates that deletion of sequences identified in this invention are useful in order to reduce the virulence of B. henselae in vitro and likely as well as in vivo.

### Material and Methods:

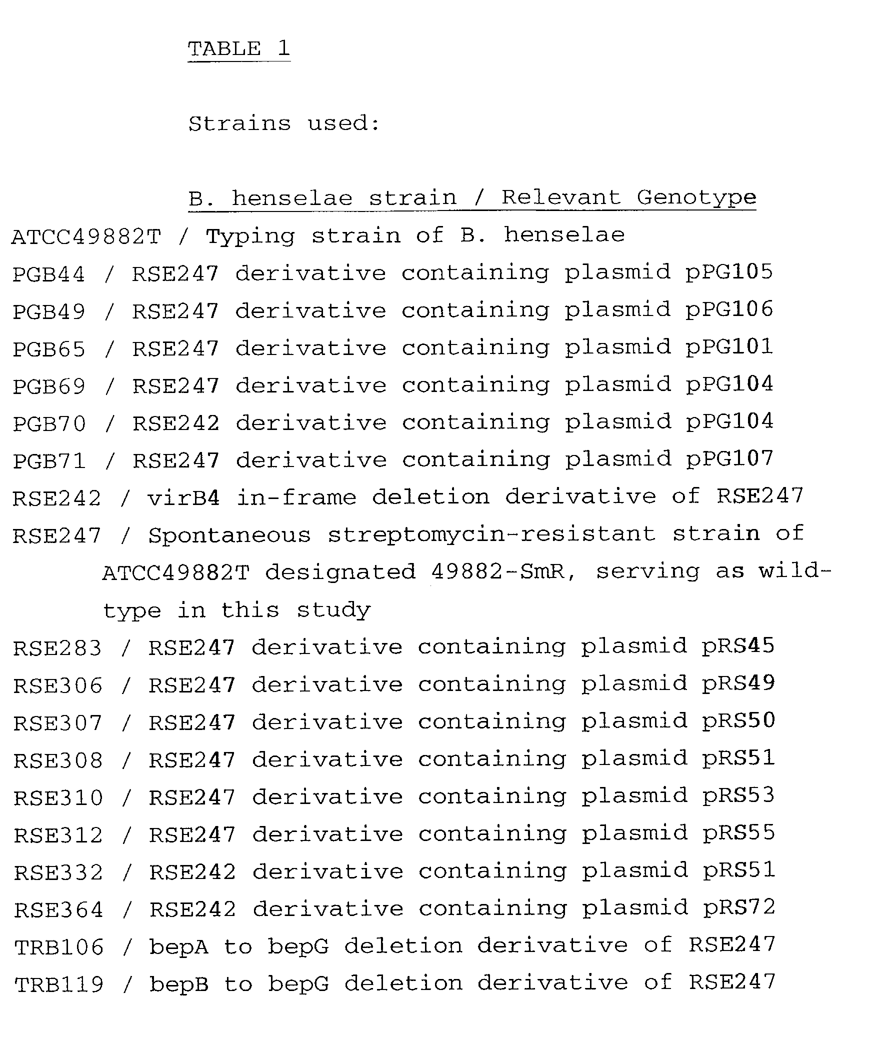

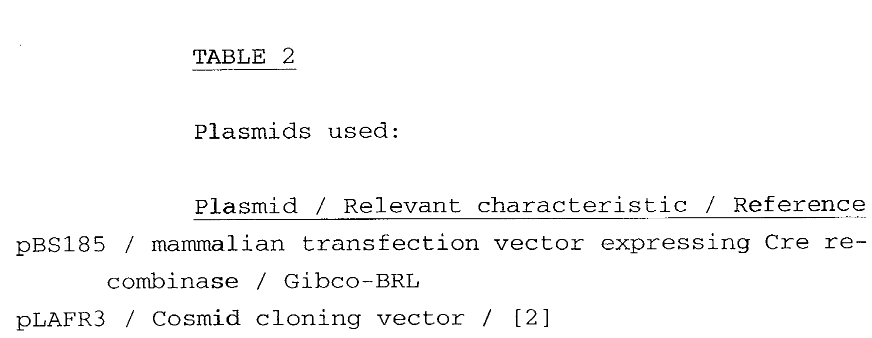

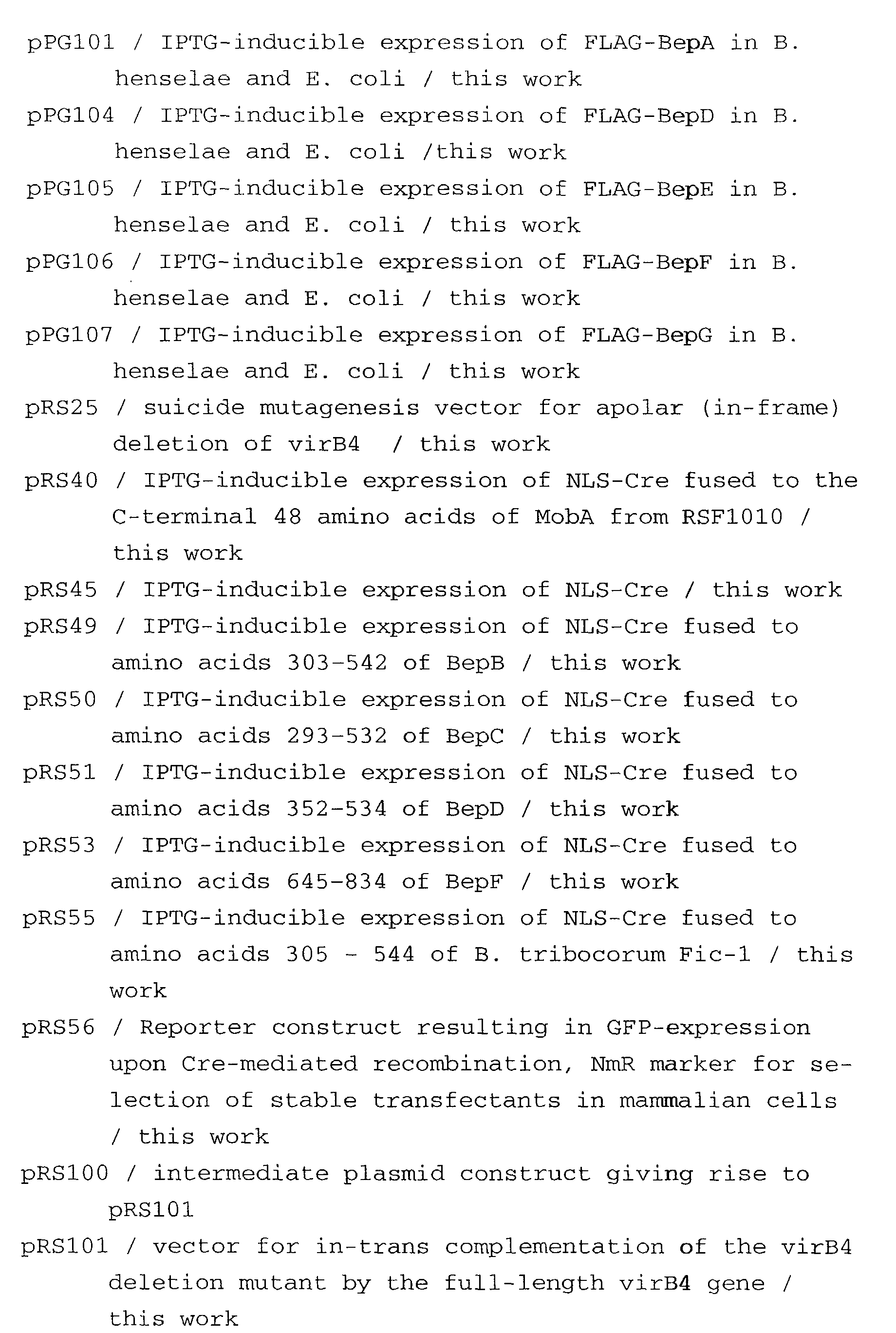

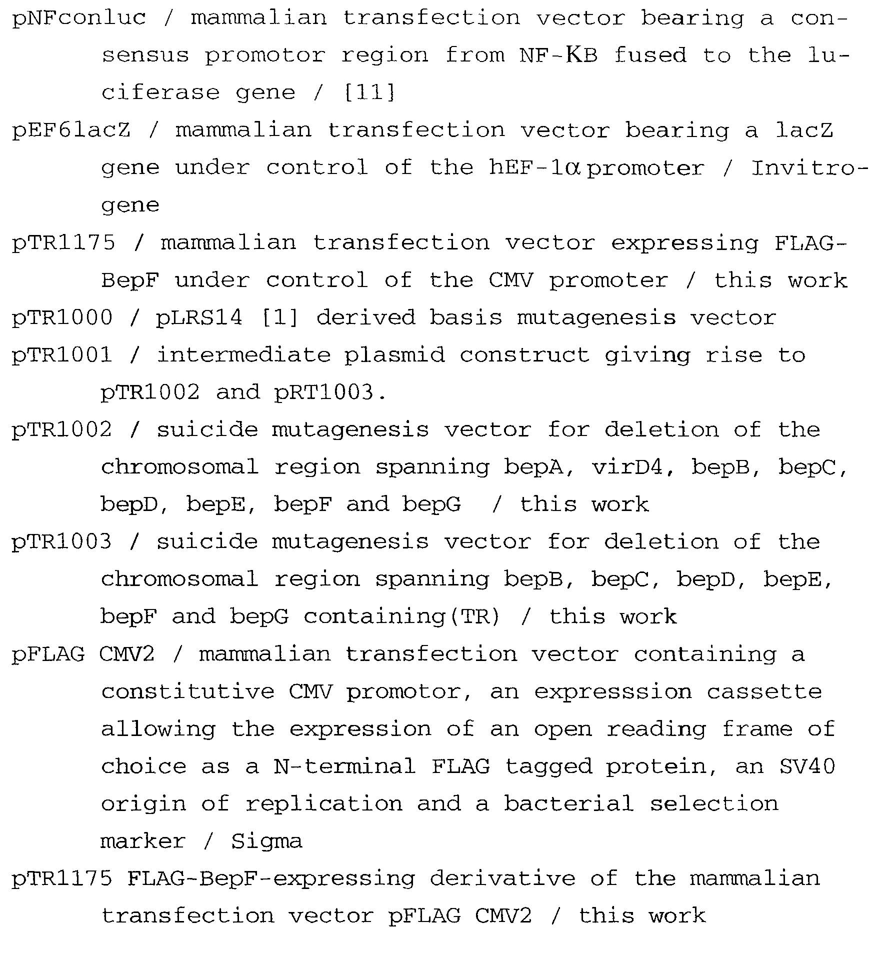

### Materials and methods:

### Bacterial strains:

B. henselae strains used herein are listed in Table 1. B. tribocorum IBS 506T was used as well. E. coli Novablue was used as cloning strain, and beta2150 harbouring various plasmids, i.e. as specified in Table 2 was used as donor for conjugation with B. henselae strains [1,15,16].

### Bacterial culture:

E. coli, B. henselae and B. tribocorum strains were cultured as described previously [1,14].

### Plasmid constructions:

Plasmids used herein are listed in Table 2.
- pPG100: was constructed by exchanging the SacI-Insert of pRS40 with a fragment of 387 bp amplified from pRS40 with the primers prPG89 (CGCGAGCTCTTTAAGAAGGAGATATACAAATGGACTATAAAGACGACGATGACAAACATATGGGATGCCTGGCAGTTTAT) (Seq. Id. No. 38) and prPG91(CGCGAGCTCTAAA TCAGAACGCAGAAGCG) (Seq. Id. No. 39).
- pPG101: was constructed as follows: bepA was amplified with primers prPG92 (GGAATTCCATATGCCAAAGGCAAAAGCAAAAA) Seq. Id. No. 40) and prPG93 (GGAATTCCATATGTTAGCTA GCCATGGCAAGC) (Seq. Id. No. 41). The resulting fragment was digested with NdeI and cloned into the NdeI-site of pPG100. The correct insertion direction was checked by sequencing with m13rev (GGAAACAGCTATGACCATG) (Seq. Id. No. 42). The resulting construct expresses the complete BepA fused to an N-terminal FLAG-tag ("MDYKDDDDKH") (Seq. Id. No. 43) , giving rise to FLAG-BepA.
- pPG104: was constructed as follows: bepD was amplified with primers prPG98 (GGAATTCCATATGAAAAAAAATCGACCATCCC) (Seq. Id. No. 44) and prPG99 (GGAATTCCATATGTTACATACCAAAGGCCATTCC) (Seq. Id. No. 45). The resulting Fragment was digested with NdeI and cloned into the NdeI-site of pPG100. The correct insertion direction was checked by sequencing with m13rev (GGAAACAGCTATGACCATG) (Seq. Id. No. 42). The resulting construct expresses full-length BepD fused to an N-terminal FLAG-tag ("MDYKDDDDKH") (Seq. Id. No. 43), giving rise to FLAG-BepD.
- pPG105: was constructed as follows: bepE was amplified with primers prPG100 (GGAATTCCATATGAAAAGAAATCAACCACCC) (Seq. Id. No. 46) and prPG101 (GGAATTCCATATGTTAG ATGGCGAAAGCTATTGC) (Seq. Id. No. 47). The resulting Fragment was digested with NdeI and cloned into the Ndel-site of pPG100. The correct insertion direction was checked by sequencing with m13rev (GGAAACAGCTATGACCATG) (Seq. Id. No. 42). The resulting construct expresses full-length BepE fused to an N-terminal FLAG-tag ("MDYKDDDDKH") (Seq. Id. No. 43), giving rise to FLAG-BepE.
- pPG106: was constructed as follows: bepF was amplified with primers prPG102 (GGAATTCCATATGAAAAAAAACCAACCATCCT) (Seq. Id. No. 48) and prPG103 (GGAATTCCATATGTTAGAG TGCCAGCACCATTT) (Seq. Id. No. 49). The resulting Fragment was digested with NdeI and cloned into the NdeI-site of pPG100. The right insertion direction was checked by sequencing with m13rev (GGAAACAGCTATGACCATG) (Seq. Id. No. 42). The resulting construct expresses full-length BepF fused to an N-terminal FLAG-tag ("MDYKDDDDKH") (Seq. Id. No. 43), given rise to FLAG-BepF.
- pPG107: was constructed as follows: bepG was amplified with the primers prPG104 (CGCGCTTATTAATGGACATTCTCTC TGAAAGG) (Seq. Id. No. 50) and prPG105 (CGCGCTTATTAATTTATCTACTCATAGAAACTACTT) (Seq. Id. No. 51). The resulting Fragment was digested with NdeI and cloned into the NdeI-site of pPG100. The correct insertion direction was checked by sequencing with m13rev (GGAAACAGCTATGACCATG) (Seq. Id. No. 42). The resulting construct expresses native BepG.
- pRS25: used for generating the B. henselae virB4 deletion mutant derived by exchanging the B. tribocorum virB4 BamHI fragment of pRS14 [1] by a BamHI fragment of a mutant B. henselae virB4 gene represented by a cryptic 252 bp open reading frame. This BamHI fragment was constructed by megaprime PCR of two PCR products. Product 1 was amplified using oligonucleotide primers prRS7 (CGGGATCCCGTGTAGGTATTG GCTGGATGT) (Seq. Id. No. 52) and prRS8 (TACACCTTCAACAACCATCAC) (Seq. Id. No. 53) and contained the first bp of the virB4 gene at the 5'-end. Product 2 resulted from PCR amplification by oligonucleotide primers prRS09 (TGATGGTTGTTGAAGGT GTAAATGATGAGATCGCCGTC) (Seq. Id. No. 54) and prRS10 (CGGGATCCCGCTCTTCACGCAATTCTTCT) (Seq. Id. No. 55) and contained the last bp of the virB4 gene at the 3'-end. Complementary sequences introduced by the oligonucleotide primers prRS8 and prRS9 allowed the megapriming between the PCR products 1 and 2.
- pRS40: is a plasmid of 7898 bp composed of the following sequences: Positions 1 to 180 correspond to positions 2482 to 2661 of pK18 (accession M17626), positions 181 to 513 correspond to positions 1 to 133 of pK18 (accession M17626), position 514 is C, positions 515 to 1298 correspond to positions 97 to 880 of pAComegaGm (accession U22104), positions 1299 to 2823 correspond to positions 925 to 2449 of pK18 (accession M17626), positions 2824 to 2827 are GTAC, positions 2828 to 4454 correspond to positions 8669 to 10295 of pCD341 (accession AF053122), positions 4455 to 4550 are TTCGAGCTCACAGGAGCGATATCATGGATAAAGCGGAATTAATTCCCGAGCCTCCAAAAAAGAAGAGAAAGGTCGAATTGGGTACCGGTCGAGATC (Seq. Id. No. 56), positions 4551 to 5577 correspond to positions 486 to 1512 of bacteriophage P1 cre (accession X03453), positions 5578 to 5585 are TAGTCGAC, positions 5586 to 5734 correspond to positions 6121 to 6269 of pCD341 (accession no. AF053122), positions 5735 to 5739 are CCCGG, positions 5740 to 6067 correspond to positions 560 to 233 of pCD341 (accession no. AF053122), positions 6068 to 6072 are CCGGG, positions 6073 to 7512 correspond to positions 142 to 1581 of B. grahamii mob/rep (accession AJ422079), positions 7513 to 7535 are CTCTAGAGTCGATCGACCTGCAG (Seq. Id. No. 57), positions 7536 to 7897 correspond to positions 29465 to 29826 of pSB4 (accession no. AB027254) and positions 7898 is T.
- pRS45: corresponds to pRS40 digested with SalI and XmaI, filled-in, and recirculised by ligation of the vector backbone. Thereby the MobA sequences fused to the C-terminus of Cre are deleted, resulting in the production of Cre extended C-terminally by the amino acid sequence: SRPGCLAVYGGRPARHPPGRCFATFKS APGGFVLLRRAFTDKQQIKRKAQSFD (Seq. Id. No. 58).
- pRS49: corresponds to pRS40 with the SalI/XmaI fragment being replaced by a SalI/XmaI fragment encoding amino acids 303 to 542 of BepB in the same reading frame as Cre resulting in a N-terminal fusion. This fragment was generated by PCR using oligonucleotide primers pRS169 (TCCCCCCGGGTTAGCTGGCAATAGCAAGCG) (Seq. Id. No. 59) and pRS181 (ATGGTGTCGACAAGAGCTT GAAAACACGCTC) (Seq. Id. No. 60) and B. henselae 49882T-SmR template DNA, followed by digestion with SalI and XmaI.
- pRS50: corresponds to pRS40 with the SalI/XmaI fragment being replaced by a SalI/XmaI fragment encoding amino acids 293 to 532 of BepC in the same reading frame as Cre resulting in a N-terminal fusion. This fragment was generated by PCR using oligonucleotide primers pRS171 (TCCCCCCGGGTTAGTTGGTAAGAGCCCTTG) (Seq. Id. No. 61) and pRS182 (ATGGTGTCGACTCGATCT GAAAGAAATATTGAT) (Seq. Id. No. 62) and B. henselae 49882T-SmR template DNA, followed by digestion with SalI and XmaI.
- pRS51: corresponds to pRS40 with the SalI/Xmal fragment being replaced by a SalI/XmaI fragment encoding amino acids 352 to 534 of BepD in the same reading frame as Cre resulting in a N-terminal fusion. This fragment was generated by PCR using oligonucleotide primers pRS173 (TCCCCCCGGGTTACATACCAAAGGCCATTCC) (Seq. Id. No. 63) and pRS183 (ATGGTGTCGACAAAATC CCCTCTACGAAGG) (Seq. Id. No. 64) and B. henselae 49882T-SmR template DNA, followed by digestion with SalI and XmaI.
- pRS53: corresponds to pRS40 with the SalI/XmaI fragment being replaced by a SalI/XmaI fragment encoding amino acids 645 to 834 of BepF in the same reading frame as Cre resulting in a N-terminal fusion. This fragment was generated by PCR using oligonucleotide primers pRS175 (TCCCCCCGGGTTAGATGGCGAAAGCTATTGC) (Seq. Id. No. 65) and pRS184 (ATGGTGTCGACCGACG TCTGTTCATAAACTC) (Seq. Id. No. 66) and B. henselae 49882T-SmR template DNA, followed by digestion with salI and XmaI.
- pRS55: corresponds to pRS40 with the SalI/XmaI fragment being replaced by a SalI/XmaI fragment encoding the C-terminal 240 amino acids of B. tribocorum Fic-1 in the reading frame resulting in a N-terminal fusion with Cre. This fragment was generated by PCR using oligonucleotide primers pRS175 (TCCCCCCG GGTTAGATGGCGAAAGCTATTGC) (Seq. Id. No. 65) and pRS184 (ATGGTGTCGACCGACGTCTGTTCATAAACTC) (Seq. Id. No. 66) and B. tribocorum IBS 506T template DNA, followed by digestion with SalI and XmaI.
- pRS56: was constructed by replacing the DsRed gene of plasmid Cre-Stoplight [9] via flanking SacI-sites by a neomycin phosphotransferase (neo) gene amplified by PCR using pCB6 [17] as template DNA. The following two primers carrying terminal SacI-sites were used for amplification: pRS189 (CGAGGAGCTCATTACCTCATATAGCATACATTATACGAAGTTATAATTGCCCTTGCCACCATGATTGAACAAGATGGATTGC) (Seq. Id. No. 67) and pRS190 (CGAGGAGCTCTCAGAAGAACTCGTCAAGAAG) (Seq. Id. No. 68). In the generated PCR-fragment, pRS189 introduces an improved Kozak sequence upstream of the start codon of neo, as well as a LoxH-sequence upstream of the Kozak sequence and next to the SacI site. The generated PCR fragment was digested by SacI and ligated with the SacI-digested vector backbone of Cre-Stoplight. In the resulting plasmid pRS56 the orientation of the neo gene is like the adjacent eGFP gene.
- pRS101: containing a virB4 expression cassette for complementation of the virB4 mutant strain RSE242 in trans was generated as follows: A PCR fragment containing the putative B. henselae virB promoter was amplified using primers prRS198 (TAGCATCGATGGATC CGTTTCATTGCCCTTTCGTATT) (Seq. Id. No. 69) and prRS199 (AAGGATCGATCTCGAGTTATCCTGGATATAGTGTCTGTCAT) (Seq. Id. No. 70). This product was cut at its terminal ClaI restriction sites and introduced into the backbone of dam-methylated plasmid pRS20 [1] cut by XhoI and ClaI before Klenow fill-in. The resulting plasmid pRS100 was cleaved by XhoI. Into this restriction site a PCR fragment amplified by primers pRS200 (AAGGCTCGAGGAGGAACTAAACAGGTGAAAC) (Seq. Id. No. 71) and pRS201 (AAGGCTCGAGTCATTGATTTTCTCTCCTTTG) (Seq. Id. No. 72) containing the full length virB4 gene of B. henselae was introduced after cleaving it at terminal XhoI-sites. pTR1000 was constructed by elimination of a BamHI insert (1.3 kb) from pLRS14 [1].
- pTR1001: was constructed by introducing into pTR1000 a PCR fragment amplified by primers prTR001 (ACGCGTCGACAAAGTAGTTTCTATGAGTAGATAA) (Seq. Id. No. 73) and prTR002 (CCGCTCGAGTGCAAATTTTTTAATCAGGATCAT) (Seq. Id. No. 74). The generated PCR fragment containing terminal restriction sites for SalI and XhoI was digested by these enzymes and introduced into the pTR1000 vector backbone cut by Sail only. Notably the ligation of XhoI restriction site into a SalI restriction site cannot be recut by SalI thereafter. The sequence amplified is the distal homology region necessary for gene replacement in both suicidal mutagenesis vectors pTR1003 and pTR1002, spanning the region from bepG to the adjacent mvin gene.
- pTR1003: (TR) was constructed by introducing into pTR1001 a PCR fragment amplified by primers prTR005 (GCTCTAGATGGTATGTCCGTTCAGAACC) (Seq. Id. No. 75)and prTR006 (ACGCGTCGACAGAGGGATCCCTCAAATTCAT) (Seq. Id. No. 76). The generated PCR fragment contains termi- nal restriction sites for XbaI and SalI and was digested with these enzymes and introduced into the vector pTR1001 linearised accordingly. The amplified sequence corresponds to the proximal homology region spanning virD4 to bepB, necessary for deletion of bepB to bepG. Taken together, the proximal and distal homology region in this vector are connected in-frame via the SalI restriction site. Following the mutagenesis procedure by double cross-over recombination [1], this gives rise to a cryptic fusion protein encoded by the ORF "ATGAATTTGAGGGATCCCTCTGTCGACAAAGTAGTTTCTATGAGTAGATAA" (Seq. Id. No. 77), which is composed of BepB (N-terminal) and BepG (C-terminal) sequences connected by a linker sequence.
- pTR1002: (TR) was constructed by introducing into pTR1001 a PCR fragment amplified by primers prTR004 (GCTCTAGAGGGCGCTTCCATTCCGTGT) (Seq. Id. No. 78) and prTR003 (ACGCGTCGACCGTTTTTGCTTTTGCCTTTGGCAT) (Seq. Id. No. 80). The generated PCR fragment contains terminal restriction sites for XbaI and SalI, which after digestion by these enzymes was introduced into the vector pTR1001 linearised accordingly. The amplified sequence corresponds to the proximal homology region spanning virB11 to bepB, necessary for deletion of bepA to bepG. Taken together proximal and distal homology region in this vector are connected in-frame via the SalI restriction site. Following the mutagenesis procedure by double cross-over recombination [1], this gives rise to the open reading frame "ATGCCAAAGGCAAAAGCAAAAACGGTCGACAAAGTAGTTTCTATGAGTAGATAA" (Seq. Id. No. 79) encoding a cryptic fusion protein composed of BepA (N-terminal) and BepG (C-terminal) sequences connected by a linker sequence.
- pTR1175: TR was constructed by introducing a PCR fragment amplified by primers prTR017 (ATAAGAATGCGGCCGCGA TGAAAAAAAACCAACCATCCTC, Seq. Id. No. 27) and prTR018 (CGGGATCCTTAGAGTGCCAGCACCATTTT, Seq. Id. No. 28) into the pFLAG CMV2 (Sigma) expression vector. The PCR fragment containing terminal restriction sites for NotI and BamHI was digested and introduced into the vector digested accordingly.

### Conjugation form E. coli to B. henselae:

Conjugations were performed by three-parental matings as described [1,15,16].

### Generation of deletion mutants in B. henselae:

Deletion mutants RSE242, TR106, and TR119 were constructed in B. henselae strain RSE247 (49882-SmR wild-type) by a two-step double-crossover method as previously described for B. tribocorum [1]. RSE242 was constructed using the mutagenesis vector pRS25. Introduction of the complementation plasmid pRS101 into RSE242 resulted in the establishment of RSE364. TR106 was constructed using the mutagenesis vector pTR1003, and TR119 by using pTR1002.

### Cell culture:

If not specified further, cells were cultured as described [7,11,14].

### Cell infection assays:

If not specified further, bacterial infections were carried out as described [14].

### REFERENCES

1. Schulein R, Dehio C: **The VirB/VirD4 type IV secretion system of *Bartonella* is essential for establishing intraerythrocytic infection**. *Mol. Microbiol.* 2002, **46**:1053-1067.
2. Staskawicz B, Dahlbeck D, Keen N, Napoli C: **Molecular characterization of cloned avirulence genes from race 0 and race 1 of *Pseudomonas syringae* pv. *glycinea.*** *J. Bacteriol.* 1987, **169**:5789-5794.
3. Eddy SR: **Profile hidden Markov models.** *Bioinformatics* 1998, **14**:755-763.
4. Yaffe MB, Leparc GG, Lai J, Obata T, Volinia S, Cantley LC: **A motif-based profile scanning approach for genome-wide prediction of signaling pathways.** *Nat. Biotechnol.* 2001, **19**:348-353.
5. Dehio C, Prevost MC, Sansonetti PJ: Invasion of **epithelial cells by *Shigella flexneri* induces tyrosine phosphorylation of cortactin by a pp60c-src-mediated signalling pathway.** *EMBO J.* 1995, **14**:2471-2482.
6. Seubert A, Falch C, Birtles RJ, Schulein R, Dehio C: **Characterization of the cryptic plasmid pBGR1 from *Bartonella grahamii* and construction of a versatile *Escherichia coli-Bartonella* spp. shuttle cloning vector.** *Plasmid* 2003, **49**:44-52.
7. Kempf VA, Schaller M, Behrendt S, Volkmann B, Aepfel-bacher M, Cakman I, Autenrieth IB: **Interaction of *Bartonella henselae* with endothelial cells results in rapid bacterial rRNA synthesis and replication.** *Cell. Microbiol.* 2000, **2**:431-441.
8. Vergunst AC, Schrammeijer B, den Dulk-Ras A, de Vlaam CM, Regensburg-Tuink TJ, Hooykaas PJ: **VirB/D4-dependent protein translocation from Agrobacterium into plant cells.** *Science* 2000, **290**:979-982.
9. Yang YS, Hughes TE: **Cre stoplight: a red/green fluorescent reporter of Cre recombinase expression in** **living cells.** *Biotechniques* 2001, **31**:1036, 1038, 1040-1031.
10. Kirby JE, Nekorchuk DM: ***Bartonella-*associated endothelial proliferation depends on inhibition of apoptosis.** *Proc. Natl. Acad. Sci. U. S.* A. 2002, **99**:4656-4661.
11. Beyaert R, Cuenda A, Vanden Berghe W, Plaisance S, Lee JC, Haegeman G, Cohen P, Fiers W: **The p38/RK mitogen-activated protein kinase pathway regulates interleukin-6 synthesis response to tumor necrosis factor.** *Embo J.* 1996, **15**:1914-1923.
12. Horton JK, Seddon LJ, Williams AS, Baxendale PM: A method for measuring intracellular cycl ic AMP levels with **immunoassay technology and novel, cellular lysis reagents.** *Br. J. Pharmacol.* 1998, **123** (Suppl.):31.
13. Livak KJ, Schmittgen TD: **Analysis of relative gene expression data using real-time quantitative PCR and the 2(-Delta Delta C(T)) Method.** *Methods* 2001, **25**:402-408.
14. Dehio C, Meyer M, Berger J, Schwarz H, Lanz C: **Interaction of *Bartonella henselae* with endothelial cells results in bacterial aggregation on the cell surface and the subsequent engulfment and internalisation of the bacterial aggregate by a unique structure, the invasome.** *J*. *Cell Sci.* 1997, **110**:2141-2154.
15. Dehio C, Meyer M: **Maintenance of broad-host-range incompatibility group P and group Q plasmids and transposition of Tn5 in *Bartonella henselae* following conjugal plasmid transfer from *Escherichia coli.*** *J Bacteriol* 1997, **179**:538-540.
16. Dehio M, Knorre A, Lanz C, Dehio C: **Construction of versatile high-level expression vectors for *Bartonella henselae* and the use of green fluorescent protein as a new expression marker.** *Gene* 1998, **215**:223-229.
17. Brewer CB, Roth MG: **A single amino acid change in the cytoplasmic domain alters the polarized delivery of influenza virus hemagglutinin.** *J. Cell Biol.* 1991, **114:**413-421.

## Claims

1. A polypeptide comprising at least one VTP (VirB/VirD-translocated protein) domain or a functional fragment thereof wherein said polypeptide is translocated into a cell by the VirB/VirD4 translocation system.

2. The polypeptide of claim 1 wherein said VirB/VirD4 translocation system is from a *Bartonella species*, preferably *Bartonella henselae.*

3. The polypeptide of claim 1 or 2 with the proviso that said polypeptide does not comprise a sequence as set forth in Seq. Id. No. 29.

4. The polypeptide of claims 1 to 3 wherein said at least one VTP domain has a sequence which is at least 15% identical to the consensus of the VTP domain set forth in Seq. Id. No. 30 when aligned over the full length consensus sequence, more preferably 30%, even more preferably 40%, even more preferably 50% and most preferably 60%.

5. The polypeptide of claims 1 to 4 wherein said polypeptide comprises a sequence which is at least 75% identical, more preferably at least 80% identical, even more preferably at least 90% identical and most preferably 100% identical to a sequence as set forth in Seq. Id. No. 3, 7 or 9.

6. The polypeptide of claims 1 to 4 wherein said polypeptide comprises a sequence which is at least 30% identical, more preferably 50% identical, even more preferably 75% identical and most preferably 100% identical to an amino acid sequence selected from the group consisting of Seq. Id. No. 11 (BepD), 13 (BepE), 15 (BepF) and 17 (BepG).

7. The polypeptide of claims 1 to 4 having the sequence set forth in Seq. Id. No. 18 (Fic-1).

8. A nucleotide sequence encoding a polypeptide of claims 1 to 6.

9. The nucleotide sequence of claim 8 wherein said sequence is selected from the group consisting of the coding sequences (CDS) of Seq. Id. No. 2, 6, 8, 10, 12, 14 and 16.

10. A fusion polypeptide comprising a VTP domain or a functional fragment thereof and a polypeptide to be translocated into a cell by the VirB/VirD4 translocation system.

11. The fusion polypeptide of claim 10 further comprising C-terminal to the VTP domain a positively charged amino acid sequence motif comprising at least three positively charged amino acids, preferably said motif comprises the amino acid sequence HRHHRRHH (Seq. Id. No. 35).

12. The fusion polypeptide of claim 10 or 11 wherein said VTP domain has a sequence which is at least 15% identical to the consensus of the VTP domain set forth in Seq. Id. No. 30 when aligned over the full length consensus sequence, more preferably 30%, even more preferably 40%, even more preferably 50% and most preferably 60% and/or stems from a polypeptide of claims 1 to 7.

13. The fusion polypeptide of claims 10 to 12 further comprising a fic-domain which shows at least 22% identity, preferably 25% identity over at least 100 aligned amino acids or at least 40% identity over 40 aligned amino acids to the consensus fic sequence set forth in Seq. Id. No. 31 and with a central conserved motif HPFXXGNG (Seq. Id. No. 36) in which at least 4 amino acids are identical.

14. The fusion polypeptide of claims 10 to 13 further comprising a repeated tyrosine-containing domain having a conserved tyrosine residue and which shows at least 25%, preferably 40%, even more preferably 70% and most preferably 80% sequence identity over at least 12 aligned aminoacids to one of the motifs set forth in Seq. Id. Nos. 32 to 34.

15. The polypeptide of claims 10 to 14 wherein said polypeptide to be translocated into a cell is fused N-terminally to said VTP domain or its functional fragment.

16. The polypeptide of claims 10 to 15 wherein said protein to be translocated is selected from the group consisting of antibody, antigen, immunmodulatory protein, enzyme, receptor protein, signalling molecule, structural protein, fluorescent protein, hormone and a toxin or portions thereof.

17. A method for introducing polypeptides into eukaryotic cells comprising:
contacting said eukaryotic cells with bacteria encoding a functional type IV secretion apparatus, said bacteria expressing a fusion protein to be introduced into said eukaryotic cells, wherein said fusion protein comprises (i) a VTP domain or a functional fragment thereof and (ii) a polypeptide to be translocated into said cells.

18. The method of claim 17 wherein said fusion polypeptide is a polypeptide of claims 10 to 16.

19. The method of claim 17 or 18 wherein said bacteria encoding a functional type IV secretion apparatus are Gram-negative bacteria, preferably said bacteria belong to the genus Bartonella, most preferably Bartonella henselae.

20. The method of claims 17 to 19 wherein said eukaryotic cells are of mammalian origin, preferably human or animal cells, more preferably a cell type selected from the group consisting of endothelial cells, fibroblasts, epithelial cells, leukocytes and erythrocytes, most preferably endothelial cells.

21. A method for the identification of compounds which modulate the type IV secretion process into eukaryotic cells comprising:
contacting eukaryotic cells comprising a reporter system for the detection of protein translocation into the cytosol of said eukaryotic cells with
(i) bacteria encoding a functional type IV secretion apparatus, said bacteria expressing a fusion protein to be introduced into said eukaryotic cells, wherein said fusion protein comprises (i) a VTP domain or a functional fragment thereof and (ii) a polypeptide showing a biological activity and
(ii) a candidate compound and
detecting compounds having a modulatory activity on the protein translocation process by comparing the read out of the reporter system contacted with the candidate compound to a control sample.

22. The method of claim 21 wherein said reporter system comprises a nucleic acid encoding a detectable protein wherein said detectable protein is only expressed when said fusion protein comprising said polypeptide with a biological activity is translocated into said eukaryotic cells.

23. The method of claim 21 or 22 wherein said fusion protein further comprises C-terminal to the VTP domain an amino acid motif which comprises at least three charged amino acids, preferably said motif comprises the amino acid sequence HRHHRRHH (Seq. Id. No. 35).

24. The method of claims 22 to 23 wherein said detectable protein is a fluorescent protein, preferably a GFP.

25. The method of claims 22 to 24 wherein said nucleic acid encoding said detectable protein is flanked by target sites for a recombinase and said polypeptide to be translocated has recombinase activity recognizing said target sites.

26. Use of the polypeptides of claims 1 to 7 or the fusion polypeptides of claims 10 to 16 or the nucleic acids of claims 8 and 9 for the manipulation of cells, preferably mammalian cells, more preferably endothelial cells.

27. A polypeptide of claims 1 to 7 and/or a nucleic acid of claims 8 and 9 for use as medicament.

28. The polypeptide of claim 27 further comprising a repeated tyrosine-containing domain having a conserved tyrosine residue and which shows at least 25%, preferably 40%, even more preferably 70% and most preferably 80% sequence identity over at least 12 aligned aminoacids to one of the motifs set forth in Seq. Id. Nos. 32 to 34.

29. The polypeptide of claims 27 or 28 further comprising a fic-domain which shows at least 22% identity, preferably 25% identity over at least 100 aligned amino acids or at least 40% identity over 40 aligned amino acids to the consensus fic sequence set forth in Seq. Id. No. 31 and with a central conserved motif HPFXXGNG (Seq. Id. No. 36) in which at least 4 amino acids are identical.

30. A method for the generation of attenuated bacteria comprising the following steps:
introducing at least one mutation in at least one nucleic acid encoding a protein of claims 1 to 7 and
selecting attenuated cells.

31. The method of claims 30 wherein said bacteria are selected from the genus Bartonella.

32. An ex vivo method for the diagnosis of a bacterial infection comprising the following steps:
detecting in a body fluid and/or tissue sample of an individual a protein of claims 1 to 7 or a nucleic acid of claims 8 and 9.


SEQUENCE LISTING

<110>  Universität Basel

<120>  Polypeptides translocated into cells by the VirB/VirD4 type IV secretion
system
       and uses thereof

<130>  08155EP

<160>  80

<170>  PatentIn version 3.1

<210>  1
<211>  18895
<212>  DNA
<213>  Bartonella henselae

<400>  1
   aattgaaaaa gcgccgtgaa gcggttgatg cagtgatcag tacccatgca cttgaaggta      60
   tagcgcttca tcccaaaacg ctcaaaattt tagaaggata tgcgagagga aatacctcac     120
   tagaagaatt taacacgctt atggacaacg ctaaattata aggaggtcgt tatgccaaag     180
   gcaaaagcaa aaacgaaaaa tacagaaatt atttcccccc atcattatgt ctatcctaac     240
   accacaacac tcaaaaataa atatggaata aaaaacttaa atgcttttct ggaaaaatgc     300
   tcgcatgata cagccaaagc aatgatcaat ctgcgtgaag aatccttacc agaatatttt     360
   gataccgcct atctatgcca tattcatcaa cagttgttta aaaatacatt tgaatgggct     420
   ggataccttc gccatatccc cttcacattt gcagatggca ccacagccgc catgccagaa     480
   atgaaaagaa cgggttggaa aaatgctttt gcaattggtg atgagatcca agaaggcttg     540
   caaagattag atcaaacact tgccgaaaaa aacaatttac aaggtttaac gcgcgaagaa     600
   tttaactctg aagcaataga actgtttaat tctctcaacc aactccaccc attcagagaa     660
   gggaatggac gaacacaacg gctatttttt gaaaatcttg ccaaagcagc aggacatcag     720
   cttaacttct cgcttatcac aaaagaacgc atgatggttg ccagtgttgc ggtagcagaa     780
   aatggtgacc tagaacctat gcaacatttg tttgaggata tctccaatcc agaaaaaatt     840
   cgtcttttaa aagaatttat gcacaccatg aaaaatactg ggcgcaatgt taatgatcgt     900
   cctgttatgg ttgcaaagga aggggaaact tacacgggca cctatagagg tgctggtctt     960
   gaaggttttg cactcaatgt aaaaggtgct tacattatcg gcaatataga ccacctccca    1020
   ccagaacaac taaaaatatt aaaacctggt gataaaatta cctttacagc tccaaaagcc    1080
   gaagaattaa aaaaaacact catcccaaaa gagacattag ttcctttgac aaaacttgaa    1140
   atagctgaaa tggttgcaga agatgctttt gtccacacat gtcgagacca aatttgcagt    1200
   ctatcaaaaa ttgtttatgg cagccaaggt gttttgaata aaaacataat agaaatcatt    1260
   aaaaatccaa gcaagggtca gcaactcgcc acacaaattg aaagaacacc ttactctgtt    1320
   catagtcttg caggttttga cctcatttgc tttaaaactg gggcacgggt acgtgctgaa 1380
   aagcatgttg cactcctcag ttgtgcagtt gcaaatttta cccatgcagt aaaacatgca 1440
   agacaagaaa tcacaaaaga acatcaagca gaacaaaacc gccttagaca agaggtccct 1500
   atgccaagtc aaagtttgca agatcttctt tccctgccaa aagagttcca gcaaaaagcc 1560
   ttaggggtct ctcctctgct tcaaaaagag ctcacgagtc ttttacaaaa agtcaatagc 1620
   cgtctctcct caagtgaaca aagagcactt agagaaaata atcatgagac actggcgaaa 1680
   aatcttggtg tttcagaaca gaaagcaaag gaaattacaa aaactgttat gaaagccaga 1740
   gaagtacagc aaaaatcgca aacacgcaca gtcagtcact caaaaacgct tgccatggct 1800
   agctaaagag tttgctcaaa attgcaaatt tttgcacctt ccttttgaat cttcagggga 1860
   aatggaatgt tatgctgtgc tgcccataaa aaagcgttca gcgctttatt accgatttta 1920
   acaagaagcc tttacggcat attccgataa aaccgaaagt aagacacgat gaaatataca 1980
   aagacgcaac tggcactcat ttccatgcca atcgcttcag gtgctttaac gatatttctt 2040
   gttcctcata tgttgtcatt tgtgataaat gatctgaaaa ccaaccaaat ctattggtat 2100
   gtccgttcag aacccttatt aacgctgatg ctggttgctg ctgtatcatt attttatact 2160
   ctctcacaaa agctgcactt gcgcaaagcg atcacattcg tctcaacggc ctttttttgc 2220
   atcactgctc tttactatat cggcagtgaa ataaaacgct taaatcccta tgttggacaa 2280
   caggggataa cctggggcta tgctttaaaa ttcatggatc cgatggttgt tttcggcgtc 2340
   attttaggct ttgttctttt agcaatccaa gtcataatca cctctccacg cacgagcaat 2400
   gtcaaacgtg ccaaaaaagg aatttttggt gacgccgcat ggatgaattt aaaagaagcg 2460
   gcaagaattt ttccctccaa tggccaaatt gttattggcg aaagataccg tgttgatcaa 2520
   gataatgttc gcaacattcc ctttgcacca ggcaataaaa caacatgggg caagggtgga 2580
   acagcccctt tgctgacctt taatcttgat tttggttcaa cgcacatgat ttttttcgcc 2640
   ggttctggtg gatataaaac aacaagcaca gtggttccaa cctgtctaac ctacacaggc 2700
   cctattgttt gccttgatcc ttcaacagaa attgcaccca tggtcaagtt tgcgcgcaaa 2760
   aaaatgggca atagaaatgt tattattctc gatcccaatt cgctcttaac aaaaaatttt 2820
   aatgtgctcg attggctttt agacgaaaat ataccgcgca caaggcgtga agccaatatt 2880
   gtgagctttt ccaaactgct tctctcagag aaaaaatcag aaaattcttc agcagaatat 2940
   ttttcaacac aagcgcataa tcttttaacc gcacttcttg cacatgtaat cttctctgat 3000
   aaatatgaag acagtgagcg taatctcaaa acattacgcg cgatcctctc tcagtcagaa 3060
   acagctgttg tcaatcaatt acgtatgatt caagaaacaa caccgtctcc ttttattcgt 3120
   gaaatggttg gtattttcac ggaaatggca gatcaaacct tttcaggggt ctacacaact 3180
   gcctccaaag acacccaatg gctttctttg tcaaattacg ctgatcttgt ttgtgggaat 3240
   gattttgcat cctcggatat tgcaaacgga aaaacagatg tttttcttaa tcttcctgcc 3300
   agtattttga acagttatcc agcgattggc cgcgtgatca ttggcgcttt tttaaatgcg 3360
   atggttacag cagatggaaa ctataaaaag cgtgttttgt ttgtcttgga tgaagtcgat 3420
   cttcttggtt atatgaatat tttagaagaa gcacgtgatc gtgggcgtaa gtacggcaca 3480
   tccttgatgc ttttttatca atcctctggt cagttggtta atcactttgg agaagcagga 3540
   gcgcgttcat ggtttgaaag ctgctctttt gtgagttacg ccgccattaa agatctccaa 3600
   acagccaaag acatttcgga acgctgtggt caaatgacca ttgaagtaac tggaacgagc 3660
   aaatcgagag gtttgtcttt aacaaaaggt tctcaaaaca tcaactatca acaaagagca 3720
   ctgattttac cccacgagat tattcaagag atgcgccaag atgaacaaat tattcttatg 3780
   caagggcacc cccctttacg atgcggtcga gcgatctatt ttagaaggaa agagatgtta 3840
   gcagctactg aaaaaaaccg ctttgcccca caagcaaaga aaagctgaaa aagcaccctc 3900
   tgacggctgc tcttaagcct caaaacaaga agaacagcat acaataaaaa aatcagagat 3960
   tcttgtttta taaaaacgtc ttaaaaagga attttcacat gacccaacaa gaaaaacatg 4020
   gtgaaaaaag acctttgagc agtgttgttt ttcacgggct taccaatgcc agcactcttt 4080
   atgaagcgct ctcctcatca ccaaacaatg cgtaccaatc atatctatct taagatacct 4140
   ctcaagaaac ttttgaagaa agctctttta caggaaaatc gcctagaaaa atttttcaca 4200
   aaaaataaaa agacaccatt catgaaaaca acaaagaccg atccttctct cttaaaaaaa 4260
   gaaaaaagca ccaagataag gattggtaca agcaaaaaca ctcatgaatt tgagggatcc 4320
   ctcttccctc gttcaaagct tgaaatctca aaagaacatg cgagggggaa tatttcatga 4380
   gaagaattta acactctcat ggacagtgcg acatgataag gagttaacaa tgccaaaagc 4440
   aaaagcaaaa aatatctcca ccgcttctcc ccataattat atctatcctg gtacccaaat 4500
   actcaaaaat aaatatggag aaacagattt aaaactcctt ctggaaaaat gcttacatga 4560
   tagggaacaa gcaatgatga atctgcgtgc agaatcccta ccagaatatt ttgattgcgc 4620
   ttatctatgc tatatccacc aacaattgtt tcaaaaaacc tttgaatggg ctggccatct 4680
   ccgccacact cccttcacat ttgcagatgg tagcattgcc gccatgccag aaatgaaaag 4740
   aacagaatgg ggcaaagatt ttgcaaaaag tgaaaaaatc cccgaacttt tacaaaaatt 4800
   agatcaagag cttgccgaaa aagacaattt gcaaggctta acacgcgaag agtttatcaa 4860
   gcaagcaacg gagctatttt attctcttca caaaatccac ccattcatag atggcaatga 4920
   acacacagaa caattctttt ttgaaaatct cgccaaagcc gccggacatc aacttgactt 4980
   ttcgcttgtc acacaaaaac gcatgatggc agtctgtagc gaagcaatgc aatatggcga 5040
   tacacaactg atgaaagatc tttttgaaga tatctccaat ccagaaaaaa tacgtctttt 5100
   aaaagacttt atgaacaaca tcaacaatac aggacgcaac gtgaatgatt gccttgttat 5160
   ggtgacaaag gcaggagaaa cctacagagg aacctatcaa ggttgttatt cagaagcttt 5220
   tgtgctcgat atgcaaggga cttacatcat cggcaaaaaa gatgatctca caccagaacg 5280
   attaaaatca ttaaaacctg gtgatacaat tacctttatc gcccccaaaa acaaagagct 5340
   tgaaaacacg ctcattccaa aagagacatt agcgcctcta acaaaaagtg aatgtgccag 5400
   aacggttgcg caaactagcc atgtcctcac agctcaaaag aaagtccggc aacactcaaa 5460
   aattgtttat ggcaatgcaa acacattaaa cgaacaaata gaagagatta ctaaaaatcc 5520
   agaattaggt caactgctcg ccgatcagat tagacaaaca ccgcactctc tttctcctct 5580
   tgcaggtttc agtctatgcg gtttacaaaa tccggcacgt gcaaacgctc gaaatcatct 5640
   tgatgcactc gctactacag ttgcaaatta tgcacatatt gtagaacata catatcatgt 5700
   aatcactcaa aaacatcaaa ttgaacaaga acgccttggt aaaacagtag aaaagcccag 5760
   tcaaaacttg caaaaccttt ttgccttatc accggaacaa caaagagaaa tcttgtctca 5820
   atctcctcaa ttataccaag agctccgtac ttttatatgc tgtgtagagc atcgtctctc 5880
   atcaaacgag tggagtgcga tcaaaaacaa agattatgaa acacttgctc aaagtatcgg 5940
   tgtatcagaa cagacagcac gagagattac caatactgtt caaaaagcca gagagttaca 6000
   taaacaagtc tacacacacg caatcactcg ctcaaatacg cttgctattg ccagctaaaa 6060
   aatatgaggc cttgttgctt aatctgaaca cccatgtgga agaaatgttt taatattccc 6120
   acacccatga aagaacagag cacttaagcg ccctattact aattttgcaa atagcataga 6180
   catcatatcc agattaattg ggcggatgat tatgagtacc tttttaaacg ccaggattac 6240
   agctgacgga aactacaaaa agcgtgtgtt gtttatctta gatgaagtgg accttcatgg 6300
   ttataggaat attttaaaag ggcgctcatt ttacctcccc gaaaaacgcg ccaaaatgaa 6360
   taaattattc tcatgcaaga acatccccct ttacgatgtg gttgcacgat ctattttaga 6420
   aggaaaaaaa tgttagcaac tgctgaaaag aactgttttg cccccaaagc acagaaaaac 6480
   taaaaaccat cctcttatgg agaccatgta tcaaaacaaa aacaacaaca tcaaaggata 6540
   aaagcaaagg ttctgtttca taaaagcata taaacaagga atttccgcat gatccagcaa 6600
   gaaaaataag atgaaaaaag atctttgagc aatgctgttt tgcatgggct taccaacacg 6660
   cgcactcttc gtaaagagtc cctaaacgca caagcggtca atgacaatga tacgcaccat 6720
   gtccatatgg aagagtccct tatttaaaca cccagaaggc gggctttatg aaaacaactt 6780
   tattaaaagc taggaaaaat tatcatttat gaaaatgata cacaaagggg gaatacatac 6840
   caaaaaagct ttccacaaaa tgaaaaaagg aaagaaatct gcatgaaaaa acccaaacgg 6900
   actgttttgc agtttttata ccagaagaat taaaaaaaca caatgaaacg gttgatacat 6960
   ccttcagcac acatgttctc atcatctatc agtcttcatg tttttcagga agcgttcttc 7020
   cctgaaaaaa aatgttcgaa atcccaacaa aatacgcgaa aggggacttt tcacatgaaa 7080
   attttaacat tctcataaac cacacacctc tctcaaggag aaaatatatg ttagagcata 7140
   attatctgta taaaaacagc aacacactga aaaataaata tggcataaaa gacccgcaaa 7200
   gactgtatga gcgctgtgcc catgatacag ccagagaggt tatgaatctt cgctttgaac 7260
   cacccccaca aagatttggt ctcgcttatt tgaaattgat ccattggacc ctcttccata 7320
   ggagttttga atgggcgggt catactcgtg atgagccctt tacatttgaa gatggtagca 7380
   ccgcccgtat gccagctatg cgcccaaaag gtcatagggt tccttttgcc gttggtccac 7440
   agattcaaaa agaacttaaa caattagaga gaaaacttac cgcaaagaat aacttacaag 7500
   gcttatcacg ccaagagttt gctgaaagtg ctgcggaagt ttttatgact ctcgaccacg 7560
   cgcatccttt cagaaaaggc aatgggcgcg cgcaaagaat atttatggaa aagcttggac 7620
   aagcagcagg ctataagatt gacttttctt ttatcacaaa agagcgcctc acccaagcta 7680
   gtattgccgc aatgcaacat ggcaacacac aacccatgaa agatcttttt gaggatatca 7740
   cccacccgca aaaattcctt cttttaaagg aattcatctc tcaaatgaga aatagcggac 7800
   ttgaggaaat caacgaacat attgttgtcg tagcaaaaga aggtgtaaag tatgatggca 7860
   tctataaagg ttgtgctgca gaaggttttg tcatagaatt agaagatggt tttgttgtcg 7920
   gccacaaaga tgatcttacg ccagaacaag tcaaaacatt acagaacggt gatcgcatag 7980
   tctttgaaaa aagcaatgtt ctcgatctga aagaaatatt gattccaaaa gagaccttgc 8040
   cccctctcac caatgaagaa cttgccaaaa ggttcacaga taattgcggt gttgaatcat 8100
   accgccaaga agtcgaacat ttatcaaaac gtgtttatgg caagtcacaa gcactcaacg 8160
   aaatgataga gtcgatcaat atagatccaa gcttaggcgc agagtttgcc gatcaaattg 8220
   ctcaaaatcc taaatcattt tgtaaacttg cggggaggaa aatattaggc ataagaagtc 8280
   caagtcgcag gcgtgccgaa gaggctgttc cacagctctg tgaagccctt aaaagttatg 8340
   ctgatatggc acaacataca atggagaacc tcatagagca gcaccaaaga gaacaaagac 8400
   gcatagcaca ctctgtggaa aaaccaggaa gagatttgca acagcttttt accctctcac 8460
   cagaacaaca aagagaagtc ctgtctcatt ctcctacact gcaaaaacaa ctccatactt 8520
   ttgcgcgcca actacagaat cgtctctcct cagaagagca tagagctata caagaaaaca 8580
   atcctctaaa actttcttgc atgcttggtg tatcggaaag caaagcaaaa gaaattacta 8640
   aagctgtaaa acaaaccaag gaagcacact gtcagctgcg aactctcaaa atcagttgtt 8700
   cctcagcaag ggctcttacc aactaaacac gtgagatatc tataattctc tctctgagca 8760
   catcggcaag caaaatacaa gagattatga aaaccgtaaa ataaactcaa aaagtacaat 8820
   aatacaaact gcgcacactc caagtcggcc aatgcgcagt aaaggctttt agcagcaata 8880
   caggaaagag agctgtacct tttcctctcc tcttttggta caagtcattt tataaatgat 8940
   tgcgtgaaaa aatgttacaa aatatgacag aaaatatcca ccaaaataga ctcggtagtt 9000
   ttatataact tattgataaa atttcacaaa aaaactgtat aaaagacata aatgtcgatt 9060
   atgataattg tgaatagcgg aggatctttg gaggtcattc aagtaaccat cttcctttgt 9120
   ataaaagcta ttaaagcata aaggtggagg atgcaccaaa gagtttttct aaaaacaaaa 9180
   agcttgaaag gaagcatgca tgaaaaaaaa tcgaccatcc cctccgacat ctcgttctaa 9240
   agaacaagaa caagaacagg caaggacacc ttcaccgcaa gcagaacccc tctatgcaca 9300
   ggtaaataaa ccacccagag agcaccgtgc ccaatcatca gaagaaacta tctatgcacc 9360
   tcaaaaccca ccagaaacta tctatgcacc ccaaaaacct ctaggaaatc cctatgacag 9420
   acttggtggg aggccgagga atgggcgacg tgccgagaaa ctagtagacc cctatgcagt 9480
   aactgatgta cagaatctag ataggggggc agactttcaa accttagaaa atcccctcta 9540
   tgagggagtt ggcgggggtg cccatggtgg gccacatcct caagaaccag aacatctcta 9600
   tgcagagctt gaatttgaca cacaaagtgg gcgctccccc caaaaacctg tagaatctgt 9660
   ctatgcaaca gttggcatgg gagcagaggg tgggcaagac actcaaacct taaaaaatcc 9720
   cctctacgaa ggagttggct caggaaagac aacaccccct ccgacacccc attctacgga 9780
   aaaagaacaa gaacaggcaa gggcaccttc accgcaagca gaacccctct atgcacaggt 9840
   aaataaacca cccagagagc accgtgccca atcatcagaa gaaactatct atgcacctca 9900
   aaacccacca gaaactatct atgcacccca aaaacctcta ggaaatccct atgacagact 9960
   tggtgggagg ccgaggaatg ggcgacgtgc caagaaacta gtagacccct atgcagtaac 10020
   tgatgtacag aatctagata ggggggcaga ctttcaaacc ttagaaaatc ccctctatga 10080
   gggagttggc gggggtgccc atggtgggcc acatcctcaa gaaccagaac atctctatgc 10140
   agagcttgaa tttgacacac aaggtgggcg ctccccccaa aaacccgtag aatctgtcta 10200
   tgcaacagtt ggcatgggaa cagagggtgg gcaagacact caaaccttaa aaaatcccct 10260
   ctacgaagga gttggcccag gaagggcaac atctcctcca agatccccga aagatgaagt 10320
   tacttcaaag cttttaaaac acacagactt tcaacatggt gtaaaagaag tccaagagtg 10380
   gtgtaaaatt gtttatggta accagtatgc cttgaataaa aaactctgta agattcttga 10440
   gaatcctaaa gcaggagaca caattctatg ggaccttgca gcagatcctg aaagcgctgg 10500
   taaacttgcg ggtcaaaaaa tacttggcat aaaaagtcca gaccgcaaac aagctgaaga 10560
   aggttttggc tccctttgtg ctgcttttga aagacatgta gctattacac aaaagctaca 10620
   caaagatttt acccttgagc aggaaaaaca acgtggacat gagagagaga caagcccaga 10680
   aaggcaggaa cacagacatc atcgtcgtca tcactcacaa gagagagaac agggtgcttc 10740
   agagcaggaa gcacgacgca gaaataatcc cacaaatcaa aaaggaatgg cctttggtat 10800
   gtaagcgtgc ataaaaaata cagatatttt tgcctttcgt tgatttcttg gtgcaattaa 10860
   aataagatga tgtgcgccaa gaagtgttat gaaatgttac aaaaaaatca ctaaaaaagc 10920
   ttcggagatt ttatataact tattgataaa atttcacaaa aaaactgtat aaaagacata 10980
   aatgtcgatt atctggatag caaatagcgg aggatctttg gaagtcattc aagtagctat 11040
   cctcctttgt ataaaagctc ttaaagctta aaggtggaga gtgcaccaaa gaggttttct 11100
   aaaaacaaaa ggcttgaaag gaagcatgca tgaaaagaaa tcaaccaccc cctccgacaa 11160
   ctcattccac agaaaaacaa agagaaccct atggacaatc tgcgataaga gcaccttcac 11220
   cgcaaccaga acctttatac gcaacggtga acaaaagacc accgcgcgca aaagacagag 11280
   aactaaacaa accactaagc caacaggaag aagtcgtata cgcagcgctt gattttgaaa 11340
   atagatcaca tcatccaaga gagagatacc cggaaaggaa tctagaaagc gaaactatat 11400
   atacgacagt ttcttctcaa agcacaacac aagcagaaat actctacgca gatgtcactc 11460
   acacaccctt gcacagcaaa cacacgagga gagatccggc aagcgaaact ttatacgcag 11520
   aagttgctat gcaaagcaca ataccatctc taacaagaga ggaaattgca gataggatgc 11580
   aacacaatcc attggtccaa gcgtatcaac aagaagtgat gcattggtgc aaaattgttt 11640
   atggaaattc agatgttttg aaggagaaaa tgcaggaggt actccagaaa ccgtctgagg 11700
   gagaagatct ctcacgacaa gttgcagaaa atccgacgtc tgttcataaa ctcgcaggtc 11760
   gcaacctgtg tggcttgaaa acaaatgcgc gcagacaagc tgaagaaggt tttatgcacc 11820
   tgtgccaagc tcttgatggt tatacaagtg ctgtaacaca agcacaagaa aatattaaac 11880
   atgttccaca ggcagaagca agacgctatg gacaagagag tgaacaaaga gccgagaggc 11940
   tacaacaatc gcgtcatcct gaaagggaga gacaatctct ctcaaatcaa gaacttcttg 12000
   gtatggtgca agagaatgca tcaatacaaa gatatcaagc acaagttgag cattggtgta 12060
   aaattgttta tggcaattca agtgttttga aggaaaaaat ggaaggcatt ctccaagacc 12120
   tatctgaggg agaagagctt tcatggcaag ttgcagcata tcctcaatct gttcataagc 12180
   ttgcggggca taatgtatgt ggcctaaaaa caagtgcacg cagacatgct gaatcaggtc 12240
   tttcacattt atgtgatgcc attgataact ataccgatgc tgttcgacaa ataaaagaaa 12300
   gtattacgcg aggacatcag gctcagacga gacaaaaccg ccaagaacaa tctgttggag 12360
   tgaagcaagg attgcacaaa gaaaaagcct tatcaagtcc taaacagcat gggcatcaaa 12420
   cccatcatca aggtgcggaa gcctctaagc gaacacacca acaatcaccg gaagttccac 12480
   cacgcaaagt tggaggagca aaggcaatag ctttcgccat ctaaacggca tcaacgctca 12540
   gcgcgcatca gctttgctgt ttcatgagat tgtttgatct aatctcttgg ctctagtcgt 12600
   tttagaagga ttgcgtcaag agtgtgtgtt tgatgttgtg gtacatattt catcaaacac 12660
   cccgtcactg aacctgaata acgcatcaaa ttacaaagac aaatattgat tacactctgg 12720
   gcaaaaaacg caaaatcctt tgggggcatt caaatgtgta tccttctttg aagaaaaaac 12780
   tatcaaaata taaaggtgga gagcgcacca aagaggtttt ctaagaacga atagtacaaa 12840
   atcttgaaag gaaacatgca tgaaaaaaaa ccaaccatcc tcttccacat ctccctcagt 12900
   gaaagaatta aaaaagcgct atgaacaaac tgccgcagaa gcctctactc tacaccaact 12960
   ctccgcaggg agtgctccaa aagaaaaaaa acagccttcc tcgaggttgg aagaattaag 13020
   aaaacgctat gaacaaactg cttccctaac agcttctcaa gaagctctct ctgcaagggg 13080
   ttttccaaaa gaaaaaagac aacctcacac acagcaaagt cctgaacaat tgccaataca 13140
   ccatgaacag caatttgctg taacgccgca acaaagttca tcacaaacgc ctctctacgc 13200
   aacacctctt ccacaacaac cagttcgcac gcctccacaa agaccaccgc gcgcaaaaga 13260
   caaagagcta cacaaaacag ccccccaaga cagcacaccc ctatacgcga caccttctcc 13320
   acaacaatca gttcagacac ctccacaaag accaccgcgc gcaaaagaca aggagctaca 13380
   caaagcagcc tcccaagaca gcacacccct atacgcgaca ccttctcccc acaaacaaca 13440
   gcccatgaga gaaagggcgc aaagcaacac atcaagccaa gacagcgaac ctctctacgc 13500
   aacacctctt ccacaaagac accagcccat gagagaaagg gcgcaaagca acacatcaaa 13560
   ccaagacagc gaacctctct acgcaacacc tcttccacaa agacaccagc ccgtgagaaa 13620
   aaggacgcaa agcaacacat caagccaaga cagcgaacct ctctacgcaa cacctcttcc 13680
   acaaagacgc cagcccgtga gaaaaaggac gcaaagcaac acatcaagcc aagacaacga 13740
   acctctctac gcaacagctg ctccatcaca atcaccacgc atggaagaga aatctgtaaa 13800
   cacaatgcaa cgaaacttat tgcttgaagc atataaggaa gaaattaaat attggtgcgg 13860
   aattgtttat ggcgatcggc ttattttgca gaaaagaata gaagagattc aagaaaaccc 13920
   tgctctggga gaacagctgt catgggaggt ttcagaacat cctaagtcta tttctaagct 13980
   tgcgggtaaa aaaatgcttg gtgtgaaaac gaaggcacgc agaaaagctg aagaaaatta 14040
   tctagctttg cgtgacacca taaatagtta tgtttatact ttaaaatatt cacaacaaaa 14100
   acctttgcat gctccgcata cagaacacac ccgcgatgaa caacaaacac acagaacgca 14160
   aagcgctgaa aaggagagag cacctctctc taatcaggaa cttgccgaca gaattcgaac 14220
   agagccatcg gttctatata gccaaacaga agtccaatat tggagcaaaa ttgtttttgg 14280
   caacccttat attttgcagt acagaattga agacattcaa aaaaatcccg atatgggaga 14340
   agaattttca tggcaagttg caaacaatcc tagtcttttt tctccgctcg caggaaagag 14400
   gatacttggc ataaaaaatg atgcgcgcag acacgcagaa gcaagtcttt cttgcctttg 14460
   tagcgccatt gaaggttacg cagatgctgt aaaacaagca aaagaggata ttgtacaaga 14520
   atatcaagta caacaaagtc gccaagaact ttctactgaa ccggctaaac agttgcaaaa 14580
   acaacaaact ttatccaaac cacataaact gcctgaacac tcaccaacag acgcgcatca 14640
   agaaactact caaacctcca tgcaaactga gtgggagcgg cttggtgctc gaccacgcac 14700
   agtgacaaca cacaaaaaac acacacaaga gagcactttg cctacttctc atgcagagca 14760
   aaaaacaacg agagagcaaa caactgaaaa tcagcaaaaa tcccttacac ctgaaaaaga 14820
   aaaaacacct ctctcacata aagaaattgc aagcagagtt caaaacgatc tctctgttca 14880
   acgtgctcaa atagaaatct acaattggtg taatattgtt tataataacc cacttgtttt 14940
   gcaacatacc actgaagata tcaaaaaaat tcccatgcta ggagaagaac tctcatggca 15000
   agttgcaaac ttttctacaa tatttgctcc gcttgctggc aagcaagtgc ttggcataaa 15060
   aaataacgca cgtaaacatg ctgaagaagc tattccctcc ctatgcactg ccattgatca 15120
   ttacacagag attgtaaaac aagtaagaga ggacattgta aaaaaccatc aggaacaatc 15180
   tccaaagaaa gataaaaacg tgaaacgaca gcaaagctta tcgaaacctc ctaaattacc 15240
   tgagcgctca acagaaatcc cacgtcatca ggctttggaa gcctctaggc aagcacaaca 15300
   aaaaccgtcg aacgttcgat caagcgaagc tgagaaaaca caaaaaatgg tgctggcact 15360
   ctaaagatgc atcagctttc atctgacctt tcggtactta tcactttata aaaagtcccc 15420
   aagcagtatt gggcagtatt acggaaaatc tataacaccc cctccccgtc atgtgccctt 15480
   actatataaa aattacaaat acaaatagtg attatgatcc atgaaaaaaa acttttgaaa 15540
   ttattcaaat ggacattctc tctgaaagga aaaactctca aaacataaaa gtggagaata 15600
   caccaaagag gttttctaaa agcaaattaa aatctcgaaa ggagacacac atgaaaaaaa 15660
   aacaaccagc cccttcaaca ccttcatccg caagcgttac tcagatggcg tctctatcga 15720
   atgaggaaat tttcaacaga gttcaaagag atccattggt tcaatcttgt gaagaagaaa 15780
   tccaatattg gtgcaaaatt gtttttggca acgcttatgt tttgcagtac agaattgaag 15840
   acatgcaaaa aattcctgag ataggagaag aactttcatg gcaaatttca gaaaatcctc 15900
   acttttttca taagcttgca ggcagtcaaa cccttggcat aaaaaacagt gcacgcaaag 15960
   aagctgaagc aggtatctct tccctgtgta acgccattga aacatatgca gatacggtaa 16020
   aacaggtaag aaaaagcatt gtacaagccc atcaagcaca acaaaaccgc caagaacttt 16080
   cctctgaacc ttctcaagac ttgcaaaaac agcaaagctt atcgaaacct cctaaactac 16140
   ctgaacactc accagaagac agacatcaag aatctactga aagctctatg caagctaaat 16200
   ggaaccggct tggtgctcga ccacgcacag tgcctctcag tcaaaaaacg caaaaacagc 16260
   aagaacaact tggtctgcat caacctatta tggaaggtga tggcaaagag gctgcaaaag 16320
   tcagcatgtc agcaattaaa gcaacgaaag ttacaacaaa gtccgaagca ttgaaagcag 16380
   tagaacagct aattcaaccc ccaaaagttg aaaccgcaaa agtagtttca gtgcctctcg 16440
   ctcaagaagc gcaaaaaaca cttattgaaa aggaaacaat tccatctctt accaacgaag 16500
   aagttgcttc aaagattcga gaaagtgaag tcgttaaaag cagcatgcaa aaaattaaaa 16560
   ctttgtgtgg tgttgtttat ggcaatcccc atattttgga aggtaaaatg ccgaagatgg 16620
   gaattccagt gacaaataga aatgtcgagg agttagaaaa gttcgcgcgc caagttggaa 16680
   gctttccctc ctcttttggt cgggttgccg gcttcagttt tttggggata aaaagtggag 16740
   cacgtgccca tgctgaagag aattttttgc ccttaagcca tgctattgtt agttacacac 16800
   ataatgtaaa acaagcagaa aaagacattc tagaagccca ttctgggaaa caagaacgct 16860
   atgcacagtc ggtagaaaca cccagtgaag aaatacaaaa tctattttct ctcactcaag 16920
   aagcgcaaaa aatgcttatt gaaaaagaaa taattccacc tcttagttac gtggacgttg 16980
   cttcaaagat tcgagaaagt gaagtcgtta aaagcagcaa gcaaaaaatt gaaactttgt 17040
   gtagtgttgt ttatggcaat ccccgtattt tggaagacaa aatgccgaag atgggaattc 17100
   cagtgacaaa taagaatgtc gaggagttag aaaagttcgc gcgtcaagtt ggaaactttc 17160
   catcctcttg tggtaagatc gttggcttca attttttggg gataaaaagt gaagcacgtg 17220
   cccatgctga agagaatttt ttgcccttaa gccatgctat ttttagttac gcacatagtg 17280
   taaaacaagc agaaaaggat attctagaag cctattttaa ggaacaagaa cgctgtgcac 17340
   agtcggtaga aacacccagt gaagagataa caaatctatt ttctttcact caagaacaac 17400
   agaaagaaat tttatcaaac tctcctaaac taagaacaca ggtaaaggct tattctcaaa 17460
   aactccataa ccgtctctca ccaagtgatc ttcaggctat aagtgaaaga tctcatacaa 17520
   aacttgctga aagtcttggc acatcagtaa atcaagcaga agaaattgca caaatcctca 17580
   cacaaacaaa agacgttgtc cagattcttc aacagcagga aaaacttggt ctgtatcaat 17640
   ctatcatgaa aggtgatggc aaagagactg caaaagtcag catgtcagca attaaggcaa 17700
   cggaagttac aacaaagttc gaagcatcca aagcagtaga acaggcagtt cgaccgccaa 17760
   aagttgaaac cgcaaaagta gtttcagtat ctttcactca agaaacgcaa aaaatgctta 17820
   ttgaaaaaga aataattcca cctcttagtt acgtggacgt tgcttcaaag attcgagaaa 17880
   gtgaagtcgt taaaagcagc atgcaaaaaa ttaaaacttt gtgtagtgtt gtttatggca 17940
   atcccgatat tttggaaggc aaaatgccga agatgggaat tccagtgaca aataagaatg 18000
   tcgaggagtt agaaaagttc gcgcgccagg ttggaaactt tccatcctct tgtggtaaga 18060
   tcgttggctt cagttttttg gggataaaaa gtgaagcacg tgcccatgct gaagagaatt 18120
   ttttgccctt aagccatgct atttttagtt acgcacatag tgtaaaacaa gcagaaaagg 18180
   atattctaga agcctatttt aaggaacaag aacgctgtgc acagtcggta gaaacaccca 18240
   gtgaagagat aacaaatcta ttttctctca ctcaagaaca acagaaagaa gttttattaa 18300
   cctctcctga actgagaaca caggtaaagg cttattctca aaaactctat gaccgtctct 18360
   caccaagtga tcttcaggct ataagtgaaa gatctcatac aaaacttgct gaaagtcttg 18420
   gcacatcagt aaatcaagca gaaaaaattg cacaaatcct cacacaaaca aaagacgttg 18480
   tccagattct tcaacagcag gaaaaacttg gtctgtatca atctatcatg aaaagtgatg 18540
   gcagagagac tgcaaaagtc aacatgtcag caattaaagc aacgcaaatg acaaaaaaga 18600
   tcacatcatt aaaagcagta gaacagatag ttcgaccgcc aaaagttgaa actgcaaaag 18660
   tagtttctat gagtagataa gcatacctga aactgtgttt atatggtccc tttgtgcgat 18720
   tgttttgtgt agattgtcga agagatgttg tttaatcata aaaattgaca tttctctttt 18780
   aagtaaagct ataaaaataa cgtaaaggtg gagagtgcac tttttaagct ttcaaaaaca 18840
   aatattgcta ttgagaaagc ttgaaaggaa atatctatga aaaaaaccaa acgaa 18895

<210> 2
<211> 1860
<212> DNA
<213> Bartonella henselae

<220>
<221> CDS
<222> (172)..(1806)
<223> bepA

<400> 2
aattgaaaaa gcgccgtgaa gcggttgatg cagtgatcag tacccatgca cttgaaggta 60

tagcgcttca tcccaaaacg ctcaaaattt tagaaggata tgcgagagga aatacctcac 120

tagaagaatt taacacgctt atggacaacg ctaaattata aggaggtcgt t atg cca 177
Met Pro
1

aag gca aaa gca aaa acg aaa aat aca gaa att att tcc ccc cat cat 225
Lys Ala Lys Ala Lys Thr Lys Asn Thr Glu Ile Ile Ser Pro His His
        5                   10                  15

tat gtc tat cct aac acc aca aca ctc aaa aat aaa tat gga ata aaa 273
Tyr Val Tyr Pro Asn Thr Thr Thr Leu Lys Asn Lys Tyr Gly Ile Lys
    20                  25                  30

aac tta aat gct ttt ctg gaa aaa tgc tcg cat gat aca gcc aaa gca 321
Asn Leu Asn Ala Phe Leu Glu Lys Cys Ser His Asp Thr Ala Lys Ala
35                  40                  45                  50

atg atc aat ctg cgt gaa gaa tcc tta cca gaa tat ttt gat acc gcc 369
Met Ile Asn Leu Arg Glu Glu Ser Leu Pro Glu Tyr Phe Asp Thr Ala
                55                  60                  65

tat cta tgc cat att cat caa cag ttg ttt aaa aat aca ttt gaa tgg 417
Tyr Leu Cys His Ile His Gln Gln Leu Phe Lys Asn Thr Phe Glu Trp
            70                  75                  80

gct gga tac ctt cgc cat atc ccc ttc aca ttt gca gat ggc acc aca 465
Ala Gly Tyr Leu Arg His Ile Pro Phe Thr Phe Ala Asp Gly Thr Thr
        85                  90                  95

gcc gcc atg cca gaa atg aaa aga acg ggt tgg aaa aat gct ttt gca 513
Ala Ala Met Pro Glu Met Lys Arg Thr Gly Trp Lys Asn Ala Phe Ala
    100                 105                 110

att ggt gat gag atc caa gaa ggc ttg caa aga tta gat caa aca ctt 561
Ile Gly Asp Glu Ile Gln Glu Gly Leu Gln Arg Leu Asp Gln Thr Leu
115                 120                 125                 130

gcc gaa aaa aac aat tta caa ggt tta acg cgc gaa gaa ttt aac tct 609

Ala Glu Lys Asn Asn Leu Gln Gly Leu Thr Arg Glu Glu Phe Asn Ser
                135                 140                 145

gaa gca ata gaa ctg ttt aat tct ctc aac caa ctc cac cca ttc aga      657
Glu Ala Ile Glu Leu Phe Asn Ser Leu Asn Gln Leu His Pro Phe Arg
            150                 155                 160

gaa ggg aat gga cga aca caa cgg cta ttt ttt gaa aat ctt gcc aaa      705
Glu Gly Asn Gly Arg Thr Gln Arg Leu Phe Phe Glu Asn Leu Ala Lys
        165                 170                 175

gca gca gga cat cag ctt aac ttc tcg ctt atc aca aaa gaa cgc atg      753
Ala Ala Gly His Gln Leu Asn Phe Ser Leu Ile Thr Lys Glu Arg Met
    180                 185                 190

atg gtt gcc agt gtt gcg gta gca gaa aat ggt gac cta gaa cct atg      801
Met Val Ala Ser Val Ala Val Ala Glu Asn Gly Asp Leu Glu Pro Met
195                 200                 205                 210

caa cat ttg ttt gag gat atc tcc aat cca gaa aaa att cgt ctt tta      849
Gln His Leu Phe Glu Asp Ile Ser Asn Pro Glu Lys Ile Arg Leu Leu
                215                 220                 225

aaa gaa ttt atg cac acc atg aaa aat act ggg cgc aat gtt aat gat      897
Lys Glu Phe Met His Thr Met Lys Asn Thr Gly Arg Asn Val Asn Asp
            230                 235                 240

cgt cct gtt atg gtt gca aag gaa ggg gaa act tac acg ggc acc tat      945
Arg Pro Val Met Val Ala Lys Glu Gly Glu Thr Tyr Thr Gly Thr Tyr
        245                 250                 255

aga ggt gct ggt ctt gaa ggt ttt gca ctc aat gta aaa ggt gct tac      993
Arg Gly Ala Gly Leu Glu Gly Phe Ala Leu Asn Val Lys Gly Ala Tyr
    260                 265                 270

att atc ggc aat ata gac cac ctc cca cca gaa caa cta aaa ata tta      1041
Ile Ile Gly Asn Ile Asp His Leu Pro Pro Glu Gln Leu Lys Ile Leu
275                 280                 285                 290

aaa cct ggt gat aaa att acc ttt aca gct cca aaa gcc gaa gaa tta      1089
Lys Pro Gly Asp Lys Ile Thr Phe Thr Ala Pro Lys Ala Glu Glu Leu
                295                 300                 305

aaa aaa aca ctc atc cca aaa gag aca tta gtt cct ttg aca aaa ctt      1137
Lys Lys Thr Leu Ile Pro Lys Glu Thr Leu Val Pro Leu Thr Lys Leu
            310                 315                 320

gaa ata gct gaa atg gtt gca gaa gat gct ttt gtc cac aca tgt cga      1185
Glu Ile Ala Glu Met Val Ala Glu Asp Ala Phe Val His Thr Cys Arg
        325                 330                 335

gac caa att tgc agt cta tca aaa att gtt tat ggc agc caa ggt gtt      1233
Asp Gln Ile Cys Ser Leu Ser Lys Ile Val Tyr Gly Ser Gln Gly Val
    340                 345                 350

ttg aat aaa aac ata ata gaa atc att aaa aat cca agc aag ggt cag      1281
Leu Asn Lys Asn Ile Ile Glu Ile Ile Lys Asn Pro Ser Lys Gly Gln
355                 360                 365                 370

caa ctc gcc aca caa att gaa aga aca cct tac tct gtt cat agt ctt      1329
Gln Leu Ala Thr Gln Ile Glu Arg Thr Pro Tyr Ser Val His Ser Leu
                375                 380                 385

gca ggt ttt gac ctc att tgc ttt aaa act ggg gca cgg gta cgt gct      1377

Ala Gly Phe Asp Leu Ile Cys Phe Lys Thr Gly Ala Arg Val Arg Ala
            390                 395                 400

gaa aag cat gtt gca ctc ctc agt tgt gca gtt gca aat ttt acc cat      1425
Glu Lys His Val Ala Leu Leu Ser Cys Ala Val Ala Asn Phe Thr His
        405                 410                 415

gca gta aaa cat gca aga caa gaa atc aca aaa gaa cat caa gca gaa      1473
Ala Val Lys His Ala Arg Gln Glu Ile Thr Lys Glu His Gln Ala Glu
    420                 425                 430

caa aac cgc ctt aga caa gag gtc cct atg cca agt caa agt ttg caa      1521
Gln Asn Arg Leu Arg Gln Glu Val Pro Met Pro Ser Gln Ser Leu Gln
435                 440                 445                 450

gat ctt ctt tcc ctg cca aaa gag ttc cag caa aaa gcc tta ggg gtc      1569
Asp Leu Leu Ser Leu Pro Lys Glu Phe Gln Gln Lys Ala Leu Gly Val
                455                 460                 465

tct cct ctg ctt caa aaa gag ctc acg agt ctt tta caa aaa gtc aat      1617
Ser Pro Leu Leu Gln Lys Glu Leu Thr Ser Leu Leu Gln Lys Val Asn
            470                 475                 480

agc cgt ctc tcc tca agt gaa caa aga gca ctt aga gaa aat aat cat      1665
Ser Arg Leu Ser Ser Ser Glu Gln Arg Ala Leu Arg Glu Asn Asn His
        485                 490                 495

gag aca ctg gcg aaa aat ctt ggt gtt tca gaa cag aaa gca aag gaa      1713
Glu Thr Leu Ala Lys Asn Leu Gly Val Ser Glu Gln Lys Ala Lys Glu
    500                 505                 510

att aca aaa act gtt atg aaa gcc aga gaa gta cag caa aaa tcg caa      1761
Ile Thr Lys Thr Val Met Lys Ala Arg Glu Val Gln Gln Lys Ser Gln
515                 520                 525                 530

aca cgc aca gtc agt cac tca aaa acg ctt gcc atg gct agc taa          1806
Thr Arg Thr Val Ser His Ser Lys Thr Leu Ala Met Ala Ser
                535                 540

agagtttgct caaaattgca aatttttgca ccttcctttt gaatcttcag ggga          1860


<210>  3
<211>  544
<212>  PRT
<213>  Bartonella henselae

<400>  3

Met Pro Lys Ala Lys Ala Lys Thr Lys Asn Thr Glu Ile Ile Ser Pro
1               5                   10                  15


His His Tyr Val Tyr Pro Asn Thr Thr Thr Leu Lys Asn Lys Tyr Gly
            20                  25                  30


Ile Lys Asn Leu Asn Ala Phe Leu Glu Lys Cys Ser His Asp Thr Ala
        35                  40                  45


Lys Ala Met Ile Asn Leu Arg Glu Glu Ser Leu Pro Glu Tyr Phe Asp
    50                  55                  60

Thr Ala Tyr Leu Cys His Ile His Gln Gln Leu Phe Lys Asn Thr Phe
65                  70                  75                  80

Glu Trp Ala Gly Tyr Leu Arg His Ile Pro Phe Thr Phe Ala Asp Gly
                85                  90                  95

Thr Thr Ala Ala Met Pro Glu Met Lys Arg Thr Gly Trp Lys Asn Ala
            100                 105                 110

Phe Ala Ile Gly Asp Glu Ile Gln Glu Gly Leu Gln Arg Leu Asp Gln
        115                 120                 125

Thr Leu Ala Glu Lys Asn Asn Leu Gln Gly Leu Thr Arg Glu Glu Phe
    130                 135                 140

Asn Ser Glu Ala Ile Glu Leu Phe Asn Ser Leu Asn Gln Leu His Pro
145                 150                 155                 160

Phe Arg Glu Gly Asn Gly Arg Thr Gln Arg Leu Phe Phe Glu Asn Leu
                165                 170                 175

Ala Lys Ala Ala Gly His Gln Leu Asn Phe Ser Leu Ile Thr Lys Glu
            180                 185                 190

Arg Met Met Val Ala Ser Val Ala Val Ala Glu Asn Gly Asp Leu Glu
        195                 200                 205

Pro Met Gln His Leu Phe Glu Asp Ile Ser Asn Pro Glu Lys Ile Arg
    210                 215                 220

Leu Leu Lys Glu Phe Met His Thr Met Lys Asn Thr Gly Arg Asn Val
225                 230                 235                 240

Asn Asp Arg Pro Val Met Val Ala Lys Glu Gly Glu Thr Tyr Thr Gly
                245                 250                 255

Thr Tyr Arg Gly Ala Gly Leu Glu Gly Phe Ala Leu Asn Val Lys Gly
            260                 265                 270

Ala Tyr Ile Ile Gly Asn Ile Asp His Leu Pro Pro Glu Gln Leu Lys
        275                 280                 285

Ile Leu Lys Pro Gly Asp Lys Ile Thr Phe Thr Ala Pro Lys Ala Glu
    290                 295                 300

Glu Leu Lys Lys Thr Leu Ile Pro Lys Glu Thr Leu Val Pro Leu Thr
305                 310                 315                 320

Lys Leu Glu Ile Ala Glu Met Val Ala Glu Asp Ala Phe Val His Thr
                325                 330                 335

Cys Arg Asp Gln Ile Cys Ser Leu Ser Lys Ile Val Tyr Gly Ser Gln
            340                 345                 350

Gly Val Leu Asn Lys Asn Ile Ile Glu Ile Ile Lys Asn Pro Ser Lys
        355                 360                 365

Gly Gln Gln Leu Ala Thr Gln Ile Glu Arg Thr Pro Tyr Ser Val His
    370                 375                 380

Ser Leu Ala Gly Phe Asp Leu Ile Cys Phe Lys Thr Gly Ala Arg Val
385                 390                 395                 400

Arg Ala Glu Lys His Val Ala Leu Leu Ser Cys Ala Val Ala Asn Phe
                405                 410                 415

Thr His Ala Val Lys His Ala Arg Gln Glu Ile Thr Lys Glu His Gln
            420                 425                 430

Ala Glu Gln Asn Arg Leu Arg Gln Glu Val Pro Met Pro Ser Gln Ser
        435                 440                 445

Leu Gln Asp Leu Leu Ser Leu Pro Lys Glu Phe Gln Gln Lys Ala Leu
    450                 455                 460

Gly Val Ser Pro Leu Leu Gln Lys Glu Leu Thr Ser Leu Leu Gln Lys
465                 470                 475                 480

Val Asn Ser Arg Leu Ser Ser Ser Glu Gln Arg Ala Leu Arg Glu Asn
                485                 490                 495

Asn His Glu Thr Leu Ala Lys Asn Leu Gly Val Ser Glu Gln Lys Ala
            500                 505                 510

Lys Glu Ile Thr Lys Thr Val Met Lys Ala Arg Glu Val Gln Gln Lys
        515                 520                 525

Ser Gln Thr Arg Thr Val Ser His Ser Lys Thr Leu Ala Met Ala Ser
    530                 535                 540

<210>  4
<211>  2040
<212>  DNA
<213>  Bartonella henselae

<220>
<221>  CDS

<222>  (109)..(2025)
<223>  virD4

<400>  4
aatggaatgt tatgctgtgc tgcccataaa aaagcgttca gcgctttatt accgatttta      60

acaagaagcc tttacggcat attccgataa aaccgaaagt aagacacg atg aaa tat      117
                                                     Met Lys Tyr
                                                     1

aca aag acg caa ctg gca ctc att tcc atg cca atc gct tca ggt gct      165
Thr Lys Thr Gln Leu Ala Leu Ile Ser Met Pro Ile Ala Ser Gly Ala
    5                   10                  15

tta acg ata ttt ctt gtt cct cat atg ttg tca ttt gtg ata aat gat      213
Leu Thr Ile Phe Leu Val Pro His Met Leu Ser Phe Val Ile Asn Asp
20                  25                  30                  35

ctg aaa acc aac caa atc tat tgg tat gtc cgt tca gaa ccc tta tta      261
Leu Lys Thr Asn Gln Ile Tyr Trp Tyr Val Arg Ser Glu Pro Leu Leu
                40                  45                  50

acg ctg atg ctg gtt gct gct gta tca tta ttt tat act ctc tca caa      309
Thr Leu Met Leu Val Ala Ala Val Ser Leu Phe Tyr Thr Leu Ser Gln
            55                  60                  65

aag ctg cac ttg cgc aaa gcg atc aca ttc gtc tca acg gcc ttt ttt      357
Lys Leu His Leu Arg Lys Ala Ile Thr Phe Val Ser Thr Ala Phe Phe
        70                  75                  80

tgc atc act gct ctt tac tat atc ggc agt gaa ata aaa cgc tta aat      405
Cys Ile Thr Ala Leu Tyr Tyr Ile Gly Ser Glu Ile Lys Arg Leu Asn
    85                  90                  95

ccc tat gtt gga caa cag ggg ata acc tgg ggc tat gct tta aaa ttc      453
Pro Tyr Val Gly Gln Gln Gly Ile Thr Trp Gly Tyr Ala Leu Lys Phe
100                 105                 110                 115

atg gat ccg atg gtt gtt ttc ggc gtc att tta ggc ttt gtt ctt tta      501
Met Asp Pro Met Val Val Phe Gly Val Ile Leu Gly Phe Val Leu Leu
                120                 125                 130

gca atc caa gtc ata atc acc tct cca cgc acg agc aat gtc aaa cgt      549
Ala Ile Gln Val Ile Ile Thr Ser Pro Arg Thr Ser Asn Val Lys Arg
            135                 140                 145

gcc aaa aaa gga att ttt ggt gac gcc gca tgg atg aat tta aaa gaa      597
Ala Lys Lys Gly Ile Phe Gly Asp Ala Ala Trp Met Asn Leu Lys Glu
        150                 155                 160

gcg gca aga att ttt ccc tcc aat ggc caa att gtt att ggc gaa aga      645
Ala Ala Arg Ile Phe Pro Ser Asn Gly Gln Ile Val Ile Gly Glu Arg
    165                 170                 175

tac cgt gtt gat caa gat aat gtt cgc aac att ccc ttt gca cca ggc      693
Tyr Arg Val Asp Gln Asp Asn Val Arg Asn Ile Pro Phe Ala Pro Gly
180                 185                 190                 195

aat aaa aca aca tgg ggc aag ggt gga aca gcc cct ttg ctg acc ttt      741
Asn Lys Thr Thr Trp Gly Lys Gly Gly Thr Ala Pro Leu Leu Thr Phe
                200                 205                 210

aat ctt gat ttt ggt tca acg cac atg att ttt ttc gcc ggt tct ggt      789

Asn Leu Asp Phe Gly Ser Thr His Met Ile Phe Phe Ala Gly Ser Gly
            215                 220                 225

gga tat aaa aca aca agc aca gtg gtt cca acc tgt cta acc tac aca      837
Gly Tyr Lys Thr Thr Ser Thr Val Val Pro Thr Cys Leu Thr Tyr Thr
        230                 235                 240

ggc cct att gtt tgc ctt gat cct tca aca gaa att gca ccc atg gtc      885
Gly Pro Ile Val Cys Leu Asp Pro Ser Thr Glu Ile Ala Pro Met Val
    245                 250                 255

aag ttt gcg cgc aaa aaa atg ggc aat aga aat gtt att att ctc gat      933
Lys Phe Ala Arg Lys Lys Met Gly Asn Arg Asn Val Ile Ile Leu Asp
260                 265                 270                 275

ccc aat tcg ctc tta aca aaa aat ttt aat gtg ctc gat tgg ctt tta      981
Pro Asn Ser Leu Leu Thr Lys Asn Phe Asn Val Leu Asp Trp Leu Leu
                280                 285                 290

gac gaa aat ata ccg cgc aca agg cgt gaa gcc aat att gtg agc ttt      1029
Asp Glu Asn Ile Pro Arg Thr Arg Arg Glu Ala Asn Ile Val Ser Phe
            295                 300                 305

tcc aaa ctg ctt ctc tca gag aaa aaa tca gaa aat tct tca gca gaa      1077
Ser Lys Leu Leu Leu Ser Glu Lys Lys Ser Glu Asn Ser Ser Ala Glu
        310                 315                 320

tat ttt tca aca caa gcg cat aat ctt tta acc gca ctt ctt gca cat      1125
Tyr Phe Ser Thr Gln Ala His Asn Leu Leu Thr Ala Leu Leu Ala His
    325                 330                 335

gta atc ttc tct gat aaa tat gaa gac agt gag cgt aat ctc aaa aca      1173
Val Ile Phe Ser Asp Lys Tyr Glu Asp Ser Glu Arg Asn Leu Lys Thr
340                 345                 350                 355

tta cgc gcg atc ctc tct cag tca gaa aca gct gtt gtc aat caa tta      1221
Leu Arg Ala Ile Leu Ser Gln Ser Glu Thr Ala Val Val Asn Gln Leu
                360                 365                 370

cgt atg att caa gaa aca aca ccg tct cct ttt att cgt gaa atg gtt      1269
Arg Met Ile Gln Glu Thr Thr Pro Ser Pro Phe Ile Arg Glu Met Val
            375                 380                 385

ggt att ttc acg gaa atg gca gat caa acc ttt tca ggg gtc tac aca      1317
Gly Ile Phe Thr Glu Met Ala Asp Gln Thr Phe Ser Gly Val Tyr Thr
        390                 395                 400

act gcc tcc aaa gac acc caa tgg ctt tct ttg tca aat tac gct gat      1365
Thr Ala Ser Lys Asp Thr Gln Trp Leu Ser Leu Ser Asn Tyr Ala Asp
    405                 410                 415

ctt gtt tgt ggg aat gat ttt gca tcc tcg gat att gca aac gga aaa      1413
Leu Val Cys Gly Asn Asp Phe Ala Ser Ser Asp Ile Ala Asn Gly Lys
420                 425                 430                 435

aca gat gtt ttt ctt aat ctt cct gcc agt att ttg aac agt tat cca      1461
Thr Asp Val Phe Leu Asn Leu Pro Ala Ser Ile Leu Asn Ser Tyr Pro
                440                 445                 450

gcg att ggc cgc gtg atc att ggc gct ttt tta aat gcg atg gtt aca      1509
Ala Ile Gly Arg Val Ile Ile Gly Ala Phe Leu Asn Ala Met Val Thr
            455                 460                 465

gca gat gga aac tat aaa aag cgt gtt ttg ttt gtc ttg gat gaa gtc      1557

Ala Asp Gly Asn Tyr Lys Lys Arg Val Leu Phe Val Leu Asp Glu Val
        470                 475                 480

gat ctt ctt ggt tat atg aat att tta gaa gaa gca cgt gat cgt ggg      1605
Asp Leu Leu Gly Tyr Met Asn Ile Leu Glu Glu Ala Arg Asp Arg Gly
    485                 490                 495

cgt aag tac ggc aca tcc ttg atg ctt ttt tat caa tcc tct ggt cag      1653
Arg Lys Tyr Gly Thr Ser Leu Met Leu Phe Tyr Gln Ser Ser Gly Gln
500                 505                 510                 515

ttg gtt aat cac ttt gga gaa gca gga gcg cgt tca tgg ttt gaa agc      1701
Leu Val Asn His Phe Gly Glu Ala Gly Ala Arg Ser Trp Phe Glu Ser
                520                 525                 530

tgc tct ttt gtg agt tac gcc gcc att aaa gat ctc caa aca gcc aaa      1749
Cys Ser Phe Val Ser Tyr Ala Ala Ile Lys Asp Leu Gln Thr Ala Lys
            535                 540                 545

gac att tcg gaa cgc tgt ggt caa atg acc att gaa gta act gga acg      1797
Asp Ile Ser Glu Arg Cys Gly Gln Met Thr Ile Glu Val Thr Gly Thr
        550                 555                 560

agc aaa tcg aga ggt ttg tct tta aca aaa ggt tct caa aac atc aac      1845
Ser Lys Ser Arg Gly Leu Ser Leu Thr Lys Gly Ser Gln Asn Ile Asn
    565                 570                 575

tat caa caa aga gca ctg att tta ccc cac gag att att caa gag atg      1893
Tyr Gln Gln Arg Ala Leu Ile Leu Pro His Glu Ile Ile Gln Glu Met
580                 585                 590                 595

cgc caa gat gaa caa att att ctt atg caa ggg cac ccc cct tta cga      1941
Arg Gln Asp Glu Gln Ile Ile Leu Met Gln Gly His Pro Pro Leu Arg
                600                 605                 610

tgc ggt cga gcg atc tat ttt aga agg aaa gag atg tta gca gct act      1989
Cys Gly Arg Ala Ile Tyr Phe Arg Arg Lys Glu Met Leu Ala Ala Thr
            615                 620                 625

gaa aaa aac cgc ttt gcc cca caa gca aag aaa agc tgaaaaagca ccctc     2040
Glu Lys Asn Arg Phe Ala Pro Gln Ala Lys Lys Ser
        630                 635


<210>  5
<211>  639
<212>  PRT
<213>  Bartonella henselae

<400>  5

Met Lys Tyr Thr Lys Thr Gln Leu Ala Leu Ile Ser Met Pro Ile Ala
1               5                   10                  15


Ser Gly Ala Leu Thr Ile Phe Leu Val Pro His Met Leu Ser Phe Val
            20                  25                  30


Ile Asn Asp Leu Lys Thr Asn Gln Ile Tyr Trp Tyr Val Arg Ser Glu
        35                  40                  45


Pro Leu Leu Thr Leu Met Leu Val Ala Ala Val Ser Leu Phe Tyr Thr

50                  55                  60

Leu Ser Gln Lys Leu His Leu Arg Lys Ala Ile Thr Phe Val Ser Thr
65                  70                  75                  80

Ala Phe Phe Cys Ile Thr Ala Leu Tyr Tyr Ile Gly Ser Glu Ile Lys
                85                  90                  95

Arg Leu Asn Pro Tyr Val Gly Gln Gln Gly Ile Thr Trp Gly Tyr Ala
            100                 105                 110

Leu Lys Phe Met Asp Pro Met Val Val Phe Gly Val Ile Leu Gly Phe
        115                 120                 125

Val Leu Leu Ala Ile Gln Val Ile Ile Thr Ser Pro Arg Thr Ser Asn
    130                 135                 140

Val Lys Arg Ala Lys Lys Gly Ile Phe Gly Asp Ala Ala Trp Met Asn
145                 150                 155                 160

Leu Lys Glu Ala Ala Arg Ile Phe Pro Ser Asn Gly Gln Ile Val Ile
                165                 170                 175

Gly Glu Arg Tyr Arg Val Asp Gln Asp Asn Val Arg Asn Ile Pro Phe
            180                 185                 190

Ala Pro Gly Asn Lys Thr Thr Trp Gly Lys Gly Gly Thr Ala Pro Leu
        195                 200                 205

Leu Thr Phe Asn Leu Asp Phe Gly Ser Thr His Met Ile Phe Phe Ala
    210                 215                 220

Gly Ser Gly Gly Tyr Lys Thr Thr Ser Thr Val Val Pro Thr Cys Leu
225                 230                 235                 240

Thr Tyr Thr Gly Pro Ile Val Cys Leu Asp Pro Ser Thr Glu Ile Ala
                245                 250                 255

Pro Met Val Lys Phe Ala Arg Lys Lys Met Gly Asn Arg Asn Val Ile
            260                 265                 270

Ile Leu Asp Pro Asn Ser Leu Leu Thr Lys Asn Phe Asn Val Leu Asp
        275                 280                 285

Trp Leu Leu Asp Glu Asn Ile Pro Arg Thr Arg Arg Glu Ala Asn Ile
    290                 295                 300

Val Ser Phe Ser Lys Leu Leu Leu Ser Glu Lys Lys Ser Glu Asn Ser

305                 310                 315                 320

Ser Ala Glu Tyr Phe Ser Thr Gln Ala His Asn Leu Leu Thr Ala Leu
                325                 330                 335

Leu Ala His Val Ile Phe Ser Asp Lys Tyr Glu Asp Ser Glu Arg Asn
            340                 345                 350

Leu Lys Thr Leu Arg Ala Ile Leu Ser Gln Ser Glu Thr Ala Val Val
        355                 360                 365

Asn Gln Leu Arg Met Ile Gln Glu Thr Thr Pro Ser Pro Phe Ile Arg
    370                 375                 380

Glu Met Val Gly Ile Phe Thr Glu Met Ala Asp Gln Thr Phe Ser Gly
385                 390                 395                 400

Val Tyr Thr Thr Ala Ser Lys Asp Thr Gln Trp Leu Ser Leu Ser Asn
                405                 410                 415

Tyr Ala Asp Leu Val Cys Gly Asn Asp Phe Ala Ser Ser Asp Ile Ala
            420                 425                 430

Asn Gly Lys Thr Asp Val Phe Leu Asn Leu Pro Ala Ser Ile Leu Asn
        435                 440                 445

Ser Tyr Pro Ala Ile Gly Arg Val Ile Ile Gly Ala Phe Leu Asn Ala
    450                 455                 460

Met Val Thr Ala Asp Gly Asn Tyr Lys Lys Arg Val Leu Phe Val Leu
465                 470                 475                 480

Asp Glu Val Asp Leu Leu Gly Tyr Met Asn Ile Leu Glu Glu Ala Arg
                485                 490                 495

Asp Arg Gly Arg Lys Tyr Gly Thr Ser Leu Met Leu Phe Tyr Gln Ser
            500                 505                 510

Ser Gly Gln Leu Val Asn His Phe Gly Glu Ala Gly Ala Arg Ser Trp
        515                 520                 525

Phe Glu Ser Cys Ser Phe Val Ser Tyr Ala Ala Ile Lys Asp Leu Gln
    530                 535                 540

Thr Ala Lys Asp Ile Ser Glu Arg Cys Gly Gln Met Thr Ile Glu Val
545                 550                 555                 560

Thr Gly Thr Ser Lys Ser Arg Gly Leu Ser Leu Thr Lys Gly Ser Gln

565                 570                 575

Asn Ile Asn Tyr Gln Gln Arg Ala Leu Ile Leu Pro His Glu Ile Ile
            580                 585                 590

Gln Glu Met Arg Gln Asp Glu Gln Ile Ile Leu Met Gln Gly His Pro
        595                 600                 605

Pro Leu Arg Cys Gly Arg Ala Ile Tyr Phe Arg Arg Lys Glu Met Leu
    610                 615                 620

Ala Ala Thr Glu Lys Asn Arg Phe Ala Pro Gln Ala Lys Lys Ser
625                 630                 635

<210>  6
<211>  2160
<212>  DNA
<213>  Bartonella henselae

<220>
<221>  CDS
<222>  (530)..(2155)
<223>  bepB

<400>  6
tgacggctgc tcttaagcct caaaacaaga agaacagcat acaataaaaa aatcagagat      60

tcttgtttta taaaaacgtc ttaaaaagga attttcacat gacccaacaa gaaaaacatg     120

gtgaaaaaag acctttgagc agtgttgttt ttcacgggct taccaatgcc agcactcttt     180

atgaagcgct ctcctcatca ccaaacaatg cgtaccaatc atatctatct taagatacct     240

ctcaagaaac ttttgaagaa agctctttta caggaaaatc gcctagaaaa atttttcaca     300

aaaaataaaa agacaccatt catgaaaaca acaaagaccg atccttctct cttaaaaaaa     360

gaaaaaagca ccaagataag gattggtaca agcaaaaaca ctcatgaatt tgagggatcc     420

ctcttccctc gttcaaagct tgaaatctca aaagaacatg cgagggggaa tatttcatga     480

gaagaattta acactctcat ggacagtgcg acatgataag gagttaaca atg cca aaa     538
                                                      Met Pro Lys
                                                      1

gca aaa gca aaa aat atc tcc acc gct tct ccc cat aat tat atc tat     586
Ala Lys Ala Lys Asn Ile Ser Thr Ala Ser Pro His Asn Tyr Ile Tyr
    5                   10                  15

cct ggt acc caa ata ctc aaa aat aaa tat gga gaa aca gat tta aaa     634
Pro Gly Thr Gln Ile Leu Lys Asn Lys Tyr Gly Glu Thr Asp Leu Lys
20                  25                  30                  35

ctc ctt ctg gaa aaa tgc tta cat gat agg gaa caa gca atg atg aat     682
Leu Leu Leu Glu Lys Cys Leu His Asp Arg Glu Gln Ala Met Met Asn
                40                  45                  50

ctg cgt gca gaa tcc cta cca gaa tat ttt gat tgc gct tat cta tgc     730

Leu Arg Ala Glu Ser Leu Pro Glu Tyr Phe Asp Cys Ala Tyr Leu Cys
            55                  60                  65

tat atc cac caa caa ttg ttt caa aaa acc ttt gaa tgg gct ggc cat      778
Tyr Ile His Gln Gln Leu Phe Gln Lys Thr Phe Glu Trp Ala Gly His
        70                  75                  80

ctc cgc cac act ccc ttc aca ttt gca gat ggt agc att gcc gcc atg      826
Leu Arg His Thr Pro Phe Thr Phe Ala Asp Gly Ser Ile Ala Ala Met
    85                  90                  95

cca gaa atg aaa aga aca gaa tgg ggc aaa gat ttt gca aaa agt gaa      874
Pro Glu Met Lys Arg Thr Glu Trp Gly Lys Asp Phe Ala Lys Ser Glu
100                 105                 110                 115

aaa atc ccc gaa ctt tta caa aaa tta gat caa gag ctt gcc gaa aaa      922
Lys Ile Pro Glu Leu Leu Gln Lys Leu Asp Gln Glu Leu Ala Glu Lys
                120                 125                 130

gac aat ttg caa ggc tta aca cgc gaa gag ttt atc aag caa gca acg      970
Asp Asn Leu Gln Gly Leu Thr Arg Glu Glu Phe Ile Lys Gln Ala Thr
            135                 140                 145

gag cta ttt tat tct ctt cac aaa atc cac cca ttc ata gat ggc aat      1018
Glu Leu Phe Tyr Ser Leu His Lys Ile His Pro Phe Ile Asp Gly Asn
        150                 155                 160

gaa cac aca gaa caa ttc ttt ttt gaa aat ctc gcc aaa gcc gcc gga      1066
Glu His Thr Glu Gln Phe Phe Phe Glu Asn Leu Ala Lys Ala Ala Gly
    165                 170                 175

cat caa ctt gac ttt tcg ctt gtc aca caa aaa cgc atg atg gca gtc      1114
His Gln Leu Asp Phe Ser Leu Val Thr Gln Lys Arg Met Met Ala Val
180                 185                 190                 195

tgt agc gaa gca atg caa tat ggc gat aca caa ctg atg aaa gat ctt      1162
Cys Ser Glu Ala Met Gln Tyr Gly Asp Thr Gln Leu Met Lys Asp Leu
                200                 205                 210

ttt gaa gat atc tcc aat cca gaa aaa ata cgt ctt tta aaa gac ttt      1210
Phe Glu Asp Ile Ser Asn Pro Glu Lys Ile Arg Leu Leu Lys Asp Phe
            215                 220                 225

atg aac aac atc aac aat aca gga cgc aac gtg aat gat tgc ctt gtt      1258
Met Asn Asn Ile Asn Asn Thr Gly Arg Asn Val Asn Asp Cys Leu Val
        230                 235                 240

atg gtg aca aag gca gga gaa acc tac aga gga acc tat caa ggt tgt      1306
Met Val Thr Lys Ala Gly Glu Thr Tyr Arg Gly Thr Tyr Gln Gly Cys
    245                 250                 255

tat tca gaa gct ttt gtg ctc gat atg caa ggg act tac atc atc ggc      1354
Tyr Ser Glu Ala Phe Val Leu Asp Met Gln Gly Thr Tyr Ile Ile Gly
260                 265                 270                 275

aaa aaa gat gat ctc aca cca gaa cga tta aaa tca tta aaa cct ggt      1402
Lys Lys Asp Asp Leu Thr Pro Glu Arg Leu Lys Ser Leu Lys Pro Gly
                280                 285                 290

gat aca att acc ttt atc gcc ccc aaa aac aaa gag ctt gaa aac acg      1450
Asp Thr Ile Thr Phe Ile Ala Pro Lys Asn Lys Glu Leu Glu Asn Thr
            295                 300                 305

ctc att cca aaa gag aca tta gcg cct cta aca aaa agt gaa tgt gcc      1498

Leu Ile Pro Lys Glu Thr Leu Ala Pro Leu Thr Lys Ser Glu Cys Ala
        310                 315                 320

aga acg gtt gcg caa act agc cat gtc ctc aca gct caa aag aaa gtc      1546
Arg Thr Val Ala Gln Thr Ser His Val Leu Thr Ala Gln Lys Lys Val
    325                 330                 335

cgg caa cac tca aaa att gtt tat ggc aat gca aac aca tta aac gaa      1594
Arg Gln His Ser Lys Ile Val Tyr Gly Asn Ala Asn Thr Leu Asn Glu
340                 345                 350                 355

caa ata gaa gag att act aaa aat cca gaa tta ggt caa ctg ctc gcc      1642
Gln Ile Glu Glu Ile Thr Lys Asn Pro Glu Leu Gly Gln Leu Leu Ala
                360                 365                 370

gat cag att aga caa aca ccg cac tct ctt tct cct ctt gca ggt ttc      1690
Asp Gln Ile Arg Gln Thr Pro His Ser Leu Ser Pro Leu Ala Gly Phe
            375                 380                 385

agt cta tgc ggt tta caa aat ccg gca cgt gca aac gct cga aat cat      1738
Ser Leu Cys Gly Leu Gln Asn Pro Ala Arg Ala Asn Ala Arg Asn His
        390                 395                 400

ctt gat gca ctc gct act aca gtt gca aat tat gca cat att gta gaa      1786
Leu Asp Ala Leu Ala Thr Thr Val Ala Asn Tyr Ala His Ile Val Glu
    405                 410                 415

cat aca tat cat gta atc act caa aaa cat caa att gaa caa gaa cgc      1834
His Thr Tyr His Val Ile Thr Gln Lys His Gln Ile Glu Gln Glu Arg
420                 425                 430                 435

ctt ggt aaa aca gta gaa aag ccc agt caa aac ttg caa aac ctt ttt      1882
Leu Gly Lys Thr Val Glu Lys Pro Ser Gln Asn Leu Gln Asn Leu Phe
                440                 445                 450

gcc tta tca ccg gaa caa caa aga gaa atc ttg tct caa tct cct caa      1930
Ala Leu Ser Pro Glu Gln Gln Arg Glu Ile Leu Ser Gln Ser Pro Gln
            455                 460                 465

tta tac caa gag ctc cgt act ttt ata tgc tgt gta gag cat cgt ctc      1978
Leu Tyr Gln Glu Leu Arg Thr Phe Ile Cys Cys Val Glu His Arg Leu
        470                 475                 480

tca tca aac gag tgg agt gcg atc aaa aac aaa gat tat gaa aca ctt      2026
Ser Ser Asn Glu Trp Ser Ala Ile Lys Asn Lys Asp Tyr Glu Thr Leu
    485                 490                 495

gct caa agt atc ggt gta tca gaa cag aca gca cga gag att acc aat      2074
Ala Gln Ser Ile Gly Val Ser Glu Gln Thr Ala Arg Glu Ile Thr Asn
500                 505                 510                 515

act gtt caa aaa gcc aga gag tta cat aaa caa gtc tac aca cac gca      2122
Thr Val Gln Lys Ala Arg Glu Leu His Lys Gln Val Tyr Thr His Ala
                520                 525                 530

atc act cgc tca aat acg ctt gct att gcc agc taaaa                    2160
Ile Thr Arg Ser Asn Thr Leu Ala Ile Ala Ser
            535                 540

<210>  7
<211>  542
<212>  PRT
<213>  Bartonella henselae

<400> 7

Met Pro Lys Ala Lys Ala Lys Asn Ile Ser Thr Ala Ser Pro His Asn
1               5                   10                  15

Tyr Ile Tyr Pro Gly Thr Gln Ile Leu Lys Asn Lys Tyr Gly Glu Thr
            20                  25                  30

Asp Leu Lys Leu Leu Leu Glu Lys Cys Leu His Asp Arg Glu Gln Ala
        35                  40                  45

Met Met Asn Leu Arg Ala Glu Ser Leu Pro Glu Tyr Phe Asp Cys Ala
    50                  55                  60

Tyr Leu Cys Tyr Ile His Gln Gln Leu Phe Gln Lys Thr Phe Glu Trp
65                  70                  75                  80

Ala Gly His Leu Arg His Thr Pro Phe Thr Phe Ala Asp Gly Ser Ile
                85                  90                  95

Ala Ala Met Pro Glu Met Lys Arg Thr Glu Trp Gly Lys Asp Phe Ala
            100                 105                 110

Lys Ser Glu Lys Ile Pro Glu Leu Leu Gln Lys Leu Asp Gln Glu Leu
        115                 120                 125

Ala Glu Lys Asp Asn Leu Gln Gly Leu Thr Arg Glu Glu Phe Ile Lys
    130                 135                 140

Gln Ala Thr Glu Leu Phe Tyr Ser Leu His Lys Ile His Pro Phe Ile
145                 150                 155                 160

Asp Gly Asn Glu His Thr Glu Gln Phe Phe Phe Glu Asn Leu Ala Lys
                165                 170                 175

Ala Ala Gly His Gln Leu Asp Phe Ser Leu Val Thr Gln Lys Arg Met
            180                 185                 190

Met Ala Val Cys Ser Glu Ala Met Gln Tyr Gly Asp Thr Gln Leu Met
        195                 200                 205

Lys Asp Leu Phe Glu Asp Ile Ser Asn Pro Glu Lys Ile Arg Leu Leu
    210                 215                 220

Lys Asp Phe Met Asn Asn Ile Asn Asn Thr Gly Arg Asn Val Asn Asp
225                 230                 235                 240

Cys Leu Val Met Val Thr Lys Ala Gly Glu Thr Tyr Arg Gly Thr Tyr

                245                 250                 255

Gln Gly Cys Tyr Ser Glu Ala Phe Val Leu Asp Met Gln Gly Thr Tyr
            260                 265                 270

Ile Ile Gly Lys Lys Asp Asp Leu Thr Pro Glu Arg Leu Lys Ser Leu
        275                 280                 285

Lys Pro Gly Asp Thr Ile Thr Phe Ile Ala Pro Lys Asn Lys Glu Leu
    290                 295                 300

Glu Asn Thr Leu Ile Pro Lys Glu Thr Leu Ala Pro Leu Thr Lys Ser
305                 310                 315                 320

Glu Cys Ala Arg Thr Val Ala Gln Thr Ser His Val Leu Thr Ala Gln
                325                 330                 335

Lys Lys Val Arg Gln His Ser Lys Ile Val Tyr Gly Asn Ala Asn Thr
            340                 345                 350

Leu Asn Glu Gln Ile Glu Glu Ile Thr Lys Asn Pro Glu Leu Gly Gln
        355                 360                 365

Leu Leu Ala Asp Gln Ile Arg Gln Thr Pro His Ser Leu Ser Pro Leu
    370                 375                 380

Ala Gly Phe Ser Leu Cys Gly Leu Gln Asn Pro Ala Arg Ala Asn Ala
385                 390                 395                 400

Arg Asn His Leu Asp Ala Leu Ala Thr Thr Val Ala Asn Tyr Ala His
                405                 410                 415

Ile Val Glu His Thr Tyr His Val Ile Thr Gln Lys His Gln Ile Glu
            420                 425                 430

Gln Glu Arg Leu Gly Lys Thr Val Glu Lys Pro Ser Gln Asn Leu Gln
        435                 440                 445

Asn Leu Phe Ala Leu Ser Pro Glu Gln Gln Arg Glu Ile Leu Ser Gln
    450                 455                 460

Ser Pro Gln Leu Tyr Gln Glu Leu Arg Thr Phe Ile Cys Cys Val Glu
465                 470                 475                 480

His Arg Leu Ser Ser Asn Glu Trp Ser Ala Ile Lys Asn Lys Asp Tyr
                485                 490                 495

Glu Thr Leu Ala Gln Ser Ile Gly Val Ser Glu Gln Thr Ala Arg Glu

500                 505                 510

Ile Thr Asn Thr Val Gln Lys Ala Arg Glu Leu His Lys Gln Val Tyr
        515                 520                 525

Thr His Ala Ile Thr Arg Ser Asn Thr Leu Ala Ile Ala Ser
    530                 535                 540

<210>  8
<211>  2700
<212>  DNA
<213>  Bartonella henselae

<220>
<221>  CDS
<222>  (1068)..(2663)
<223>  bepC

<400>  8
aatatgaggc cttgttgctt aatctgaaca cccatgtgga agaaatgttt taatattccc      60

acacccatga aagaacagag cacttaagcg ccctattact aattttgcaa atagcataga     120

catcatatcc agattaattg ggcggatgat tatgagtacc tttttaaacg ccaggattac     180

agctgacgga aactacaaaa agcgtgtgtt gtttatctta gatgaagtgg accttcatgg     240

ttataggaat attttaaaag ggcgctcatt ttacctcccc gaaaaacgcg ccaaaatgaa     300

taaattattc tcatgcaaga acatccccct ttacgatgtg gttgcacgat ctattttaga     360

aggaaaaaaa tgttagcaac tgctgaaaag aactgttttg cccccaaagc acagaaaaac     420

taaaaaccat cctcttatgg agaccatgta tcaaaacaaa aacaacaaca tcaaaggata     480

aaagcaaagg ttctgtttca taaaagcata taaacaagga atttccgcat gatccagcaa     540

gaaaaataag atgaaaaaag atctttgagc aatgctgttt tgcatgggct taccaacacg     600

cgcactcttc gtaaagagtc cctaaacgca caagcggtca atgacaatga tacgcaccat     660

gtccatatgg aagagtccct tatttaaaca cccagaaggc gggctttatg aaaacaactt     720

tattaaaagc taggaaaaat tatcatttat gaaaatgata cacaaagggg gaatacatac     780

caaaaaagct ttccacaaaa tgaaaaaagg aaagaaatct gcatgaaaaa acccaaacgg     840

actgttttgc agtttttata ccagaagaat taaaaaaaca caatgaaacg gttgatacat     900

ccttcagcac acatgttctc atcatctatc agtcttcatg tttttcagga agcgttcttc     960

cctgaaaaaa aatgttcgaa atcccaacaa aatacgcgaa aggggacttt tcacatgaaa    1020

attttaacat tctcataaac cacacacctc tctcaaggag aaaatat atg tta gag      1076
                                                    Met Leu Glu
                                                    1

cat aat tat ctg tat aaa aac agc aac aca ctg aaa aat aaa tat ggc      1124
His Asn Tyr Leu Tyr Lys Asn Ser Asn Thr Leu Lys Asn Lys Tyr Gly
    5                   10                  15

ata aaa gac ccg caa aga ctg tat gag cgc tgt gcc cat gat aca gcc 1172
Ile Lys Asp Pro Gln Arg Leu Tyr Glu Arg Cys Ala His Asp Thr Ala
20                  25                  30                  35

aga gag gtt atg aat ctt cgc ttt gaa cca ccc cca caa aga ttt ggt 1220
Arg Glu Val Met Asn Leu Arg Phe Glu Pro Pro Pro Gln Arg Phe Gly
                40                  45                  50

ctc gct tat ttg aaa ttg atc cat tgg acc ctc ttc cat agg agt ttt 1268
Leu Ala Tyr Leu Lys Leu Ile His Trp Thr Leu Phe His Arg Ser Phe
            55                  60                  65

gaa tgg gcg ggt cat act cgt gat gag ccc ttt aca ttt gaa gat ggt 1316
Glu Trp Ala Gly His Thr Arg Asp Glu Pro Phe Thr Phe Glu Asp Gly
        70                  75                  80

agc acc gcc cgt atg cca gct atg cgc cca aaa ggt cat agg gtt cct 1364
Ser Thr Ala Arg Met Pro Ala Met Arg Pro Lys Gly His Arg Val Pro
    85                  90                  95

ttt gcc gtt ggt cca cag att caa aaa gaa ctt aaa caa tta gag aga 1412
Phe Ala Val Gly Pro Gln Ile Gln Lys Glu Leu Lys Gln Leu Glu Arg
100                 105                 110                 115

aaa ctt acc gca aag aat aac tta caa ggc tta tca cgc caa gag ttt 1460
Lys Leu Thr Ala Lys Asn Asn Leu Gln Gly Leu Ser Arg Gln Glu Phe
                120                 125                 130

gct gaa agt gct gcg gaa gtt ttt atg act ctc gac cac gcg cat cct 1508
Ala Glu Ser Ala Ala Glu Val Phe Met Thr Leu Asp His Ala His Pro
            135                 140                 145

ttc aga aaa ggc aat ggg cgc gcg caa aga ata ttt atg gaa aag ctt 1556
Phe Arg Lys Gly Asn Gly Arg Ala Gln Arg Ile Phe Met Glu Lys Leu
        150                 155                 160

gga caa gca gca ggc tat aag att gac ttt tct ttt atc aca aaa gag 1604
Gly Gln Ala Ala Gly Tyr Lys Ile Asp Phe Ser Phe Ile Thr Lys Glu
    165                 170                 175

cgc ctc acc caa gct agt att gcc gca atg caa cat ggc aac aca caa 1652
Arg Leu Thr Gln Ala Ser Ile Ala Ala Met Gln His Gly Asn Thr Gln
180                 185                 190                 195

ccc atg aaa gat ctt ttt gag gat atc acc cac ccg caa aaa ttc ctt 1700
Pro Met Lys Asp Leu Phe Glu Asp Ile Thr His Pro Gln Lys Phe Leu
                200                 205                 210

ctt tta aag gaa ttc atc tct caa atg aga aat agc gga ctt gag gaa 1748
Leu Leu Lys Glu Phe Ile Ser Gln Met Arg Asn Ser Gly Leu Glu Glu
            215                 220                 225

atc aac gaa cat att gtt gtc gta gca aaa gaa ggt gta aag tat gat 1796
Ile Asn Glu His Ile Val Val Val Ala Lys Glu Gly Val Lys Tyr Asp
        230                 235                 240

ggc atc tat aaa ggt tgt gct gca gaa ggt ttt gtc ata gaa tta gaa 1844
Gly Ile Tyr Lys Gly Cys Ala Ala Glu Gly Phe Val Ile Glu Leu Glu
    245                 250                 255

gat ggt ttt gtt gtc ggc cac aaa gat gat ctt acg cca gaa caa gtc 1892
Asp Gly Phe Val Val Gly His Lys Asp Asp Leu Thr Pro Glu Gln Val
260                 265                 270                 275

aaa aca tta cag aac ggt gat cgc ata gtc ttt gaa aaa agc aat gtt     1940
Lys Thr Leu Gln Asn Gly Asp Arg Ile Val Phe Glu Lys Ser Asn Val
                280                 285                 290

ctc gat ctg aaa gaa ata ttg att cca aaa gag acc ttg ccc cct ctc     1988
Leu Asp Leu Lys Glu Ile Leu Ile Pro Lys Glu Thr Leu Pro Pro Leu
            295                 300                 305

acc aat gaa gaa ctt gcc aaa agg ttc aca gat aat tgc ggt gtt gaa     2036
Thr Asn Glu Glu Leu Ala Lys Arg Phe Thr Asp Asn Cys Gly Val Glu
        310                 315                 320

tca tac cgc caa gaa gtc gaa cat tta tca aaa cgt gtt tat ggc aag     2084
Ser Tyr Arg Gln Glu Val Glu His Leu Ser Lys Arg Val Tyr Gly Lys
    325                 330                 335

tca caa gca ctc aac gaa atg ata gag tcg atc aat ata gat cca agc     2132
Ser Gln Ala Leu Asn Glu Met Ile Glu Ser Ile Asn Ile Asp Pro Ser
340                 345                 350                 355

tta ggc gca gag ttt gcc gat caa att gct caa aat cct aaa tca ttt     2180
Leu Gly Ala Glu Phe Ala Asp Gln Ile Ala Gln Asn Pro Lys Ser Phe
                360                 365                 370

tgt aaa ctt gcg ggg agg aaa ata tta ggc ata aga agt cca agt cgc     2228
Cys Lys Leu Ala Gly Arg Lys Ile Leu Gly Ile Arg Ser Pro Ser Arg
            375                 380                 385

agg cgt gcc gaa gag gct gtt cca cag ctc tgt gaa gcc ctt aaa agt     2276
Arg Arg Ala Glu Glu Ala Val Pro Gln Leu Cys Glu Ala Leu Lys Ser
        390                 395                 400

tat gct gat atg gca caa cat aca atg gag aac ctc ata gag cag cac     2324
Tyr Ala Asp Met Ala Gln His Thr Met Glu Asn Leu Ile Glu Gln His
    405                 410                 415

caa aga gaa caa aga cgc ata gca cac tct gtg gaa aaa cca gga aga     2372
Gln Arg Glu Gln Arg Arg Ile Ala His Ser Val Glu Lys Pro Gly Arg
420                 425                 430                 435

gat ttg caa cag ctt ttt acc ctc tca cca gaa caa caa aga gaa gtc     2420
Asp Leu Gln Gln Leu Phe Thr Leu Ser Pro Glu Gln Gln Arg Glu Val
                440                 445                 450

ctg tct cat tct cct aca ctg caa aaa caa ctc cat act ttt gcg cgc     2468
Leu Ser His Ser Pro Thr Leu Gln Lys Gln Leu His Thr Phe Ala Arg
            455                 460                 465

caa cta cag aat cgt ctc tcc tca gaa gag cat aga gct ata caa gaa     2516
Gln Leu Gln Asn Arg Leu Ser Ser Glu Glu His Arg Ala Ile Gln Glu
        470                 475                 480

aac aat cct cta aaa ctt tct tgc atg ctt ggt gta tcg gaa agc aaa     2564
Asn Asn Pro Leu Lys Leu Ser Cys Met Leu Gly Val Ser Glu Ser Lys
    485                 490                 495

gca aaa gaa att act aaa gct gta aaa caa acc aag gaa gca cac tgt     2612
Ala Lys Glu Ile Thr Lys Ala Val Lys Gln Thr Lys Glu Ala His Cys
500                 505                 510                 515

cag ctg cga act ctc aaa atc agt tgt tcc tca gca agg gct ctt acc     2660
Gln Leu Arg Thr Leu Lys Ile Ser Cys Ser Ser Ala Arg Ala Leu Thr
                520                 525                 530

aac taaacacgtg agatatctat aattctctct ctgagca                    2700
Asn

<210>  9
<211>  532
<212>  PRT
<213>  Bartonella henselae

<400>  9

Met Leu Glu His Asn Tyr Leu Tyr Lys Asn Ser Asn Thr Leu Lys Asn
1               5                   10                  15

Lys Tyr Gly Ile Lys Asp Pro Gln Arg Leu Tyr Glu Arg Cys Ala His
            20                  25                  30

Asp Thr Ala Arg Glu Val Met Asn Leu Arg Phe Glu Pro Pro Pro Gln
        35                  40                  45

Arg Phe Gly Leu Ala Tyr Leu Lys Leu Ile His Trp Thr Leu Phe His
    50                  55                  60

Arg Ser Phe Glu Trp Ala Gly His Thr Arg Asp Glu Pro Phe Thr Phe
65                  70                  75                  80

Glu Asp Gly Ser Thr Ala Arg Met Pro Ala Met Arg Pro Lys Gly His
                85                  90                  95

Arg Val Pro Phe Ala Val Gly Pro Gln Ile Gln Lys Glu Leu Lys Gln
            100                 105                 110

Leu Glu Arg Lys Leu Thr Ala Lys Asn Asn Leu Gln Gly Leu Ser Arg
        115                 120                 125

Gln Glu Phe Ala Glu Ser Ala Ala Glu Val Phe Met Thr Leu Asp His
    130                 135                 140

Ala His Pro Phe Arg Lys Gly Asn Gly Arg Ala Gln Arg Ile Phe Met
145                 150                 155                 160

Glu Lys Leu Gly Gln Ala Ala Gly Tyr Lys Ile Asp Phe Ser Phe Ile
                165                 170                 175

Thr Lys Glu Arg Leu Thr Gln Ala Ser Ile Ala Ala Met Gln His Gly
            180                 185                 190

Asn Thr Gln Pro Met Lys Asp Leu Phe Glu Asp Ile Thr His Pro Gln
        195                 200                 205

Lys Phe Leu Leu Leu Lys Glu Phe Ile Ser Gln Met Arg Asn Ser Gly
    210                 215                 220

Leu Glu Glu Ile Asn Glu His Ile Val Val Val Ala Lys Glu Gly Val
225                 230                 235                 240

Lys Tyr Asp Gly Ile Tyr Lys Gly Cys Ala Ala Glu Gly Phe Val Ile
                245                 250                 255

Glu Leu Glu Asp Gly Phe Val Val Gly His Lys Asp Asp Leu Thr Pro
            260                 265                 270

Glu Gln Val Lys Thr Leu Gln Asn Gly Asp Arg Ile Val Phe Glu Lys
        275                 280                 285

Ser Asn Val Leu Asp Leu Lys Glu Ile Leu Ile Pro Lys Glu Thr Leu
    290                 295                 300

Pro Pro Leu Thr Asn Glu Glu Leu Ala Lys Arg Phe Thr Asp Asn Cys
305                 310                 315                 320

Gly Val Glu Ser Tyr Arg Gln Glu Val Glu His Leu Ser Lys Arg Val
                325                 330                 335

Tyr Gly Lys Ser Gln Ala Leu Asn Glu Met Ile Glu Ser Ile Asn Ile
            340                 345                 350

Asp Pro Ser Leu Gly Ala Glu Phe Ala Asp Gln Ile Ala Gln Asn Pro
        355                 360                 365

Lys Ser Phe Cys Lys Leu Ala Gly Arg Lys Ile Leu Gly Ile Arg Ser
    370                 375                 380

Pro Ser Arg Arg Arg Ala Glu Glu Ala Val Pro Gln Leu Cys Glu Ala
385                 390                 395                 400

Leu Lys Ser Tyr Ala Asp Met Ala Gln His Thr Met Glu Asn Leu Ile
                405                 410                 415

Glu Gln His Gln Arg Glu Gln Arg Arg Ile Ala His Ser Val Glu Lys
            420                 425                 430

Pro Gly Arg Asp Leu Gln Gln Leu Phe Thr Leu Ser Pro Glu Gln Gln
        435                 440                 445

Arg Glu Val Leu Ser His Ser Pro Thr Leu Gln Lys Gln Leu His Thr
    450                 455                 460

Phe Ala Arg Gln Leu Gln Asn Arg Leu Ser Ser Glu Glu His Arg Ala
465                 470                 475                 480

Ile Gln Glu Asn Asn Pro Leu Lys Leu Ser Cys Met Leu Gly Val Ser
                485                 490                 495

Glu Ser Lys Ala Lys Glu Ile Thr Lys Ala Val Lys Gln Thr Lys Glu
            500                 505                 510

Ala His Cys Gln Leu Arg Thr Leu Lys Ile Ser Cys Ser Ser Ala Arg
        515                 520                 525

Ala Leu Thr Asn
    530

<210>  10
<211>  2100
<212>  DNA
<213>  Bartonella henselae

<220>
<221>  CDS
<222>  (440)..(2041)
<223>  bepD

<400>  10
catcggcaag caaaatacaa gagattatga aaaccgtaaa ataaactcaa aaagtacaat      60

aatacaaact gcgcacactc caagtcggcc aatgcgcagt aaaggctttt agcagcaata     120

caggaaagag agctgtacct tttcctctcc tcttttggta caagtcattt tataaatgat     180

tgcgtgaaaa aatgttacaa aatatgacag aaaatatcca ccaaaataga ctcggtagtt     240

ttatataact tattgataaa atttcacaaa aaaactgtat aaaagacata aatgtcgatt     300

atgataattg tgaatagcgg aggatctttg gaggtcattc aagtaaccat cttcctttgt     360

ataaaagcta ttaaagcata aaggtggagg atgcaccaaa gagtttttct aaaaacaaaa     420

agcttgaaag gaagcatgc atg aaa aaa aat cga cca tcc cct ccg aca tct      472
                     Met Lys Lys Asn Arg Pro Ser Pro Pro Thr Ser
                     1               5                   10

cgt tct aaa gaa caa gaa caa gaa cag gca agg aca cct tca ccg caa      520
Arg Ser Lys Glu Gln Glu Gln Glu Gln Ala Arg Thr Pro Ser Pro Gln
            15                  20                  25

gca gaa ccc ctc tat gca cag gta aat aaa cca ccc aga gag cac cgt      568
Ala Glu Pro Leu Tyr Ala Gln Val Asn Lys Pro Pro Arg Glu His Arg
        30                  35                  40

gcc caa tca tca gaa gaa act atc tat gca cct caa aac cca cca gaa      616
Ala Gln Ser Ser Glu Glu Thr Ile Tyr Ala Pro Gln Asn Pro Pro Glu
    45                  50                  55

act atc tat gca ccc caa aaa cct cta gga aat ccc tat gac aga ctt      664

Thr Ile Tyr Ala Pro Gln Lys Pro Leu Gly Asn Pro Tyr Asp Arg Leu
60                  65                  70                  75

ggt ggg agg ccg agg aat ggg cga cgt gcc gag aaa cta gta gac ccc      712
Gly Gly Arg Pro Arg Asn Gly Arg Arg Ala Glu Lys Leu Val Asp Pro
                80                  85                  90

tat gca gta act gat gta cag aat cta gat agg ggg gca gac ttt caa      760
Tyr Ala Val Thr Asp Val Gln Asn Leu Asp Arg Gly Ala Asp Phe Gln
            95                  100                 105

acc tta gaa aat ccc ctc tat gag gga gtt ggc ggg ggt gcc cat ggt      808
Thr Leu Glu Asn Pro Leu Tyr Glu Gly Val Gly Gly Gly Ala His Gly
        110                 115                 120

ggg cca cat cct caa gaa cca gaa cat ctc tat gca gag ctt gaa ttt      856
Gly Pro His Pro Gln Glu Pro Glu His Leu Tyr Ala Glu Leu Glu Phe
    125                 130                 135

gac aca caa agt ggg cgc tcc ccc caa aaa cct gta gaa tct gtc tat      904
Asp Thr Gln Ser Gly Arg Ser Pro Gln Lys Pro Val Glu Ser Val Tyr
140                 145                 150                 155

gca aca gtt ggc atg gga gca gag ggt ggg caa gac act caa acc tta      952
Ala Thr Val Gly Met Gly Ala Glu Gly Gly Gln Asp Thr Gln Thr Leu
                160                 165                 170

aaa aat ccc ctc tac gaa gga gtt ggc tca gga aag aca aca ccc cct      1000
Lys Asn Pro Leu Tyr Glu Gly Val Gly Ser Gly Lys Thr Thr Pro Pro
            175                 180                 185

ccg aca ccc cat tct acg gaa aaa gaa caa gaa cag gca agg gca cct      1048
Pro Thr Pro His Ser Thr Glu Lys Glu Gln Glu Gln Ala Arg Ala Pro
        190                 195                 200

tca ccg caa gca gaa ccc ctc tat gca cag gta aat aaa cca ccc aga      1096
Ser Pro Gln Ala Glu Pro Leu Tyr Ala Gln Val Asn Lys Pro Pro Arg
    205                 210                 215

gag cac cgt gcc caa tca tca gaa gaa act atc tat gca cct caa aac      1144
Glu His Arg Ala Gln Ser Ser Glu Glu Thr Ile Tyr Ala Pro Gln Asn
220                 225                 230                 235

cca cca gaa act atc tat gca ccc caa aaa cct cta gga aat ccc tat      1192
Pro Pro Glu Thr Ile Tyr Ala Pro Gln Lys Pro Leu Gly Asn Pro Tyr
                240                 245                 250

gac aga ctt ggt ggg agg ccg agg aat ggg cga cgt gcc aag aaa cta      1240
Asp Arg Leu Gly Gly Arg Pro Arg Asn Gly Arg Arg Ala Lys Lys Leu
            255                 260                 265

gta gac ccc tat gca gta act gat gta cag aat cta gat agg ggg gca      1288
Val Asp Pro Tyr Ala Val Thr Asp Val Gln Asn Leu Asp Arg Gly Ala
        270                 275                 280

gac ttt caa acc tta gaa aat ccc ctc tat gag gga gtt ggc ggg ggt      1336
Asp Phe Gln Thr Leu Glu Asn Pro Leu Tyr Glu Gly Val Gly Gly Gly
    285                 290                 295

gcc cat ggt ggg cca cat cct caa gaa cca gaa cat ctc tat gca gag      1384
Ala His Gly Gly Pro His Pro Gln Glu Pro Glu His Leu Tyr Ala Glu
300                 305                 310                 315

ctt gaa ttt gac aca caa ggt ggg cgc tcc ccc caa aaa ccc gta gaa      1432

Leu Glu Phe Asp Thr Gln Gly Gly Arg Ser Pro Gln Lys Pro Val Glu
                320                 325                 330

tct gtc tat gca aca gtt ggc atg gga aca gag ggt ggg caa gac act      1480
Ser Val Tyr Ala Thr Val Gly Met Gly Thr Glu Gly Gly Gln Asp Thr
            335                 340                 345

caa acc tta aaa aat ccc ctc tac gaa gga gtt ggc cca gga agg gca      1528
Gln Thr Leu Lys Asn Pro Leu Tyr Glu Gly Val Gly Pro Gly Arg Ala
        350                 355                 360

aca tct cct cca aga tcc ccg aaa gat gaa gtt act tca aag ctt tta      1576
Thr Ser Pro Pro Arg Ser Pro Lys Asp Glu Val Thr Ser Lys Leu Leu
    365                 370                 375

aaa cac aca gac ttt caa cat ggt gta aaa gaa gtc caa gag tgg tgt      1624
Lys His Thr Asp Phe Gln His Gly Val Lys Glu Val Gln Glu Trp Cys
380                 385                 390                 395

aaa att gtt tat ggt aac cag tat gcc ttg aat aaa aaa ctc tgt aag      1672
Lys Ile Val Tyr Gly Asn Gln Tyr Ala Leu Asn Lys Lys Leu Cys Lys
                400                 405                 410

att ctt gag aat cct aaa gca gga gac aca att cta tgg gac ctt gca      1720
Ile Leu Glu Asn Pro Lys Ala Gly Asp Thr Ile Leu Trp Asp Leu Ala
            415                 420                 425

gca gat cct gaa agc gct ggt aaa ctt gcg ggt caa aaa ata ctt ggc      1768
Ala Asp Pro Glu Ser Ala Gly Lys Leu Ala Gly Gln Lys Ile Leu Gly
        430                 435                 440

ata aaa agt cca gac cgc aaa caa gct gaa gaa ggt ttt ggc tcc ctt      1816
Ile Lys Ser Pro Asp Arg Lys Gln Ala Glu Glu Gly Phe Gly Ser Leu
    445                 450                 455

tgt gct gct ttt gaa aga cat gta gct att aca caa aag cta cac aaa      1864
Cys Ala Ala Phe Glu Arg His Val Ala Ile Thr Gln Lys Leu His Lys
460                 465                 470                 475

gat ttt acc ctt gag cag gaa aaa caa cgt gga cat gag aga gag aca      1912
Asp Phe Thr Leu Glu Gln Glu Lys Gln Arg Gly His Glu Arg Glu Thr
                480                 485                 490

agc cca gaa agg cag gaa cac aga cat cat cgt cgt cat cac tca caa      1960
Ser Pro Glu Arg Gln Glu His Arg His His Arg Arg His His Ser Gln
            495                 500                 505

gag aga gaa cag ggt gct tca gag cag gaa gca cga cgc aga aat aat      2008
Glu Arg Glu Gln Gly Ala Ser Glu Gln Glu Ala Arg Arg Arg Asn Asn
        510                 515                 520

ccc aca aat caa aaa gga atg gcc ttt ggt atg taagcgtgca taaaaatac      2061
Pro Thr Asn Gln Lys Gly Met Ala Phe Gly Met
    525                 530

agatattttt gcctttcgtt gatttcttgg tgcaattaa                            2100

<210>  11
<211>  534
<212>  PRT
<213>  Bartonella henselae

<400>  11

Met Lys Lys Asn Arg Pro Ser Pro Pro Thr Ser Arg Ser Lys Glu Gln
1               5                   10                  15

Glu Gln Glu Gln Ala Arg Thr Pro Ser Pro Gln Ala Glu Pro Leu Tyr
            20                  25                  30

Ala Gln Val Asn Lys Pro Pro Arg Glu His Arg Ala Gln Ser Ser Glu
        35                  40                  45

Glu Thr Ile Tyr Ala Pro Gln Asn Pro Pro Glu Thr Ile Tyr Ala Pro
    50                  55                  60

Gln Lys Pro Leu Gly Asn Pro Tyr Asp Arg Leu Gly Gly Arg Pro Arg
65                  70                  75                  80

Asn Gly Arg Arg Ala Glu Lys Leu Val Asp Pro Tyr Ala Val Thr Asp
                85                  90                  95

Val Gln Asn Leu Asp Arg Gly Ala Asp Phe Gln Thr Leu Glu Asn Pro
            100                 105                 110

Leu Tyr Glu Gly Val Gly Gly Gly Ala His Gly Gly Pro His Pro Gln
        115                 120                 125

Glu Pro Glu His Leu Tyr Ala Glu Leu Glu Phe Asp Thr Gln Ser Gly
    130                 135                 140

Arg Ser Pro Gln Lys Pro Val Glu Ser Val Tyr Ala Thr Val Gly Met
145                 150                 155                 160

Gly Ala Glu Gly Gly Gln Asp Thr Gln Thr Leu Lys Asn Pro Leu Tyr
                165                 170                 175

Glu Gly Val Gly Ser Gly Lys Thr Thr Pro Pro Pro Thr Pro His Ser
            180                 185                 190

Thr Glu Lys Glu Gln Glu Gln Ala Arg Ala Pro Ser Pro Gln Ala Glu
        195                 200                 205

Pro Leu Tyr Ala Gln Val Asn Lys Pro Pro Arg Glu His Arg Ala Gln
    210                 215                 220

Ser Ser Glu Glu Thr Ile Tyr Ala Pro Gln Asn Pro Pro Glu Thr Ile
225                 230                 235                 240

Tyr Ala Pro Gln Lys Pro Leu Gly Asn Pro Tyr Asp Arg Leu Gly Gly
                245                 250                 255

Arg Pro Arg Asn Gly Arg Arg Ala Lys Lys Leu Val Asp Pro Tyr Ala
            260                 265                 270

Val Thr Asp Val Gln Asn Leu Asp Arg Gly Ala Asp Phe Gln Thr Leu
        275                 280                 285

Glu Asn Pro Leu Tyr Glu Gly Val Gly Gly Gly Ala His Gly Gly Pro
    290                 295                 300

His Pro Gln Glu Pro Glu His Leu Tyr Ala Glu Leu Glu Phe Asp Thr
305                 310                 315                 320

Gln Gly Gly Arg Ser Pro Gln Lys Pro Val Glu Ser Val Tyr Ala Thr
                325                 330                 335

Val Gly Met Gly Thr Glu Gly Gly Gln Asp Thr Gln Thr Leu Lys Asn
            340                 345                 350

Pro Leu Tyr Glu Gly Val Gly Pro Gly Arg Ala Thr Ser Pro Pro Arg
        355                 360                 365

Ser Pro Lys Asp Glu Val Thr Ser Lys Leu Leu Lys His Thr Asp Phe
    370                 375                 380

Gln His Gly Val Lys Glu Val Gln Glu Trp Cys Lys Ile Val Tyr Gly
385                 390                 395                 400

Asn Gln Tyr Ala Leu Asn Lys Lys Leu Cys Lys Ile Leu Glu Asn Pro
                405                 410                 415

Lys Ala Gly Asp Thr Ile Leu Trp Asp Leu Ala Ala Asp Pro Glu Ser
            420                 425                 430

Ala Gly Lys Leu Ala Gly Gln Lys Ile Leu Gly Ile Lys Ser Pro Asp
        435                 440                 445

Arg Lys Gln Ala Glu Glu Gly Phe Gly Ser Leu Cys Ala Ala Phe Glu
    450                 455                 460

Arg His Val Ala Ile Thr Gln Lys Leu His Lys Asp Phe Thr Leu Glu
465                 470                 475                 480

Gln Glu Lys Gln Arg Gly His Glu Arg Glu Thr Ser Pro Glu Arg Gln
                485                 490                 495

Glu His Arg His His Arg Arg His His Ser Gln Glu Arg Glu Gln Gly
            500                 505                 510

Ala Ser Glu Gln Glu Ala Arg Arg Arg Asn Asn Pro Thr Asn Gln Lys
        515                 520                 525

Gly Met Ala Phe Gly Met
    530

<210>  12
<211>  1680
<212>  DNA
<213>  Bartonella henselae

<220>
<221>  CDS
<222>  (270)..(1661)
<223>  bepE

<400>  12
aataagatga tgtgcgccaa gaagtgttat gaaatgttac aaaaaaatca ctaaaaaagc      60

ttcggagatt ttatataact tattgataaa atttcacaaa aaaactgtat aaaagacata     120

aatgtcgatt atctggatag caaatagcgg aggatctttg gaagtcattc aagtagctat     180

cctcctttgt ataaaagctc ttaaagctta aaggtggaga gtgcaccaaa gaggttttct     240

aaaaacaaaa ggcttgaaag gaagcatgc atg aaa aga aat caa cca ccc cct      293
                                Met Lys Arg Asn Gln Pro Pro Pro
                                1               5

ccg aca act cat tcc aca gaa aaa caa aga gaa ccc tat gga caa tct      341
Pro Thr Thr His Ser Thr Glu Lys Gln Arg Glu Pro Tyr Gly Gln Ser
    10                  15                  20

gcg ata aga gca cct tca ccg caa cca gaa cct tta tac gca acg gtg      389
Ala Ile Arg Ala Pro Ser Pro Gln Pro Glu Pro Leu Tyr Ala Thr Val
25                  30                  35                  40

aac aaa aga cca ccg cgc gca aaa gac aga gaa cta aac aaa cca cta      437
Asn Lys Arg Pro Pro Arg Ala Lys Asp Arg Glu Leu Asn Lys Pro Leu
                45                  50                  55

agc caa cag gaa gaa gtc gta tac gca gcg ctt gat ttt gaa aat aga      485
Ser Gln Gln Glu Glu Val Val Tyr Ala Ala Leu Asp Phe Glu Asn Arg
            60                  65                  70

tca cat cat cca aga gag aga tac ccg gaa agg aat cta gaa agc gaa      533
Ser His His Pro Arg Glu Arg Tyr Pro Glu Arg Asn Leu Glu Ser Glu
        75                  80                  85

act ata tat acg aca gtt tct tct caa agc aca aca caa gca gaa ata      581
Thr Ile Tyr Thr Thr Val Ser Ser Gln Ser Thr Thr Gln Ala Glu Ile
    90                  95                  100

ctc tac gca gat gtc act cac aca ccc ttg cac agc aaa cac acg agg      629
Leu Tyr Ala Asp Val Thr His Thr Pro Leu His Ser Lys His Thr Arg
105                 110                 115                 120

aga gat ccg gca agc gaa act tta tac gca gaa gtt gct atg caa agc      677
Arg Asp Pro Ala Ser Glu Thr Leu Tyr Ala Glu Val Ala Met Gln Ser
                125                 130                 135

aca ata cca tct cta aca aga gag gaa att gca gat agg atg caa cac 725
Thr Ile Pro Ser Leu Thr Arg Glu Glu Ile Ala Asp Arg Met Gln His
            140                 145                 150

aat cca ttg gtc caa gcg tat caa caa gaa gtg atg cat tgg tgc aaa 773
Asn Pro Leu Val Gln Ala Tyr Gln Gln Glu Val Met His Trp Cys Lys
        155                 160                 165

att gtt tat gga aat tca gat gtt ttg aag gag aaa atg cag gag gta 821
Ile Val Tyr Gly Asn Ser Asp Val Leu Lys Glu Lys Met Gln Glu Val
    170                 175                 180

ctc cag aaa ccg tct gag gga gaa gat ctc tca cga caa gtt gca gaa 869
Leu Gln Lys Pro Ser Glu Gly Glu Asp Leu Ser Arg Gln Val Ala Glu
185                 190                 195                 200

aat ccg acg tct gtt cat aaa ctc gca ggt cgc aac ctg tgt ggc ttg 917
Asn Pro Thr Ser Val His Lys Leu Ala Gly Arg Asn Leu Cys Gly Leu
                205                 210                 215

aaa aca aat gcg cgc aga caa gct gaa gaa ggt ttt atg cac ctg tgc 965
Lys Thr Asn Ala Arg Arg Gln Ala Glu Glu Gly Phe Met His Leu Cys
            220                 225                 230

caa gct ctt gat ggt tat aca agt gct gta aca caa gca caa gaa aat 1013
Gln Ala Leu Asp Gly Tyr Thr Ser Ala Val Thr Gln Ala Gln Glu Asn
        235                 240                 245

att aaa cat gtt cca cag gca gaa gca aga cgc tat gga caa gag agt 1061
Ile Lys His Val Pro Gln Ala Glu Ala Arg Arg Tyr Gly Gln Glu Ser
    250                 255                 260

gaa caa aga gcc gag agg cta caa caa tcg cgt cat cct gaa agg gag 1109
Glu Gln Arg Ala Glu Arg Leu Gln Gln Ser Arg His Pro Glu Arg Glu
265                 270                 275                 280

aga caa tct ctc tca aat caa gaa ctt ctt ggt atg gtg caa gag aat 1157
Arg Gln Ser Leu Ser Asn Gln Glu Leu Leu Gly Met Val Gln Glu Asn
                285                 290                 295

gca tca ata caa aga tat caa gca caa gtt gag cat tgg tgt aaa att 1205
Ala Ser Ile Gln Arg Tyr Gln Ala Gln Val Glu His Trp Cys Lys Ile
            300                 305                 310

gtt tat ggc aat tca agt gtt ttg aag gaa aaa atg gaa ggc att ctc 1253
Val Tyr Gly Asn Ser Ser Val Leu Lys Glu Lys Met Glu Gly Ile Leu
        315                 320                 325

caa gac cta tct gag gga gaa gag ctt tca tgg caa gtt gca gca tat 1301
Gln Asp Leu Ser Glu Gly Glu Glu Leu Ser Trp Gln Val Ala Ala Tyr
    330                 335                 340

cct caa tct gtt cat aag ctt gcg ggg cat aat gta tgt ggc cta aaa 1349
Pro Gln Ser Val His Lys Leu Ala Gly His Asn Val Cys Gly Leu Lys
345                 350                 355                 360

aca agt gca cgc aga cat gct gaa tca ggt ctt tca cat tta tgt gat 1397
Thr Ser Ala Arg Arg His Ala Glu Ser Gly Leu Ser His Leu Cys Asp
                365                 370                 375

gcc att gat aac tat acc gat gct gtt cga caa ata aaa gaa agt att 1445
Ala Ile Asp Asn Tyr Thr Asp Ala Val Arg Gln Ile Lys Glu Ser Ile
            380                 385                 390

acg cga gga cat cag gct cag acg aga caa aac cgc caa gaa caa tct 1493
Thr Arg Gly His Gln Ala Gln Thr Arg Gln Asn Arg Gln Glu Gln Ser
        395                 400                 405

gtt gga gtg aag caa gga ttg cac aaa gaa aaa gcc tta tca agt cct 1541
Val Gly Val Lys Gln Gly Leu His Lys Glu Lys Ala Leu Ser Ser Pro
    410                 415                 420

aaa cag cat ggg cat caa acc cat cat caa ggt gcg gaa gcc tct aag 1589
Lys Gln His Gly His Gln Thr His His Gln Gly Ala Glu Ala Ser Lys
425                 430                 435                 440

cga aca cac caa caa tca ccg gaa gtt cca cca cgc aaa gtt gga gga 1637
Arg Thr His Gln Gln Ser Pro Glu Val Pro Pro Arg Lys Val Gly Gly
                445                 450                 455

gca aag gca ata gct ttc gcc atc taaacggcat caacgctca 1680
Ala Lys Ala Ile Ala Phe Ala Ile
            460

<210> 13
<211> 464
<212> PRT
<213> Bartonella henselae

<400> 13

Met Lys Arg Asn Gln Pro Pro Pro Pro Thr Thr His Ser Thr Glu Lys
1               5                   10                  15

Gln Arg Glu Pro Tyr Gly Gln Ser Ala Ile Arg Ala Pro Ser Pro Gln
            20                  25                  30

Pro Glu Pro Leu Tyr Ala Thr Val Asn Lys Arg Pro Pro Arg Ala Lys
        35                  40                  45

Asp Arg Glu Leu Asn Lys Pro Leu Ser Gln Gln Glu Glu Val Val Tyr
    50                  55                  60

Ala Ala Leu Asp Phe Glu Asn Arg Ser His His Pro Arg Glu Arg Tyr
65                  70                  75                  80

Pro Glu Arg Asn Leu Glu Ser Glu Thr Ile Tyr Thr Thr Val Ser Ser
                85                  90                  95

Gln Ser Thr Thr Gln Ala Glu Ile Leu Tyr Ala Asp Val Thr His Thr
            100                 105                 110

Pro Leu His Ser Lys His Thr Arg Arg Asp Pro Ala Ser Glu Thr Leu
        115                 120                 125

Tyr Ala Glu Val Ala Met Gln Ser Thr Ile Pro Ser Leu Thr Arg Glu
    130                 135                 140

Glu Ile Ala Asp Arg Met Gln His Asn Pro Leu Val Gln Ala Tyr Gln
145                 150                 155                 160

Gln Glu Val Met His Trp Cys Lys Ile Val Tyr Gly Asn Ser Asp Val
                165                 170                 175

Leu Lys Glu Lys Met Gln Glu Val Leu Gln Lys Pro Ser Glu Gly Glu
            180                 185                 190

Asp Leu Ser Arg Gln Val Ala Glu Asn Pro Thr Ser Val His Lys Leu
        195                 200                 205

Ala Gly Arg Asn Leu Cys Gly Leu Lys Thr Asn Ala Arg Arg Gln Ala
    210                 215                 220

Glu Glu Gly Phe Met His Leu Cys Gln Ala Leu Asp Gly Tyr Thr Ser
225                 230                 235                 240

Ala Val Thr Gln Ala Gln Glu Asn Ile Lys His Val Pro Gln Ala Glu
                245                 250                 255

Ala Arg Arg Tyr Gly Gln Glu Ser Glu Gln Arg Ala Glu Arg Leu Gln
            260                 265                 270

Gln Ser Arg His Pro Glu Arg Glu Arg Gln Ser Leu Ser Asn Gln Glu
        275                 280                 285

Leu Leu Gly Met Val Gln Glu Asn Ala Ser Ile Gln Arg Tyr Gln Ala
    290                 295                 300

Gln Val Glu His Trp Cys Lys Ile Val Tyr Gly Asn Ser Ser Val Leu
305                 310                 315                 320

Lys Glu Lys Met Glu Gly Ile Leu Gln Asp Leu Ser Glu Gly Glu Glu
                325                 330                 335

Leu Ser Trp Gln Val Ala Ala Tyr Pro Gln Ser Val His Lys Leu Ala
            340                 345                 350

Gly His Asn Val Cys Gly Leu Lys Thr Ser Ala Arg Arg His Ala Glu
        355                 360                 365

Ser Gly Leu Ser His Leu Cys Asp Ala Ile Asp Asn Tyr Thr Asp Ala
    370                 375                 380

Val Arg Gln Ile Lys Glu Ser Ile Thr Arg Gly His Gln Ala Gln Thr
385                 390                 395                 400

Arg Gln Asn Arg Gln Glu Gln Ser Val Gly Val Lys Gln Gly Leu His
                405                 410                 415

Lys Glu Lys Ala Leu Ser Ser Pro Lys Gln His Gly His Gln Thr His
            420                 425                 430

His Gln Gly Ala Glu Ala Ser Lys Arg Thr His Gln Gln Ser Pro Glu
        435                 440                 445

Val Pro Pro Arg Lys Val Gly Gly Ala Lys Ala Ile Ala Phe Ala Ile
    450                 455                 460

<210>  14
<211>  2880
<212>  DNA
<213>  Bartonella henselae

<220>
<221>  CDS
<222>  (320)..(2821)
<223>  bepF

<400>  14
gcgcgcatca gctttgctgt ttcatgagat tgtttgatct aatctcttgg ctctagtcgt      60

tttagaagga ttgcgtcaag agtgtgtgtt tgatgttgtg gtacatattt catcaaacac     120

cccgtcactg aacctgaata acgcatcaaa ttacaaagac aaatattgat tacactctgg     180

gcaaaaaacg caaaatcctt tgggggcatt caaatgtgta tccttctttg aagaaaaaac     240

tatcaaaata taaaggtgga gagcgcacca aagaggtttt ctaagaacga atagtacaaa     300

atcttgaaag gaaacatgc atg aaa aaa aac caa cca tcc tct tcc aca tct      352
                     Met Lys Lys Asn Gln Pro Ser Ser Ser Thr Ser
                     1               5                   10

ccc tca gtg aaa gaa tta aaa aag cgc tat gaa caa act gcc gca gaa      400
Pro Ser Val Lys Glu Leu Lys Lys Arg Tyr Glu Gln Thr Ala Ala Glu
            15                  20                  25

gcc tct act cta cac caa ctc tcc gca ggg agt gct cca aaa gaa aaa      448
Ala Ser Thr Leu His Gln Leu Ser Ala Gly Ser Ala Pro Lys Glu Lys
        30                  35                  40

aaa cag cct tcc tcg agg ttg gaa gaa tta aga aaa cgc tat gaa caa      496
Lys Gln Pro Ser Ser Arg Leu Glu Glu Leu Arg Lys Arg Tyr Glu Gln
    45                  50                  55

act gct tcc cta aca gct tct caa gaa gct ctc tct gca agg ggt ttt      544
Thr Ala Ser Leu Thr Ala Ser Gln Glu Ala Leu Ser Ala Arg Gly Phe
60                  65                  70                  75

cca aaa gaa aaa aga caa cct cac aca cag caa agt cct gaa caa ttg      592
Pro Lys Glu Lys Arg Gln Pro His Thr Gln Gln Ser Pro Glu Gln Leu
                80                  85                  90

cca ata cac cat gaa cag caa ttt gct gta acg ccg caa caa agt tca      640

Pro Ile His His Glu Gln Gln Phe Ala Val Thr Pro Gln Gln Ser Ser
            95                  100                 105

tca caa acg cct ctc tac gca aca cct ctt cca caa caa cca gtt cgc 688
Ser Gln Thr Pro Leu Tyr Ala Thr Pro Leu Pro Gln Gln Pro Val Arg
        110                 115                 120

acg cct cca caa aga cca ccg cgc gca aaa gac aaa gag cta cac aaa 736
Thr Pro Pro Gln Arg Pro Pro Arg Ala Lys Asp Lys Glu Leu His Lys
    125                 130                 135

aca gcc ccc caa gac agc aca ccc cta tac gcg aca cct tct cca caa 784
Thr Ala Pro Gln Asp Ser Thr Pro Leu Tyr Ala Thr Pro Ser Pro Gln
140                 145                 150                 155

caa tca gtt cag aca cct cca caa aga cca ccg cgc gca aaa gac aag 832
Gln Ser Val Gln Thr Pro Pro Gln Arg Pro Pro Arg Ala Lys Asp Lys
                160                 165                 170

gag cta cac aaa gca gcc tcc caa gac agc aca ccc cta tac gcg aca 880
Glu Leu His Lys Ala Ala Ser Gln Asp Ser Thr Pro Leu Tyr Ala Thr
            175                 180                 185

cct tct ccc cac aaa caa cag ccc atg aga gaa agg gcg caa agc aac 928
Pro Ser Pro His Lys Gln Gln Pro Met Arg Glu Arg Ala Gln Ser Asn
        190                 195                 200

aca tca agc caa gac agc gaa cct ctc tac gca aca cct ctt cca caa 976
Thr Ser Ser Gln Asp Ser Glu Pro Leu Tyr Ala Thr Pro Leu Pro Gln
    205                 210                 215

aga cac cag ccc atg aga gaa agg gcg caa agc aac aca tca aac caa 1024
Arg His Gln Pro Met Arg Glu Arg Ala Gln Ser Asn Thr Ser Asn Gln
220                 225                 230                 235

gac agc gaa cct ctc tac gca aca cct ctt cca caa aga cac cag ccc 1072
Asp Ser Glu Pro Leu Tyr Ala Thr Pro Leu Pro Gln Arg His Gln Pro
                240                 245                 250

gtg aga aaa agg acg caa agc aac aca tca agc caa gac agc gaa cct 1120
Val Arg Lys Arg Thr Gln Ser Asn Thr Ser Ser Gln Asp Ser Glu Pro
            255                 260                 265

ctc tac gca aca cct ctt cca caa aga cgc cag ccc gtg aga aaa agg 1168
Leu Tyr Ala Thr Pro Leu Pro Gln Arg Arg Gln Pro Val Arg Lys Arg
        270                 275                 280

acg caa agc aac aca tca agc caa gac aac gaa cct ctc tac gca aca 1216
Thr Gln Ser Asn Thr Ser Ser Gln Asp Asn Glu Pro Leu Tyr Ala Thr
    285                 290                 295

gct gct cca tca caa tca cca cgc atg gaa gag aaa tct gta aac aca 1264
Ala Ala Pro Ser Gln Ser Pro Arg Met Glu Glu Lys Ser Val Asn Thr
300                 305                 310                 315

atg caa cga aac tta ttg ctt gaa gca tat aag gaa gaa att aaa tat 1312
Met Gln Arg Asn Leu Leu Leu Glu Ala Tyr Lys Glu Glu Ile Lys Tyr
                320                 325                 330

tgg tgc gga att gtt tat ggc gat cgg ctt att ttg cag aaa aga ata 1360
Trp Cys Gly Ile Val Tyr Gly Asp Arg Leu Ile Leu Gln Lys Arg Ile
            335                 340                 345

gaa gag att caa gaa aac cct gct ctg gga gaa cag ctg tca tgg gag 1408

Glu Glu Ile Gln Glu Asn Pro Ala Leu Gly Glu Gln Leu Ser Trp Glu
        350                 355                 360

gtt tca gaa cat cct aag tct att tct aag ctt gcg ggt aaa aaa atg 1456
Val Ser Glu His Pro Lys Ser Ile Ser Lys Leu Ala Gly Lys Lys Met
    365                 370                 375

ctt ggt gtg aaa acg aag gca cgc aga aaa gct gaa gaa aat tat cta 1504
Leu Gly Val Lys Thr Lys Ala Arg Arg Lys Ala Glu Glu Asn Tyr Leu
380                 385                 390                 395

gct ttg cgt gac acc ata aat agt tat gtt tat act tta aaa tat tca 1552
Ala Leu Arg Asp Thr Ile Asn Ser Tyr Val Tyr Thr Leu Lys Tyr Ser
                400                 405                 410

caa caa aaa cct ttg cat gct ccg cat aca gaa cac acc cgc gat gaa 1600
Gln Gln Lys Pro Leu His Ala Pro His Thr Glu His Thr Arg Asp Glu
            415                 420                 425

caa caa aca cac aga acg caa agc gct gaa aag gag aga gca cct ctc 1648
Gln Gln Thr His Arg Thr Gln Ser Ala Glu Lys Glu Arg Ala Pro Leu
        430                 435                 440

tct aat cag gaa ctt gcc gac aga att cga aca gag cca tcg gtt cta 1696
Ser Asn Gln Glu Leu Ala Asp Arg Ile Arg Thr Glu Pro Ser Val Leu
    445                 450                 455

tat agc caa aca gaa gtc caa tat tgg agc aaa att gtt ttt ggc aac 1744
Tyr Ser Gln Thr Glu Val Gln Tyr Trp Ser Lys Ile Val Phe Gly Asn
460                 465                 470                 475

cct tat att ttg cag tac aga att gaa gac att caa aaa aat ccc gat 1792
Pro Tyr Ile Leu Gln Tyr Arg Ile Glu Asp Ile Gln Lys Asn Pro Asp
                480                 485                 490

atg gga gaa gaa ttt tca tgg caa gtt gca aac aat cct agt ctt ttt 1840
Met Gly Glu Glu Phe Ser Trp Gln Val Ala Asn Asn Pro Ser Leu Phe
            495                 500                 505

tct ccg ctc gca gga aag agg ata ctt ggc ata aaa aat gat gcg cgc 1888
Ser Pro Leu Ala Gly Lys Arg Ile Leu Gly Ile Lys Asn Asp Ala Arg
        510                 515                 520

aga cac gca gaa gca agt ctt tct tgc ctt tgt agc gcc att gaa ggt 1936
Arg His Ala Glu Ala Ser Leu Ser Cys Leu Cys Ser Ala Ile Glu Gly
    525                 530                 535

tac gca gat gct gta aaa caa gca aaa gag gat att gta caa gaa tat 1984
Tyr Ala Asp Ala Val Lys Gln Ala Lys Glu Asp Ile Val Gln Glu Tyr
540                 545                 550                 555

caa gta caa caa agt cgc caa gaa ctt tct act gaa ccg gct aaa cag 2032
Gln Val Gln Gln Ser Arg Gln Glu Leu Ser Thr Glu Pro Ala Lys Gln
                560                 565                 570

ttg caa aaa caa caa act tta tcc aaa cca cat aaa ctg cct gaa cac 2080
Leu Gln Lys Gln Gln Thr Leu Ser Lys Pro His Lys Leu Pro Glu His
            575                 580                 585

tca cca aca gac gcg cat caa gaa act act caa acc tcc atg caa act 2128
Ser Pro Thr Asp Ala His Gln Glu Thr Thr Gln Thr Ser Met Gln Thr
        590                 595                 600

gag tgg gag cgg ctt ggt gct cga cca cgc aca gtg aca aca cac aaa 2176

Glu Trp Glu Arg Leu Gly Ala Arg Pro Arg Thr Val Thr Thr His Lys
    605                 610                 615

aaa cac aca caa gag agc act ttg cct act tct cat gca gag caa aaa      2224
Lys His Thr Gln Glu Ser Thr Leu Pro Thr Ser His Ala Glu Gln Lys
620                 625                 630                 635

aca acg aga gag caa aca act gaa aat cag caa aaa tcc ctt aca cct      2272
Thr Thr Arg Glu Gln Thr Thr Glu Asn Gln Gln Lys Ser Leu Thr Pro
                640                 645                 650

gaa aaa gaa aaa aca cct ctc tca cat aaa gaa att gca agc aga gtt      2320
Glu Lys Glu Lys Thr Pro Leu Ser His Lys Glu Ile Ala Ser Arg Val
            655                 660                 665

caa aac gat ctc tct gtt caa cgt gct caa ata gaa atc tac aat tgg      2368
Gln Asn Asp Leu Ser Val Gln Arg Ala Gln Ile Glu Ile Tyr Asn Trp
        670                 675                 680

tgt aat att gtt tat aat aac cca ctt gtt ttg caa cat acc act gaa      2416
Cys Asn Ile Val Tyr Asn Asn Pro Leu Val Leu Gln His Thr Thr Glu
    685                 690                 695

gat atc aaa aaa att ccc atg cta gga gaa gaa ctc tca tgg caa gtt      2464
Asp Ile Lys Lys Ile Pro Met Leu Gly Glu Glu Leu Ser Trp Gln Val
700                 705                 710                 715

gca aac ttt tct aca ata ttt gct ccg ctt gct ggc aag caa gtg ctt      2512
Ala Asn Phe Ser Thr Ile Phe Ala Pro Leu Ala Gly Lys Gln Val Leu
                720                 725                 730

ggc ata aaa aat aac gca cgt aaa cat gct gaa gaa gct att ccc tcc      2560
Gly Ile Lys Asn Asn Ala Arg Lys His Ala Glu Glu Ala Ile Pro Ser
            735                 740                 745

cta tgc act gcc att gat cat tac aca gag att gta aaa caa gta aga      2608
Leu Cys Thr Ala Ile Asp His Tyr Thr Glu Ile Val Lys Gln Val Arg
        750                 755                 760

gag gac att gta aaa aac cat cag gaa caa tct cca aag aaa gat aaa      2656
Glu Asp Ile Val Lys Asn His Gln Glu Gln Ser Pro Lys Lys Asp Lys
    765                 770                 775

aac gtg aaa cga cag caa agc tta tcg aaa cct cct aaa tta cct gag      2704
Asn Val Lys Arg Gln Gln Ser Leu Ser Lys Pro Pro Lys Leu Pro Glu
780                 785                 790                 795

cgc tca aca gaa atc cca cgt cat cag gct ttg gaa gcc tct agg caa      2752
Arg Ser Thr Glu Ile Pro Arg His Gln Ala Leu Glu Ala Ser Arg Gln
                800                 805                 810

gca caa caa aaa ccg tcg aac gtt cga tca agc gaa gct gag aaa aca      2800
Ala Gln Gln Lys Pro Ser Asn Val Arg Ser Ser Glu Ala Glu Lys Thr
            815                 820                 825

caa aaa atg gtg ctg gca ctc taaagatgca tcagctttca tctgaccttt      2851
Gln Lys Met Val Leu Ala Leu
        830

cggtacttat cactttataa aaagtcccc                                      2880


<210>  15
<211>  834

<212>  PRT
<213>  Bartonella henselae

<400>  15

Met Lys Lys Asn Gln Pro Ser Ser Ser Thr Ser Pro Ser Val Lys Glu
1               5                   10                  15

Leu Lys Lys Arg Tyr Glu Gln Thr Ala Ala Glu Ala Ser Thr Leu His
            20                  25                  30

Gln Leu Ser Ala Gly Ser Ala Pro Lys Glu Lys Lys Gln Pro Ser Ser
        35                  40                  45

Arg Leu Glu Glu Leu Arg Lys Arg Tyr Glu Gln Thr Ala Ser Leu Thr
    50                  55                  60

Ala Ser Gln Glu Ala Leu Ser Ala Arg Gly Phe Pro Lys Glu Lys Arg
65                  70                  75                  80

Gln Pro His Thr Gln Gln Ser Pro Glu Gln Leu Pro Ile His His Glu
                85                  90                  95

Gln Gln Phe Ala Val Thr Pro Gln Gln Ser Ser Ser Gln Thr Pro Leu
            100                 105                 110

Tyr Ala Thr Pro Leu Pro Gln Gln Pro Val Arg Thr Pro Pro Gln Arg
        115                 120                 125

Pro Pro Arg Ala Lys Asp Lys Glu Leu His Lys Thr Ala Pro Gln Asp
    130                 135                 140

Ser Thr Pro Leu Tyr Ala Thr Pro Ser Pro Gln Gln Ser Val Gln Thr
145                 150                 155                 160

Pro Pro Gln Arg Pro Pro Arg Ala Lys Asp Lys Glu Leu His Lys Ala
                165                 170                 175

Ala Ser Gln Asp Ser Thr Pro Leu Tyr Ala Thr Pro Ser Pro His Lys
            180                 185                 190

Gln Gln Pro Met Arg Glu Arg Ala Gln Ser Asn Thr Ser Ser Gln Asp
        195                 200                 205

Ser Glu Pro Leu Tyr Ala Thr Pro Leu Pro Gln Arg His Gln Pro Met
    210                 215                 220

Arg Glu Arg Ala Gln Ser Asn Thr Ser Asn Gln Asp Ser Glu Pro Leu
225                 230                 235                 240

Tyr Ala Thr Pro Leu Pro Gln Arg His Gln Pro Val Arg Lys Arg Thr
                245                 250                 255

Gln Ser Asn Thr Ser Ser Gln Asp Ser Glu Pro Leu Tyr Ala Thr Pro
            260                 265                 270

Leu Pro Gln Arg Arg Gln Pro Val Arg Lys Arg Thr Gln Ser Asn Thr
        275                 280                 285

Ser Ser Gln Asp Asn Glu Pro Leu Tyr Ala Thr Ala Ala Pro Ser Gln
    290                 295                 300

Ser Pro Arg Met Glu Glu Lys Ser Val Asn Thr Met Gln Arg Asn Leu
305                 310                 315                 320

Leu Leu Glu Ala Tyr Lys Glu Glu Ile Lys Tyr Trp Cys Gly Ile Val
                325                 330                 335

Tyr Gly Asp Arg Leu Ile Leu Gln Lys Arg Ile Glu Glu Ile Gln Glu
            340                 345                 350

Asn Pro Ala Leu Gly Glu Gln Leu Ser Trp Glu Val Ser Glu His Pro
        355                 360                 365

Lys Ser Ile Ser Lys Leu Ala Gly Lys Lys Met Leu Gly Val Lys Thr
    370                 375                 380

Lys Ala Arg Arg Lys Ala Glu Glu Asn Tyr Leu Ala Leu Arg Asp Thr
385                 390                 395                 400

Ile Asn Ser Tyr Val Tyr Thr Leu Lys Tyr Ser Gln Gln Lys Pro Leu
                405                 410                 415

His Ala Pro His Thr Glu His Thr Arg Asp Glu Gln Gln Thr His Arg
            420                 425                 430

Thr Gln Ser Ala Glu Lys Glu Arg Ala Pro Leu Ser Asn Gln Glu Leu
        435                 440                 445

Ala Asp Arg Ile Arg Thr Glu Pro Ser Val Leu Tyr Ser Gln Thr Glu
    450                 455                 460

Val Gln Tyr Trp Ser Lys Ile Val Phe Gly Asn Pro Tyr Ile Leu Gln
465                 470                 475                 480

Tyr Arg Ile Glu Asp Ile Gln Lys Asn Pro Asp Met Gly Glu Glu Phe
                485                 490                 495

Ser Trp Gln Val Ala Asn Asn Pro Ser Leu Phe Ser Pro Leu Ala Gly
            500                 505                 510

Lys Arg Ile Leu Gly Ile Lys Asn Asp Ala Arg Arg His Ala Glu Ala
        515                 520                 525

Ser Leu Ser Cys Leu Cys Ser Ala Ile Glu Gly Tyr Ala Asp Ala Val
    530                 535                 540

Lys Gln Ala Lys Glu Asp Ile Val Gln Glu Tyr Gln Val Gln Gln Ser
545                 550                 555                 560

Arg Gln Glu Leu Ser Thr Glu Pro Ala Lys Gln Leu Gln Lys Gln Gln
                565                 570                 575

Thr Leu Ser Lys Pro His Lys Leu Pro Glu His Ser Pro Thr Asp Ala
            580                 585                 590

His Gln Glu Thr Thr Gln Thr Ser Met Gln Thr Glu Trp Glu Arg Leu
        595                 600                 605

Gly Ala Arg Pro Arg Thr Val Thr Thr His Lys Lys His Thr Gln Glu
    610                 615                 620

Ser Thr Leu Pro Thr Ser His Ala Glu Gln Lys Thr Thr Arg Glu Gln
625                 630                 635                 640

Thr Thr Glu Asn Gln Gln Lys Ser Leu Thr Pro Glu Lys Glu Lys Thr
                645                 650                 655

Pro Leu Ser His Lys Glu Ile Ala Ser Arg Val Gln Asn Asp Leu Ser
            660                 665                 670

Val Gln Arg Ala Gln Ile Glu Ile Tyr Asn Trp Cys Asn Ile Val Tyr
        675                 680                 685

Asn Asn Pro Leu Val Leu Gln His Thr Thr Glu Asp Ile Lys Lys Ile
    690                 695                 700

Pro Met Leu Gly Glu Glu Leu Ser Trp Gln Val Ala Asn Phe Ser Thr
705                 710                 715                 720

Ile Phe Ala Pro Leu Ala Gly Lys Gln Val Leu Gly Ile Lys Asn Asn
                725                 730                 735

Ala Arg Lys His Ala Glu Glu Ala Ile Pro Ser Leu Cys Thr Ala Ile
            740                 745                 750

Asp His Tyr Thr Glu Ile Val Lys Gln Val Arg Glu Asp Ile Val Lys
        755                 760                 765

Asn His Gln Glu Gln Ser Pro Lys Lys Asp Lys Asn Val Lys Arg Gln
    770                 775                 780

Gln Ser Leu Ser Lys Pro Pro Lys Leu Pro Glu Arg Ser Thr Glu Ile
785                 790                 795                 800

Pro Arg His Gln Ala Leu Glu Ala Ser Arg Gln Ala Gln Gln Lys Pro
                805                 810                 815

Ser Asn Val Arg Ser Ser Glu Ala Glu Lys Thr Gln Lys Met Val Leu
            820                 825                 830

Ala Leu

<210>  16
<211>  3475
<212>  DNA
<213>  Bartonella henselae

<220>
<221>  CDS
<222>  (231)..(3257)
<223>  bepG

<400>  16
aagcagtatt gggcagtatt acggaaaatc tataacaccc cctccccgtc atgtgccctt      60

actatataaa aattacaaat acaaatagtg attatgatcc atgaaaaaaa acttttgaaa     120

ttattcaaat ggacattctc tctgaaagga aaaactctca aaacataaaa gtggagaata     180

caccaaagag gttttctaaa agcaaattaa aatctcgaaa ggagacacac atg aaa        236
                                                       Met Lys
                                                       1

aaa aaa caa cca gcc cct tca aca cct tca tcc gca agc gtt act cag       284
Lys Lys Gln Pro Ala Pro Ser Thr Pro Ser Ser Ala Ser Val Thr Gln
        5                   10                  15

atg gcg tct cta tcg aat gag gaa att ttc aac aga gtt caa aga gat       332
Met Ala Ser Leu Ser Asn Glu Glu Ile Phe Asn Arg Val Gln Arg Asp
    20                  25                  30

cca ttg gtt caa tct tgt gaa gaa gaa atc caa tat tgg tgc aaa att       380
Pro Leu Val Gln Ser Cys Glu Glu Glu Ile Gln Tyr Trp Cys Lys Ile
35                  40                  45                  50

gtt ttt ggc aac gct tat gtt ttg cag tac aga att gaa gac atg caa       428
Val Phe Gly Asn Ala Tyr Val Leu Gln Tyr Arg Ile Glu Asp Met Gln
                55                  60                  65

aaa att cct gag ata gga gaa gaa ctt tca tgg caa att tca gaa aat       476

Lys Ile Pro Glu Ile Gly Glu Glu Leu Ser Trp Gln Ile Ser Glu Asn
            70                  75                  80

cct cac ttt ttt cat aag ctt gca ggc agt caa acc ctt ggc ata aaa      524
Pro His Phe Phe His Lys Leu Ala Gly Ser Gln Thr Leu Gly Ile Lys
        85                  90                  95

aac agt gca cgc aaa gaa gct gaa gca ggt atc tct tcc ctg tgt aac      572
Asn Ser Ala Arg Lys Glu Ala Glu Ala Gly Ile Ser Ser Leu Cys Asn
    100                 105                 110

gcc att gaa aca tat gca gat acg gta aaa cag gta aga aaa agc att      620
Ala Ile Glu Thr Tyr Ala Asp Thr Val Lys Gln Val Arg Lys Ser Ile
115                 120                 125                 130

gta caa gcc cat caa gca caa caa aac cgc caa gaa ctt tcc tct gaa      668
Val Gln Ala His Gln Ala Gln Gln Asn Arg Gln Glu Leu Ser Ser Glu
                135                 140                 145

cct tct caa gac ttg caa aaa cag caa agc tta tcg aaa cct cct aaa      716
Pro Ser Gln Asp Leu Gln Lys Gln Gln Ser Leu Ser Lys Pro Pro Lys
            150                 155                 160

cta cct gaa cac tca cca gaa gac aga cat caa gaa tct act gaa agc      764
Leu Pro Glu His Ser Pro Glu Asp Arg His Gln Glu Ser Thr Glu Ser
        165                 170                 175

tct atg caa gct aaa tgg aac cgg ctt ggt gct cga cca cgc aca gtg      812
Ser Met Gln Ala Lys Trp Asn Arg Leu Gly Ala Arg Pro Arg Thr Val
    180                 185                 190

cct ctc agt caa aaa acg caa aaa cag caa gaa caa ctt ggt ctg cat      860
Pro Leu Ser Gln Lys Thr Gln Lys Gln Gln Glu Gln Leu Gly Leu His
195                 200                 205                 210

caa cct att atg gaa ggt gat ggc aaa gag gct gca aaa gtc agc atg      908
Gln Pro Ile Met Glu Gly Asp Gly Lys Glu Ala Ala Lys Val Ser Met
                215                 220                 225

tca gca att aaa gca acg aaa gtt aca aca aag tcc gaa gca ttg aaa      956
Ser Ala Ile Lys Ala Thr Lys Val Thr Thr Lys Ser Glu Ala Leu Lys
            230                 235                 240

gca gta gaa cag cta att caa ccc cca aaa gtt gaa acc gca aaa gta     1004
Ala Val Glu Gln Leu Ile Gln Pro Pro Lys Val Glu Thr Ala Lys Val
        245                 250                 255

gtt tca gtg cct ctc gct caa gaa gcg caa aaa aca ctt att gaa aag     1052
Val Ser Val Pro Leu Ala Gln Glu Ala Gln Lys Thr Leu Ile Glu Lys
    260                 265                 270

gaa aca att cca tct ctt acc aac gaa gaa gtt gct tca aag att cga     1100
Glu Thr Ile Pro Ser Leu Thr Asn Glu Glu Val Ala Ser Lys Ile Arg
275                 280                 285                 290

gaa agt gaa gtc gtt aaa agc agc atg caa aaa att aaa act ttg tgt     1148
Glu Ser Glu Val Val Lys Ser Ser Met Gln Lys Ile Lys Thr Leu Cys
                295                 300                 305

ggt gtt gtt tat ggc aat ccc cat att ttg gaa ggt aaa atg ccg aag     1196
Gly Val Val Tyr Gly Asn Pro His Ile Leu Glu Gly Lys Met Pro Lys
            310                 315                 320

atg gga att cca gtg aca aat aga aat gtc gag gag tta gaa aag ttc     1244

Met Gly Ile Pro Val Thr Asn Arg Asn Val Glu Glu Leu Glu Lys Phe
        325                 330                 335

gcg cgc caa gtt gga agc ttt ccc tcc tct ttt ggt cgg gtt gcc ggc      1292
Ala Arg Gln Val Gly Ser Phe Pro Ser Ser Phe Gly Arg Val Ala Gly
    340                 345                 350

ttc agt ttt ttg ggg ata aaa agt gga gca cgt gcc cat gct gaa gag      1340
Phe Ser Phe Leu Gly Ile Lys Ser Gly Ala Arg Ala His Ala Glu Glu
355                 360                 365                 370

aat ttt ttg ccc tta agc cat gct att gtt agt tac aca cat aat gta      1388
Asn Phe Leu Pro Leu Ser His Ala Ile Val Ser Tyr Thr His Asn Val
                375                 380                 385

aaa caa gca gaa aaa gac att cta gaa gcc cat tct ggg aaa caa gaa      1436
Lys Gln Ala Glu Lys Asp Ile Leu Glu Ala His Ser Gly Lys Gln Glu
            390                 395                 400

cgc tat gca cag tcg gta gaa aca ccc agt gaa gaa ata caa aat cta      1484
Arg Tyr Ala Gln Ser Val Glu Thr Pro Ser Glu Glu Ile Gln Asn Leu
        405                 410                 415

ttt tct ctc act caa gaa gcg caa aaa atg ctt att gaa aaa gaa ata      1532
Phe Ser Leu Thr Gln Glu Ala Gln Lys Met Leu Ile Glu Lys Glu Ile
    420                 425                 430

att cca cct ctt agt tac gtg gac gtt gct tca aag att cga gaa agt      1580
Ile Pro Pro Leu Ser Tyr Val Asp Val Ala Ser Lys Ile Arg Glu Ser
435                 440                 445                 450

gaa gtc gtt aaa agc agc aag caa aaa att gaa act ttg tgt agt gtt      1628
Glu Val Val Lys Ser Ser Lys Gln Lys Ile Glu Thr Leu Cys Ser Val
                455                 460                 465

gtt tat ggc aat ccc cgt att ttg gaa gac aaa atg ccg aag atg gga      1676
Val Tyr Gly Asn Pro Arg Ile Leu Glu Asp Lys Met Pro Lys Met Gly
            470                 475                 480

att cca gtg aca aat aag aat gtc gag gag tta gaa aag ttc gcg cgt      1724
Ile Pro Val Thr Asn Lys Asn Val Glu Glu Leu Glu Lys Phe Ala Arg
        485                 490                 495

caa gtt gga aac ttt cca tcc tct tgt ggt aag atc gtt ggc ttc aat      1772
Gln Val Gly Asn Phe Pro Ser Ser Cys Gly Lys Ile Val Gly Phe Asn
    500                 505                 510

ttt ttg ggg ata aaa agt gaa gca cgt gcc cat gct gaa gag aat ttt      1820
Phe Leu Gly Ile Lys Ser Glu Ala Arg Ala His Ala Glu Glu Asn Phe
515                 520                 525                 530

ttg ccc tta agc cat gct att ttt agt tac gca cat agt gta aaa caa      1868
Leu Pro Leu Ser His Ala Ile Phe Ser Tyr Ala His Ser Val Lys Gln
                535                 540                 545

gca gaa aag gat att cta gaa gcc tat ttt aag gaa caa gaa cgc tgt      1916
Ala Glu Lys Asp Ile Leu Glu Ala Tyr Phe Lys Glu Gln Glu Arg Cys
            550                 555                 560

gca cag tcg gta gaa aca ccc agt gaa gag ata aca aat cta ttt tct      1964
Ala Gln Ser Val Glu Thr Pro Ser Glu Glu Ile Thr Asn Leu Phe Ser
        565                 570                 575

ttc act caa gaa caa cag aaa gaa att tta tca aac tct cct aaa cta      2012

Phe Thr Gln Glu Gln Gln Lys Glu Ile Leu Ser Asn Ser Pro Lys Leu
    580                 585                 590

aga aca cag gta aag gct tat tct caa aaa ctc cat aac cgt ctc tca      2060
Arg Thr Gln Val Lys Ala Tyr Ser Gln Lys Leu His Asn Arg Leu Ser
595                 600                 605                 610

cca agt gat ctt cag gct ata agt gaa aga tct cat aca aaa ctt gct      2108
Pro Ser Asp Leu Gln Ala Ile Ser Glu Arg Ser His Thr Lys Leu Ala
                615                 620                 625

gaa agt ctt ggc aca tca gta aat caa gca gaa gaa att gca caa atc      2156
Glu Ser Leu Gly Thr Ser Val Asn Gln Ala Glu Glu Ile Ala Gln Ile
            630                 635                 640

ctc aca caa aca aaa gac gtt gtc cag att ctt caa cag cag gaa aaa      2204
Leu Thr Gln Thr Lys Asp Val Val Gln Ile Leu Gln Gln Gln Glu Lys
        645                 650                 655

ctt ggt ctg tat caa tct atc atg aaa ggt gat ggc aaa gag act gca      2252
Leu Gly Leu Tyr Gln Ser Ile Met Lys Gly Asp Gly Lys Glu Thr Ala
    660                 665                 670

aaa gtc agc atg tca gca att aag gca acg gaa gtt aca aca aag ttc      2300
Lys Val Ser Met Ser Ala Ile Lys Ala Thr Glu Val Thr Thr Lys Phe
675                 680                 685                 690

gaa gca tcc aaa gca gta gaa cag gca gtt cga ccg cca aaa gtt gaa      2348
Glu Ala Ser Lys Ala Val Glu Gln Ala Val Arg Pro Pro Lys Val Glu
                695                 700                 705

acc gca aaa gta gtt tca gta tct ttc act caa gaa acg caa aaa atg      2396
Thr Ala Lys Val Val Ser Val Ser Phe Thr Gln Glu Thr Gln Lys Met
            710                 715                 720

ctt att gaa aaa gaa ata att cca cct ctt agt tac gtg gac gtt gct      2444
Leu Ile Glu Lys Glu Ile Ile Pro Pro Leu Ser Tyr Val Asp Val Ala
        725                 730                 735

tca aag att cga gaa agt gaa gtc gtt aaa agc agc atg caa aaa att      2492
Ser Lys Ile Arg Glu Ser Glu Val Val Lys Ser Ser Met Gln Lys Ile
    740                 745                 750

aaa act ttg tgt agt gtt gtt tat ggc aat ccc gat att ttg gaa ggc      2540
Lys Thr Leu Cys Ser Val Val Tyr Gly Asn Pro Asp Ile Leu Glu Gly
755                 760                 765                 770

aaa atg ccg aag atg gga att cca gtg aca aat aag aat gtc gag gag      2588
Lys Met Pro Lys Met Gly Ile Pro Val Thr Asn Lys Asn Val Glu Glu
                775                 780                 785

tta gaa aag ttc gcg cgc cag gtt gga aac ttt cca tcc tct tgt ggt      2636
Leu Glu Lys Phe Ala Arg Gln Val Gly Asn Phe Pro Ser Ser Cys Gly
            790                 795                 800

aag atc gtt ggc ttc agt ttt ttg ggg ata aaa agt gaa gca cgt gcc      2684
Lys Ile Val Gly Phe Ser Phe Leu Gly Ile Lys Ser Glu Ala Arg Ala
        805                 810                 815

cat gct gaa gag aat ttt ttg ccc tta agc cat gct att ttt agt tac      2732
His Ala Glu Glu Asn Phe Leu Pro Leu Ser His Ala Ile Phe Ser Tyr
    820                 825                 830

gca cat agt gta aaa caa gca gaa aag gat att cta gaa gcc tat ttt      2780

Ala His Ser Val Lys Gln Ala Glu Lys Asp Ile Leu Glu Ala Tyr Phe
835                 840                 845                 850

aag gaa caa gaa cgc tgt gca cag tcg gta gaa aca ccc agt gaa gag      2828
Lys Glu Gln Glu Arg Cys Ala Gln Ser Val Glu Thr Pro Ser Glu Glu
                855                 860                 865

ata aca aat cta ttt tct ctc act caa gaa caa cag aaa gaa gtt tta      2876
Ile Thr Asn Leu Phe Ser Leu Thr Gln Glu Gln Gln Lys Glu Val Leu
            870                 875                 880

tta acc tct cct gaa ctg aga aca cag gta aag gct tat tct caa aaa      2924
Leu Thr Ser Pro Glu Leu Arg Thr Gln Val Lys Ala Tyr Ser Gln Lys
        885                 890                 895

ctc tat gac cgt ctc tca cca agt gat ctt cag gct ata agt gaa aga      2972
Leu Tyr Asp Arg Leu Ser Pro Ser Asp Leu Gln Ala Ile Ser Glu Arg
    900                 905                 910

tct cat aca aaa ctt gct gaa agt ctt ggc aca tca gta aat caa gca      3020
Ser His Thr Lys Leu Ala Glu Ser Leu Gly Thr Ser Val Asn Gln Ala
915                 920                 925                 930

gaa aaa att gca caa atc ctc aca caa aca aaa gac gtt gtc cag att      3068
Glu Lys Ile Ala Gln Ile Leu Thr Gln Thr Lys Asp Val Val Gln Ile
                935                 940                 945

ctt caa cag cag gaa aaa ctt ggt ctg tat caa tct atc atg aaa agt      3116
Leu Gln Gln Gln Glu Lys Leu Gly Leu Tyr Gln Ser Ile Met Lys Ser
            950                 955                 960

gat ggc aga gag act gca aaa gtc aac atg tca gca att aaa gca acg      3164
Asp Gly Arg Glu Thr Ala Lys Val Asn Met Ser Ala Ile Lys Ala Thr
        965                 970                 975

caa atg aca aaa aag atc aca tca tta aaa gca gta gaa cag ata gtt      3212
Gln Met Thr Lys Lys Ile Thr Ser Leu Lys Ala Val Glu Gln Ile Val
    980                 985                 990

cga ccg cca aaa gtt gaa  act gca aaa gta gtt  tct atg agt aga      3257
Arg Pro Pro Lys Val Glu  Thr Ala Lys Val Val  Ser Met Ser Arg
995                 1000                 1005

taagcatacc tgaaactgtg tttatatggt ccctttgtgc gattgttttg tgtagattgt      3317

cgaagagatg ttgtttaatc ataaaaattg acatttctct tttaagtaaa gctataaaaa      3377

taacgtaaag gtggagagtg cactttttaa gctttcaaaa acaaatattg ctattgagaa      3437

agcttgaaag gaaatatcta tgaaaaaaac caaacgaa                              3475

<210>  17
<211>  1009
<212>  PRT
<213>  Bartonella henselae

<400>  17

Met Lys Lys Lys Gln Pro Ala Pro Ser Thr Pro Ser Ser Ala Ser Val
1               5                   10                  15

Thr Gln Met Ala Ser Leu Ser Asn Glu Glu Ile Phe Asn Arg Val Gln

            20                  25                  30

Arg Asp Pro Leu Val Gln Ser Cys Glu Glu Glu Ile Gln Tyr Trp Cys
        35                  40                  45

Lys Ile Val Phe Gly Asn Ala Tyr Val Leu Gln Tyr Arg Ile Glu Asp
    50                  55                  60

Met Gln Lys Ile Pro Glu Ile Gly Glu Glu Leu Ser Trp Gln Ile Ser
65                  70                  75                  80

Glu Asn Pro His Phe Phe His Lys Leu Ala Gly Ser Gln Thr Leu Gly
                85                  90                  95

Ile Lys Asn Ser Ala Arg Lys Glu Ala Glu Ala Gly Ile Ser Ser Leu
            100                 105                 110

Cys Asn Ala Ile Glu Thr Tyr Ala Asp Thr Val Lys Gln Val Arg Lys
        115                 120                 125

Ser Ile Val Gln Ala His Gln Ala Gln Gln Asn Arg Gln Glu Leu Ser
    130                 135                 140

Ser Glu Pro Ser Gln Asp Leu Gln Lys Gln Gln Ser Leu Ser Lys Pro
145                 150                 155                 160

Pro Lys Leu Pro Glu His Ser Pro Glu Asp Arg His Gln Glu Ser Thr
                165                 170                 175

Glu Ser Ser Met Gln Ala Lys Trp Asn Arg Leu Gly Ala Arg Pro Arg
            180                 185                 190

Thr Val Pro Leu Ser Gln Lys Thr Gln Lys Gln Gln Glu Gln Leu Gly
        195                 200                 205

Leu His Gln Pro Ile Met Glu Gly Asp Gly Lys Glu Ala Ala Lys Val
    210                 215                 220

Ser Met Ser Ala Ile Lys Ala Thr Lys Val Thr Thr Lys Ser Glu Ala
225                 230                 235                 240

Leu Lys Ala Val Glu Gln Leu Ile Gln Pro Pro Lys Val Glu Thr Ala
                245                 250                 255

Lys Val Val Ser Val Pro Leu Ala Gln Glu Ala Gln Lys Thr Leu Ile
            260                 265                 270

Glu Lys Glu Thr Ile Pro Ser Leu Thr Asn Glu Glu Val Ala Ser Lys

        275                 280                 285

Ile Arg Glu Ser Glu Val Val Lys Ser Ser Met Gln Lys Ile Lys Thr
    290                 295                 300

Leu Cys Gly Val Val Tyr Gly Asn Pro His Ile Leu Glu Gly Lys Met
305                 310                 315                 320

Pro Lys Met Gly Ile Pro Val Thr Asn Arg Asn Val Glu Glu Leu Glu
                325                 330                 335

Lys Phe Ala Arg Gln Val Gly Ser Phe Pro Ser Ser Phe Gly Arg Val
            340                 345                 350

Ala Gly Phe Ser Phe Leu Gly Ile Lys Ser Gly Ala Arg Ala His Ala
        355                 360                 365

Glu Glu Asn Phe Leu Pro Leu Ser His Ala Ile Val Ser Tyr Thr His
    370                 375                 380

Asn Val Lys Gln Ala Glu Lys Asp Ile Leu Glu Ala His Ser Gly Lys
385                 390                 395                 400

Gln Glu Arg Tyr Ala Gln Ser Val Glu Thr Pro Ser Glu Glu Ile Gln
                405                 410                 415

Asn Leu Phe Ser Leu Thr Gln Glu Ala Gln Lys Met Leu Ile Glu Lys
            420                 425                 430

Glu Ile Ile Pro Pro Leu Ser Tyr Val Asp Val Ala Ser Lys Ile Arg
        435                 440                 445

Glu Ser Glu Val Val Lys Ser Ser Lys Gln Lys Ile Glu Thr Leu Cys
    450                 455                 460

Ser Val Val Tyr Gly Asn Pro Arg Ile Leu Glu Asp Lys Met Pro Lys
465                 470                 475                 480

Met Gly Ile Pro Val Thr Asn Lys Asn Val Glu Glu Leu Glu Lys Phe
                485                 490                 495

Ala Arg Gln Val Gly Asn Phe Pro Ser Ser Cys Gly Lys Ile Val Gly
            500                 505                 510

Phe Asn Phe Leu Gly Ile Lys Ser Glu Ala Arg Ala His Ala Glu Glu
        515                 520                 525

Asn Phe Leu Pro Leu Ser His Ala Ile Phe Ser Tyr Ala His Ser Val

530                 535                 540

Lys Gln Ala Glu Lys Asp Ile Leu Glu Ala Tyr Phe Lys Glu Gln Glu
545                 550                 555                 560

Arg Cys Ala Gln Ser Val Glu Thr Pro Ser Glu Glu Ile Thr Asn Leu
                565                 570                 575

Phe Ser Phe Thr Gln Glu Gln Gln Lys Glu Ile Leu Ser Asn Ser Pro
            580                 585                 590

Lys Leu Arg Thr Gln Val Lys Ala Tyr Ser Gln Lys Leu His Asn Arg
        595                 600                 605

Leu Ser Pro Ser Asp Leu Gln Ala Ile Ser Glu Arg Ser His Thr Lys
    610                 615                 620

Leu Ala Glu Ser Leu Gly Thr Ser Val Asn Gln Ala Glu Glu Ile Ala
625                 630                 635                 640

Gln Ile Leu Thr Gln Thr Lys Asp Val Val Gln Ile Leu Gln Gln Gln
                645                 650                 655

Glu Lys Leu Gly Leu Tyr Gln Ser Ile Met Lys Gly Asp Gly Lys Glu
            660                 665                 670

Thr Ala Lys Val Ser Met Ser Ala Ile Lys Ala Thr Glu Val Thr Thr
        675                 680                 685

Lys Phe Glu Ala Ser Lys Ala Val Glu Gln Ala Val Arg Pro Pro Lys
    690                 695                 700

Val Glu Thr Ala Lys Val Val Ser Val Ser Phe Thr Gln Glu Thr Gln
705                 710                 715                 720

Lys Met Leu Ile Glu Lys Glu Ile Ile Pro Pro Leu Ser Tyr Val Asp
                725                 730                 735

Val Ala Ser Lys Ile Arg Glu Ser Glu Val Val Lys Ser Ser Met Gln
            740                 745                 750

Lys Ile Lys Thr Leu Cys Ser Val Val Tyr Gly Asn Pro Asp Ile Leu
        755                 760                 765

Glu Gly Lys Met Pro Lys Met Gly Ile Pro Val Thr Asn Lys Asn Val
    770                 775                 780

Glu Glu Leu Glu Lys Phe Ala Arg Gln Val Gly Asn Phe Pro Ser Ser

785                 790                 795                 800

Cys Gly Lys Ile Val Gly Phe Ser Phe Leu Gly Ile Lys Ser Glu Ala
                805                 810                 815

Arg Ala His Ala Glu Glu Asn Phe Leu Pro Leu Ser His Ala Ile Phe
            820                 825                 830

Ser Tyr Ala His Ser Val Lys Gln Ala Glu Lys Asp Ile Leu Glu Ala
        835                 840                 845

Tyr Phe Lys Glu Gln Glu Arg Cys Ala Gln Ser Val Glu Thr Pro Ser
    850                 855                 860

Glu Glu Ile Thr Asn Leu Phe Ser Leu Thr Gln Glu Gln Gln Lys Glu
865                 870                 875                 880

Val Leu Leu Thr Ser Pro Glu Leu Arg Thr Gln Val Lys Ala Tyr Ser
                885                 890                 895

Gln Lys Leu Tyr Asp Arg Leu Ser Pro Ser Asp Leu Gln Ala Ile Ser
            900                 905                 910

Glu Arg Ser His Thr Lys Leu Ala Glu Ser Leu Gly Thr Ser Val Asn
        915                 920                 925

Gln Ala Glu Lys Ile Ala Gln Ile Leu Thr Gln Thr Lys Asp Val Val
    930                 935                 940

Gln Ile Leu Gln Gln Gln Glu Lys Leu Gly Leu Tyr Gln Ser Ile Met
945                 950                 955                 960

Lys Ser Asp Gly Arg Glu Thr Ala Lys Val Asn Met Ser Ala Ile Lys
                965                 970                 975

Ala Thr Gln Met Thr Lys Lys Ile Thr Ser Leu Lys Ala Val Glu Gln
            980                 985                 990

Ile Val Arg Pro Pro Lys Val Glu  Thr Ala Lys Val Val  Ser Met Ser
        995                 1000                1005

Arg

<210>  18
<211>  544
<212>  PRT
<213>  Bartonella tribocorum

<400> 18

Met Pro Lys Ala Lys Glu Lys Thr Lys Asn Ile Glu Thr Ser Ser Pro
1               5                   10                  15

His His Tyr Val Tyr Pro Asn Thr Thr Thr Leu Lys Asn Lys Tyr Gly
            20                  25                  30

Ile Thr Asn Phe Asp Ala Phe Thr Ala Lys Tyr Thr His Asp Thr Ala
        35                  40                  45

Lys Ala Ile Val Asn Leu Arg Glu Ala Pro Leu Pro Glu Lys Phe Asp
    50                  55                  60

Ser Ser Tyr Leu Cys Ser Ile His His Gln Leu Phe Lys Asn Thr Phe
65                  70                  75                  80

Glu Trp Ala Gly Gln Pro Arg Asn Val Pro Phe Thr Phe Leu Asp Glu
                85                  90                  95

Ser Val Thr Ala Met Pro Glu Met Lys Arg Ala Glu Trp Asn Asn Val
            100                 105                 110

Phe Ala Asn Asp Asp Glu Ile Glu Glu Gly Leu Gln Thr Leu Asn Lys
        115                 120                 125

Thr Leu Ala Glu Lys Asn Ala Leu Arg Ser Leu Thr Arg Glu Asp Phe
    130                 135                 140

Ile Ser Glu Ala Val Pro Leu Phe Val Ser Leu Lys Asn Ile Arg Pro
145                 150                 155                 160

Phe Ile Asn Gly Asn Glu His Thr Ile His Leu Phe Phe Glu Lys Leu
                165                 170                 175

Ala Gln Ala Ala Glu His Gln Leu Asp Phe Ser Leu Val Thr Lys Glu
            180                 185                 190

Arg Met Leu Val Ala Ser Val Ala Ala Ile Gln Asn Asp Asn Leu Glu
        195                 200                 205

Pro Met Gln His Leu Phe Glu Asp Ile Ser Asn Pro Gln Lys Ile His
    210                 215                 220

Leu Leu Lys Glu Phe Met Ser Asn Met Lys Glu Leu Gly Arg Asp Ile
225                 230                 235                 240

Asn Asp Arg Ser Val Met Val Thr Lys Glu Gly Glu Thr Tyr Thr Gly
                245                 250                 255

Thr Tyr Arg Gly Ala Gly His Asp Ser Leu Ala Phe Asn Val Asn Gly
            260                 265                 270

Ala Tyr Ile Ile Gly Asp Lys Glu Asp Leu Thr Pro Glu Gln Leu Lys
        275                 280                 285

Thr Leu Lys Pro Gly Asp Lys Phe Thr Phe Thr Ala Ser Gln Thr Gln
    290                 295                 300

Glu Leu Lys Asn Ile Leu Ile Pro Lys Glu Thr Leu Ala Pro Leu Ser
305                 310                 315                 320

Lys Thr Glu Lys Ala Glu Met Ile Ala Lys Asp Ala Cys Val His Thr
                325                 330                 335

Ala Arg Asn Gln Ile Gln Arg Leu Ser Lys Thr Val Tyr Asp Asn Ala
            340                 345                 350

Lys Thr Leu Asp Lys Lys Met Val Ala Ile Ile Lys Asn Pro Ala Leu
        355                 360                 365

Gly Gln Gln Leu Ala Glu Gln Ile Glu Arg Ser Pro Asp Ser Val Ala
    370                 375                 380

His Leu Ala Gly Phe Ser Leu Cys Gly Leu Gln Asn Gln Ala Arg Ala
385                 390                 395                 400

Asn Ala Lys Asn His Leu Glu Met Leu Cys Ser Ala Val Ala Asn Phe
                405                 410                 415

Ala His Ala Val Lys His Ala Glu Lys Glu Ile Thr Gln Glu His Ala
            420                 425                 430

Thr Glu Gln Lys Arg Arg Ala Thr Thr Val Glu Met Pro Ser Lys Ser
        435                 440                 445

Leu Gln Asp Leu Phe Thr Leu Pro Lys Glu Leu Gln Gln Glu Ser Leu
    450                 455                 460

Ala Lys Asn Leu Gly Leu Gln Lys Glu Leu Ser Asn Leu Val Lys Asn
465                 470                 475                 480

Ile Asn Ser Arg Leu Ser Ala Thr Glu His Lys Ala Val Lys Asn Asn
                485                 490                 495

Asp Tyr Glu Thr Leu Ala Gln Ser Ile Gly Val Ser Glu Asn Lys Ala
            500                 505                 510

Lys Gln Ile Thr Gln Thr Val Lys Gln Ala Lys Glu Val His Gln Gln
        515                 520                 525

Ala Cys Thr Arg Thr Val Asn His Ser Asn Val Leu Ala Ile Ala Ser
    530                 535                 540

<210> 19
<211> 17
<212> DNA
<213> Artificial sequence

<220>
<223> Primer

<400> 19
atcgcccgga agtttgc                                                    17

<210> 20
<211> 20
<212> DNA
<213> Artificial sequence

<220>
<223> Primer

<400> 20
cagctctcgt ttgcgtgttg                                                 20

<210> 21
<211> 21
<212> DNA
<213> Artificial sequence

<220>
<223> Primer

<400> 21
tatgctttcc ttggagccaa a                                               21

<210> 22
<211> 18
<212> DNA
<213> Artificial sequence

<220>
<223> Primer

<400> 22
gctggaccct ctgctcac                                                   18

<210> 23
<211> 20
<212> DNA
<213> Artificial sequence

<220>

<223>  Primer

<400>  23
cgctcgaata cgatgactcg                                                    20


<210>  24
<211>  24
<212>  DNA
<213>  Artificial sequence

<220>
<223>  Primer

<400>  24
ccactgagtg ttgttttcca tgat                                               24


<210>  25
<211>  19
<212>  DNA
<213>  Artificial sequence

<220>
<223>  Primer

<400>  25
gaaggtgaag gtcggagtc                                                     19


<210>  26
<211>  20
<212>  DNA
<213>  Artificial sequence

<220>
<223>  Primer

<400>  26
gaagatggtg atgggatttc                                                    20


<210>  27
<211>  40
<212>  DNA
<213>  Artificial sequence

<220>
<223>  Primer

<400>  27
ataagaatgc ggccgcgatg aaaaaaaacc aaccatcctc                              40


<210>  28
<211>  29
<212>  DNA
<213>  artificial sequence

<220>
<223>  Primer

<400>  28
cgggatcctt agagtgccag caccatttt                                          29

<210>  29
<211>  568
<212>  PRT
<213>  Bartonella tribocorum

<400>  29

Met Gln Lys Gly Ile Phe His Ser Arg Asn Leu Thr Leu Leu Trp Thr
1               5                   10                  15

Thr Leu Asp Tyr Lys Glu Met Thr Met Pro Lys Ala Lys Glu Lys Thr
            20                  25                  30

Lys Asn Ile Glu Thr Ser Ser Pro His His Tyr Val Tyr Pro Asn Thr
        35                  40                  45

Thr Thr Leu Lys Asn Lys Tyr Gly Ile Thr Asn Phe Asp Ala Phe Thr
    50                  55                  60

Ala Lys Tyr Thr His Asp Thr Ala Lys Ala Ile Val Asn Leu Arg Glu
65                  70                  75                  80

Ala Pro Leu Pro Glu Lys Phe Asp Ser Ser Tyr Leu Cys Ser Ile His
                85                  90                  95

His Gln Leu Phe Lys Asn Thr Phe Glu Trp Ala Gly Gln Pro Arg Asn
            100                 105                 110

Val Pro Phe Thr Phe Leu Asp Glu Ser Val Thr Ala Met Pro Glu Met
        115                 120                 125

Lys Arg Ala Glu Trp Asn Asn Val Phe Ala Asn Asp Asp Glu Ile Glu
    130                 135                 140

Glu Gly Leu Gln Thr Leu Asn Lys Thr Leu Ala Glu Lys Asn Ala Leu
145                 150                 155                 160

Arg Ser Leu Thr Arg Glu Asp Phe Ile Ser Glu Ala Val Pro Leu Phe
                165                 170                 175

Val Ser Leu Lys Asn Ile Arg Pro Phe Ile Asn Gly Asn Glu His Thr
            180                 185                 190

Ile His Leu Phe Phe Glu Lys Leu Ala Gln Ala Ala Glu His Gln Leu
        195                 200                 205

Asp Phe Ser Leu Val Thr Lys Glu Arg Met Leu Val Ala Ser Val Ala
    210                 215                 220

Ala Ile Gln Asn Asp Asn Leu Glu Pro Met Gln His Leu Phe Glu Asp
225                 230                 235                 240

Ile Ser Asn Pro Gln Lys Ile His Leu Leu Lys Glu Phe Met Ser Asn
                245                 250                 255

Met Lys Glu Leu Gly Arg Asp Ile Asn Asp Arg Ser Val Met Val Thr
            260                 265                 270

Lys Glu Gly Glu Thr Tyr Thr Gly Thr Tyr Arg Gly Ala Gly His Asp
        275                 280                 285

Ser Leu Ala Phe Asn Val Asn Gly Ala Tyr Ile Ile Gly Asp Lys Glu
    290                 295                 300

Asp Leu Thr Pro Glu Gln Leu Lys Thr Leu Lys Pro Gly Asp Lys Phe
305                 310                 315                 320

Thr Phe Thr Ala Ser Gln Thr Gln Glu Leu Lys Asn Ile Leu Ile Pro
                325                 330                 335

Lys Glu Thr Leu Ala Pro Leu Ser Lys Thr Glu Lys Ala Glu Met Ile
            340                 345                 350

Ala Lys Asp Ala Cys Val His Thr Ala Arg Asn Gln Ile Gln Arg Leu
        355                 360                 365

Ser Lys Thr Val Tyr Asp Asn Ala Lys Thr Leu Asp Lys Lys Met Val
    370                 375                 380

Ala Ile Ile Lys Asn Pro Ala Leu Gly Gln Gln Leu Ala Glu Gln Ile
385                 390                 395                 400

Glu Arg Ser Pro Asp Ser Val Ala His Leu Ala Gly Phe Ser Leu Cys
                405                 410                 415

Gly Leu Gln Asn Gln Ala Arg Ala Asn Ala Lys Asn His Leu Glu Met
            420                 425                 430

Leu Cys Ser Ala Val Ala Asn Phe Ala His Ala Val Lys His Ala Glu
        435                 440                 445

Lys Glu Ile Thr Gln Glu His Ala Thr Glu Gln Lys Arg Arg Ala Thr
    450                 455                 460

Thr Val Glu Met Pro Ser Lys Ser Leu Gln Asp Leu Phe Thr Leu Pro
465                 470                 475                 480

Lys Glu Leu Gln Gln Glu Ser Leu Ala Lys Asn Leu Gly Leu Gln Lys
                485                 490                 495

Glu Leu Ser Asn Leu Val Lys Asn Ile Asn Ser Arg Leu Ser Ala Thr
            500                 505                 510

Glu His Lys Ala Val Lys Asn Asn Asp Tyr Glu Thr Leu Ala Gln Ser
        515                 520                 525

Ile Gly Val Ser Glu Asn Lys Ala Lys Gln Ile Thr Gln Thr Val Lys
    530                 535                 540

Gln Ala Lys Glu Val His Gln Gln Ala Cys Thr Arg Thr Val Asn His
545                 550                 555                 560

Ser Asn Val Leu Ala Ile Ala Ser
                565

<210>  30
<211>  137
<212>  PRT
<213>  Artificial sequence

<220>
<223>  VTP DOMAIN CONSENSUS

<400>  30

Leu Ile Glu Lys Glu Thr Leu Pro Pro Leu Ser Asn Glu Glu Ile Ala
1               5                   10                  15

Ser Arg Val Gln Glu Asn Ala Leu Val Gln Ser Tyr Gln Gln Glu Ile
            20                  25                  30

Gln His Trp Cys Lys Ile Val Tyr Gly Asn Pro Tyr Val Leu Asn Lys
        35                  40                  45

Lys Ile Glu Glu Ile Leu Lys Asn Pro Ser Leu Gly Glu Glu Leu Ser
    50                  55                  60

Trp Gln Val Ala Glu Asn Pro Lys Ser Phe His Lys Leu Ala Gly Phe
65                  70                  75                  80

Lys Leu Leu Gly Ile Lys Asn Pro Ala Arg Arg His Ala Glu Glu Gly
                85                  90                  95

Leu Ser Ser Leu Cys Asp Ala Ile Glu Ser Tyr Ala Asp Ala Val Lys
            100                 105                 110

Gln Ala Arg Glu Asp Ile Thr Gln Glu His Gln Ala Glu Gln Arg Arg

115                 120                 125

Gln Ala Gln Ser Val Glu Lys Pro Ser
    130                 135

<210>  31
<211>  309
<212>  PRT
<213>  Artificial sequence

<220>
<223>  FIC DOMAIN CONSENSUS

<400>  31

Met Pro Lys Ala Lys Ala Lys Thr Lys Asn Ile Glu Thr Tyr Ser Pro
1               5                   10                  15

His Asn Tyr Val Tyr Pro Asn Thr Thr Thr Leu Lys Asn Lys Tyr Gly
            20                  25                  30

Ile Thr Asp Leu Ser Ala Leu Leu Glu Lys Cys Ala His Asp Thr Ala
        35                  40                  45

Lys Ala Leu Val Asn Leu Arg Glu Glu Ser Leu Pro Glu Tyr Phe Asp
    50                  55                  60

Thr Ala Tyr Leu Lys Ala Ile His Gln Gln Leu Phe Gln Asn Thr Phe
65                  70                  75                  80

Glu Trp Ala Gly His Thr Arg Asn Glu Pro Phe Thr Phe Ala Asp Gly
                85                  90                  95

Ser Val Ala Ala Met Pro Glu Met Lys Arg Thr Gly Trp Lys Asn Ser
            100                 105                 110

Phe Ala Val Gly Asp Glu Ile Glu Glu Gly Leu Gln Glu Leu Asp Arg
        115                 120                 125

Thr Leu Ala Glu Lys Asn Asn Leu Gln Gly Leu Thr Arg Glu Glu Phe
    130                 135                 140

Ala Glu Glu Ala Ala Glu Leu Phe Ser Ser Leu Asn Lys Ile His Pro
145                 150                 155                 160

Phe Arg Glu Gly Asn Gly Arg Ala Gln Arg Leu Phe Phe Glu Lys Leu
                165                 170                 175

Ala Lys Ala Ala Gly His Gln Leu Asp Phe Ser Leu Ile Thr Lys Glu
            180                 185                 190

Arg Met Ile Glu Ala Ser Ile Ala Ala Met Gln Asn Gly Asp Thr Glu
        195                 200                 205

Pro Met Lys His Leu Phe Glu Asp Ile Ser Asn Pro Glu Lys Ile Arg
    210                 215                 220

Leu Leu Lys Glu Phe Met Ser Asn Met Arg Asn Ser Gly Arg Glu Ile
225                 230                 235                 240

Asn Asp His Ile Val Met Val Ala Lys Glu Gly Glu Thr Tyr Thr Gly
                245                 250                 255

Thr Tyr Arg Gly Ala Gly Asp Glu Gly Phe Val Leu Asn Val Asp Gly
            260                 265                 270

Gly Tyr Val Val Gly Asp Lys Asp Asp Leu Thr Pro Glu Gln Leu Lys
        275                 280                 285

Thr Leu Lys Pro Gly Asp Lys Ile Thr Phe Thr Ala Ser Lys Asp Lys
    290                 295                 300

Glu Leu Lys Glu Ile
305

<210>  32
<211>  18
<212>  PRT
<213>  Artificial sequence

<220>
<223>  Motif consensus

<400>  32

Glu Arg Gly Arg Arg Pro Gln Lys Pro Leu Glu Pro Leu Tyr Ala Glu
1               5                   10                  15

Val Gly

<210>  33
<211>  14
<212>  PRT
<213>  Artificial sequence

<220>
<223>  Motif consensus

<400>  33

Thr Glu Ser Thr Pro Gln Ser Glu Thr Leu Tyr Ala Thr Val
1               5                   10

<210>  34
<211>  17
<212>  PRT
<213>  Artificial sequence

<220>
<223>  motif consensus

<400>  34

Thr Ser Ser Gln Asp Ser Thr Pro Leu Tyr Ala Thr Pro Leu Pro Gln
1               5                   10                  15

Gln

<210>  35
<211>  8
<212>  PRT
<213>  Bartonella henselae

<400>  35

His Arg His His Arg Arg His His
1               5

<210>  36
<211>  8
<212>  PRT
<213>  Bartonella henselae

<220>
<221>  MISC_FEATURE
<222>  (4)..(5)
<223>

<220>
<221>  MISC_FEATURE
<222>  (4)..(5)
<223>  Any amino acid

<400>  36

His Pro Phe Xaa Xaa Gly Asn Gly
1               5

<210>  37
<211>  53
<212>  PRT
<213>  Artificial sequence

<220>
<223>  C-Terminal peptide

<400>  37

Ser Arg Pro Gly Cys Leu Ala Val Tyr Gly Gly Arg Pro Ala Arg His

1               5                   10                  15

Pro Pro Gly Arg Cys Phe Ala Thr Phe Lys Ser Ala Pro Gly Gly Phe
            20                  25                  30

Val Leu Leu Arg Arg Ala Phe Thr Asp Lys Gln Gln Ile Lys Arg Lys
        35                  40                  45

Ala Gln Ser Phe Asp
    50

<210>  38
<211>  80
<212>  DNA
<213>  Artificial sequence

<220>
<223>  PCR Primer

<400>  38
cgcgagctct ttaagaagga gatatacaaa tggactataa agacgacgat gacaaacata      60

tgggatgcct ggcagtttat                                                  80

<210>  39
<211>  29
<212>  DNA
<213>  Artificial sequence

<220>
<223>  PCR Primer

<400>  39
cgcgagctct aaatcagaac gcagaagcg                                        29

<210>  40
<211>  31
<212>  DNA
<213>  Artificial sequence

<220>
<223>  PCR Primer

<400>  40
ggaattccat atgccaaagg caaaagcaaa a                                     31

<210>  41
<211>  31
<212>  DNA
<213>  Artificial sequence

<220>
<223>  PCR Primer

<400>  41
ggattccata tgttagctag ccatggcaag c                                     31

<210>  42
<211>  19
<212>  DNA
<213>  Artificial sequence

<220>
<223>  Sequencing primer

<400>  42
ggaaacagct atgaccatg                                                    19


<210>  43
<211>  10
<212>  PRT
<213>  Artificial sequence

<220>
<223>  FLAG Peptide

<400>  43

Met Cys Tyr Lys Asp Asp Asp Asp Lys His
1               5                   10


<210>  44
<211>  32
<212>  DNA
<213>  Artificial sequence

<220>
<223>  PCR Primer

<400>  44
ggaattccat atgaaaaaaa atcgaccatc cc                                     32


<210>  45
<211>  34
<212>  DNA
<213>  Artificial sequence

<220>
<223>  PCR Primer

<400>  45
ggaattccat atgttacata ccaaaggcca ttcc                                   34


<210>  46
<211>  31
<212>  DNA
<213>  Artificial sequence

<220>
<223>  PCR Primer

<400>  46
ggaattccat atgaaaagaa atcaaccacc c                                      31


<210>  47

<211>  33
<212>  DNA
<213>  Artificial sequence

<220>
<223>  PCR Primer

<400>  47
ggaattccat atgttagatg gcgaaagtat tgc                                    33


<210>  48
<211>  32
<212>  DNA
<213>  Artificial sequence

<220>
<223>  PCR Primer

<400>  48
ggaattccat atgaaaaaaa accaaccatc ct                                     32


<210>  49
<211>  33
<212>  DNA
<213>  Artificial sequence

<220>
<223>  PCR Primer

<400>  49
ggaattccat atgttagagt gccagcacca ttt                                    33


<210>  50
<211>  32
<212>  DNA
<213>  Artificial sequence

<220>
<223>  PCR Primer

<400>  50
cgcgcttatt aatggacatt ctctctgaaa gg                                     32


<210>  51
<211>  36
<212>  DNA
<213>  Artificial sequence

<220>
<223>  PCR Primer

<400>  51
cgcgcttatt aatttatcta ctcatagaaa ctactt                                 36


<210>  52
<211>  29
<212>  DNA
<213>  Artificial sequence

<220>
<223>  PCR Primer

<400>  52
cggatcccgt gtaggtattg gctggatgt                                          29


<210>  53
<211>  21
<212>  DNA
<213>  Artificial sequence

<220>
<223>  PCR Primer

<400>  53
tacaccttca acaaccatca c                                                  21


<210>  54
<211>  38
<212>  DNA
<213>  Artificial sequence

<220>
<223>  PCR Primer

<400>  54
tcatggttgt tgaaggtgta aatgatgaga tcgccgtc                                38


<210>  55
<211>  28
<212>  DNA
<213>  Artificial sequence

<220>
<223>  PCR Primer

<400>  55
cggatcccgc tcttcacgca attcttct                                           28


<210>  56
<211>  95
<212>  DNA
<213>  Artificial sequence

<220>
<223>  Oligonucleotide for plasmid construction

<400>  56
ttcggctcac aggagcgata tcatggataa agcggaatta attcccgagc ctccaaaaaa      60

gaagagaaag gtcgaattgg gtaccggtcg agatc                                   95


<210>  57
<211>  23
<212>  DNA
<213>  Artificial sequence

<220>
<223>  Oligonucleotide for plasmid construction

<400>  57
ctctagagtc gatcgacctg cag                                              23


<210>  58
<211>  53
<212>  PRT
<213>  Artificial sequence

<220>
<223>  C-terminal peptide of extended CRE

<400>  58

Ser Arg Pro Gly Cys Leu Ala Val Tyr Gly Gly Arg Pro Ala Arg His
1               5                   10                  15

Pro Pro Gly Arg Cys Phe Ala Thr Phe Lys Ser Ala Pro Gly Gly Phe
            20                  25                  30

Val Leu Leu Arg Arg Ala Phe Thr Asp Lys Gln Gln Ile Lys Arg Lys
        35                  40                  45

Ala Gln Ser Phe Asp
    50


<210>  59
<211>  30
<212>  DNA
<213>  Artificial sequence

<220>
<223>  PCR Primer

<400>  59
tccccccggg ttagctggca atagcaagcg                                       30


<210>  60
<211>  31
<212>  DNA
<213>  Artificial sequence

<220>
<223>  PCR Primer

<400>  60
atggtgtcga caagagcttg aaaacacgct c                                     31


<210>  61
<211>  30
<212>  DNA
<213>  Artificial sequence

<220>
<223>  PCR Primer

<400>  61

tccccccggg ttagttggta agagcccttg                                    30

<210>  62
<211>  33
<212>  DNA
<213>  Artificial sequence

<220>
<223>  PCR Primer

<400>  62
atggtgtcga ctcgatctga aagaaatatt gat                                 33

<210>  63
<211>  31
<212>  DNA
<213>  Artificial sequence

<220>
<223>  PCR Primer

<400>  63
tccccccggg ttacatacca aaggccattc c                                   31

<210>  64
<211>  30
<212>  DNA
<213>  Artificial sequence

<220>
<223>  PCR Primer

<400>  64
atggtgtcga caaaatcccc tctacgaagg                                    30

<210>  65
<211>  30
<212>  DNA
<213>  Artificial sequence

<220>
<223>  PCR Primer

<400>  65
tccccccggt tagatggcga aagctattgc                                    30

<210>  66
<211>  31
<212>  DNA
<213>  Artificial sequence

<220>
<223>  PCR Primer

<400>  66
atggtgtcga ccgacgtctg ttcataaact c                                   31

<210>  67

<211>  81
<212>  DNA
<213>  Artificial sequence

<220>
<223>  PCR Primer

<400>  67
cgaggagctc attacctcat atagcataca ttatacgaag ttataattgc ccttgccaca      60

tgattgaaca agatggattg c                                                81


<210>  68
<211>  31
<212>  DNA
<213>  Artificial sequence

<220>
<223>  PCR Primer

<400>  68
cgaggagctc tcagaagaac tggtcaagaa g                                     31


<210>  69
<211>  31
<212>  DNA
<213>  Artificial sequence

<220>
<223>  PCR Primer

<400>  69
cgatggatcc gtttcattgc cctttcgtat t                                     31


<210>  70
<211>  41
<212>  DNA
<213>  Artificial sequence

<220>
<223>  PCR Primer

<400>  70
aaggatcgat ctcgagttat cctggatata gtgtctgtca t                          41


<210>  71
<211>  31
<212>  DNA
<213>  Artificial sequence

<220>
<223>  PCR Primer

<400>  71
aaggctcgag gaggaactaa acaggtgaaa c                                     31


<210>  72
<211>  31
<212>  DNA

<213>  Artificial sequence

<220>
<223>  PCR Primer

<400>  72
aaggctcgag tcattgattt tctctccttt g                                    31


<210>  73
<211>  34
<212>  DNA
<213>  Artificial sequence

<220>
<223>  PCR Primer

<400>  73
acgcgtcgac aaagtagttt ctatgagtag ataa                                 34


<210>  74
<211>  33
<212>  DNA
<213>  Artificial sequence

<220>
<223>  PCR Primer

<400>  74
ccgctcgagt gcaaattttt taatcaggat cat                                  33


<210>  75
<211>  28
<212>  DNA
<213>  Artificial sequence

<220>
<223>  PCR Primer

<400>  75
gctctagatg gtatgtccgt tcagaacc                                        28


<210>  76
<211>  31
<212>  DNA
<213>  Artificial sequence

<220>
<223>  PCR Primer

<400>  76
acgcgtcgac agagggatcc ctcaaattca t                                    31


<210>  77
<211>  50
<212>  DNA
<213>  Artificial sequence

<220>
<223>   open reading frame for cryptic fusion peptide

<400>  77
atgaatttga ggatccctct gtcgacaaag tagtttctat gagtagataa                50

<210>  78
<211>  27
<212>  DNA
<213>  Artificial sequence

<220>
<223>  PCR Primer

<400>  78
gctctagagg gcgcttccat tccgtgt                                         27

<210>  79
<211>  54
<212>  DNA
<213>  Artificial sequence

<220>
<223>  open reading frame for cryptic peptide

<400>  79
atgccaaagg caaaagcaaa aacggtcgac aaagtagttt ctatgagtag ataa           54

<210>  80
<211>  34
<212>  DNA
<213>  Artificial sequence

<220>
<223>  PCR Primer

<400>  80
acgcgtcgac cgtttttgct tttcggtttg gcat                                 34